# EUROPEAN PATENT APPLICATION

(11) **EP 3 536 708 A1**
(43) Date of publication of application: **11.09.2019**
(21) Application number: 19160808.2
(22) Date of filing: 09.04.2012
(51) Int. Cl.: C07K 16/28, A61K 39/395, A61P 35/00

(54) **COMBINATIONS OF ANTI-4-1BB ANTIBODIES AND ADCC-INDUCING ANTIBODIES FOR THE TREATMENT OF CANCER**

(30) Priority: 19.04.2011 US 201161477153 P
(62) Divisional of application: 12713849.3
(71) Applicant: Pfizer Inc., New York, NY 10017 (US)
(72) Inventor: ELLIOTT, Mark William, San Diego, CA 92128 (US); FISHER, Timothy Scott, San Diego, CA 92129 (US); SHARP, Leslie Lynne, San Diego, CA 92128 (US)
(74) Representative: Pfizer

(57) **Abstract**

Methods for treating cancer in a patient in need thereof, with a therapeutically effective amount of an anti-4-1BB antibody in combination with a therapeutically effective amount of an ADCC-inducing antibody, are disclosed.

## Description

### Background

Cancer is now the leading cause of death in the United States. Currently, it is typically treated with one or a combination of three types of therapies: surgery, radiation, and chemotherapy. Chemotherapy involves the disruption of cell replication or cell metabolism. The adverse effects of systemic chemotherapy used in the treatment of neoplastic disease can be life threatening and have become of major importance to the clinical management of cancer patients.

4-1BB (also referred to as CD137, TNFRSF9, etc) is a transmembrane protein of the Tumor Necrosis Factor receptor superfamily (TNFRS). Current understanding of 4-1BB indicates that expression is generally activation dependent and is present in a broad subset of immune cells including activated NK and NKT cells, regulatory T cells, dendritic cells (DC), stimulated mast cells, differentiating myeloid cells, monocytes, neutrophils, and eosinophils (Wang, 2009, Immunological Reviews 229: 192-215). 4-1BB expression has also been demonstrated on tumor vasculature (Broll, 2001, Amer. J Clin. Pathol. 115(4):543-549; Seaman, 2007, Cancer Cell 11: 539-554) and at sites of inflamed or atherosclerotic endothelium (Drenkard, 2007 FASEB J. 21: 456-463; Olofsson, 2008, Circulation 117: 1292-1301). The ligand that stimulates 4-1BB, i.e., 4-1BB Ligand (4-1BBL), is expressed on activated antigen-presenting cells (APCs), myeloid progenitor cells, and hematopoietic stem cells.

Human 4-1BB is a 255 amino acid protein (Accession No. NM_001561; NP_001552). The complete human 4-1BB amino acid sequence is provided in SEQ ID NO:68. The protein comprises a signal sequence (amino acid residues 1-17), followed by an extracellular domain (169 amino acids), a transmembrane region (27 amino acids), and an intracellular domain (42 amino acids) (Cheuk ATC et al. 2004 Cancer Gene Therapy 11: 215-226). The receptor is expressed on the cell surface in monomer and dimer forms and likely trimerizes with 4-1BB ligand to signal.

Numerous studies of murine and human T cells indicate that 4-1BB promotes enhanced cellular proliferation, survival, and cytokine production (Croft, 2009, Nat Rev Immunol 9:271-285). Studies have indicated that some 4-1BB agonist mAbs increase costimulatory molecule expression and markedly enhance cytolytic T lymphocyte responses, resulting in anti-tumor efficacy in various models. 4-1BB agonist mAbs have demonstrated efficacy in prophylactic and therapeutic settings. Further, 4-1BB monotherapy and combination therapy tumor models have established durable anti-tumor protective T cell memory responses (Lynch, 2008, Immunol Rev. 22: 277-286). 4-1BB agonists also have been shown to inhibit autoimmune reactions in a variety of art-recognized autoimmunity models (Vinay, 2006, J Mol Med 84:726-736). This dual activity of 4-1BB offers the potential to provide anti-tumor activity while dampening autoimmune side effects that can be associated with immunotherapy approaches that break immune tolerance.

The development of targeted therapies is focused on specific targeting of neoplastic cells while sparing normal tissues in order to decrease side effects. An alternative and/or additional approach to cancer therapy is to target the immune system rather than and/or in addition to targeting the tumor itself. ADCC has been hypothesized as a mechanism of tumor destruction resulting in direct antigen presentation and in the induction of tumor antigen specific T cell responses ('cross-priming") (Weiner et al. 2009).

There is a long-felt unmet need for antibodies that bind human 4-1BB, increase a 4-1BB-mediated response, and thereby provide a potential therapeutic for treatment of various diseases and conditions, including cancer.

### Summary

Embodiments disclosed herein relate to methods for treating cancer in a patient in need of such treatment, said method comprising administering to said patient a therapeutically effective amount of an anti-4-1BB antibody, or antigen-binding portion thereof, in combination with a therapeutically effective amount of an ADCC-inducing antibody. The anti-41BB antibody can be, for example, MOR-6032, MOR-7361, MOR-7480, MOR-7480.1, MOR-7480.2, MOR-7483, MOR-7483.1, or MOR-7483.2. In some embodiments, the ADCC-inducing antibody is an anti-CD20 antibody (e.g., rituximab). In some embodiments, the ADCC-inducing antibody is an anti-P-cadherin antibody. In additional embodiments, the ADCC-inducing antibody has enhanced ADCC activity.

Some embodiments relate to a method for the treatment of cancer in a patient in need of such treatment, said method comprising administering to said patient a therapeutically effective amount of an anti-4-1BB antibody, or antigen-binding portion thereof, in combination with a therapeutically effective amount of an anti-CD20 antibody, or antigen-binding portion thereof. In some embodiments, the anti-4-1BB antibody, or antigen-binding portion thereof, comprises:
(a) an H-CDR1 as set forth in SEQ ID NO:29;
(b) an H-CDR2 as set forth in SEQ ID NO:30;
(c) an H-CDR3 as set forth in SEQ ID NO:31;
(d) an L-CDR1 as set forth in SEQ ID NO:34;
(e) an L-CDR2 as set forth in SEQ ID NO:35; and
(f) an L-CDR3 as set forth in SEQ ID NO:36.

In some embodiments, the anti-CD20 antibody, or antigen-binding portion thereof, comprises the 6 CDRs of rituximab. In other embodiments, the anti-4-1BB antibody, or antigen-binding portion thereof, comprises a V_{H} region comprising the amino acid sequence set forth in SEQ ID NO:43 and a V_{L} region comprising the amino acid sequence set forth in SEQ ID NO:45. In further embodiments, the anti-CD20 antibody, or antigen-binding portion thereof, comprises the V_{H} region of rituximab and the V_{L} region of rituximab. In additional embodiments, the anti-4-1BB antibody, or antigen-binding portion thereof, comprises a heavy chain amino acid sequence as set forth in SEQ ID NO:44 and further comprises a light chain amino acid sequence set forth in SEQ ID NO:46, with the proviso that the C-terminal lysine residue of SEQ ID NO:44 is optionally absent. In some embodiments, the anti-CD20 antibody, or antigen-binding portion thereof, comprises the heavy chain amino acid sequence of rituximab and the light chain amino acid sequence of rituximab. In more embodiments, the method comprising administering to said patient a therapeutically effective amount of MOR-7480.1, or antigen-binding portion thereof, in combination with a therapeutically effective amount of rituximab, or antigen-binding portion thereof.

Other embodiments relate to a method for the treatment of cancer in a patient in need of such treatment, said method comprising administering to said patient a therapeutically effective amount of an anti-4-1BB antibody, or antigen-binding portion thereof, in combination with a therapeutically effective amount of an anti-P-cadherin antibody, or antigen-binding portion thereof. In some embodiments, the anti-4-1BB antibody, or antigen-binding portion thereof, comprises:
(a) an H-CDR1 as set forth in SEQ ID NO:29;
(b) an H-CDR2 as set forth in SEQ ID NO:30;
(c) an H-CDR3 as set forth in SEQ ID NO:31;
(d) an L-CDR1 as set forth in SEQ ID NO:34;
(e) an L-CDR2 as set forth in SEQ ID NO:35; and
(f) an L-CDR3 as set forth in SEQ ID NO:36.

In some embodiments, the anti-P-cadherin antibody, or antigen-binding portion thereof, comprises:
(a) an H-CDR1 as set forth in SEQ ID NO:75;
(b) an H-CDR2 as set forth in SEQ ID NO:76;
(c) an H-CDR3 as set forth in SEQ ID NO:77;
(d) an L-CDR1 as set forth in SEQ ID NO:78;
(e) an L-CDR2 as set forth in SEQ ID NO:79; and
(f) an L-CDR3 as set forth in SEQ ID NO:80.

In some embodiments, the anti-4-1BB antibody, or antigen-binding portion thereof, comprises a V_{H} region comprising the amino acid sequence set forth in SEQ ID NO:43 and a V_{L} region comprising the amino acid sequence set forth in SEQ ID NO:45. In other embodiments, the anti-P-cadherin antibody, or antigen-binding portion thereof, comprises a V_{H} region comprising the amino acid sequence set forth in SEQ ID NO:81 and a V_{L} region comprising the amino acid sequence set forth in SEQ ID NO:82. In additional embodiments, the anti-4-1BB antibody, or antigen-binding portion thereof, comprises a heavy chain amino acid sequence as set forth in SEQ ID NO:44 and further comprises a light chain amino acid sequence set forth in SEQ ID NO:46, with the proviso that the C-terminal lysine residue of SEQ ID NO:44 is optionally absent. In some embodiments, the anti-4-1BB antibody comprises MOR-7480.1.

### Brief Description of the Drawings

Figure 1 shows the combinatorial efficacy of 4-1BB and CD20 monoclonal antibodies in a lymphoma model.
Figure 2 shows the combinatorial efficacy of a 4-1BB monoclonal antibody and a P-cadherin monoclonal antibody (g-194-g09 or anti-P-cadherin1) in a colon carcinoma model.
Figure 3 shows the combinatorial efficacy of a 4-1BB monoclonal antibody and a P-cadherin monoclonal antibody (anti-P-cadherin2) in a colon carcinoma model.
Figures 4A and 4B illustrate that combination treatment using 4-1BB and CD20 monoclonal antibodies reduces circulating tumor burden in a spontaneous murine model of lymphoma.
Figure 5 shows the survival benefit of combinatorial treatment of Eµ-myc lymphoma mice with 4-1BB and CD20 monoclonal antibodies.

### Detailed Description

Embodiments disclosed herein relate to methods of treating cancer in a patient in need of such treatment using anti-4-1BB antibodies (e.g., MOR-7480.1) in combination with one or more antibody dependent cellular cytotoxicity (ADCC)-inducing antibodies *(e.g.,* an anti-CD20 such as rituximab or an anti-P-cadherin antibody such as g-194-g09). Those of skill in the art will recognize that certain subclasses of antibodies, such as IgG1 and IgG3, induce ADCC activity. ADCC activity can also be the result of introducing carbohydrate modifications, such as altered glycosylation patterns, into the Fc region as compared to the native carbohydrate pattern. Certain mutations to the Fc region of an antibody, as compared to the wild type Fc region, are also known by those in the art to enhance ADCC activity. The present disclosure demonstrates the combinatorial efficacy of anti-4-1BB antibodies and ADCC-inducing antibodies in tumor models.

Unless otherwise defined herein, scientific and technical terms used in connection with the present embodiments shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art.

The methods and techniques of the present embodiments are generally performed according to methods well known in the art and as described in various general and more specific references that are cited and discussed throughout the present specification unless otherwise indicated. Such references include, e.g., Sambrook and Russell, Molecular Cloning, A Laboratory Approach, Cold Spring Harbor Press, Cold Spring Harbor, NY (2001), Ausubel et al., Current Protocols in Molecular Biology, John Wiley & Sons, NY (2002), and Harlow and Lane Antibodies: A Laboratory Manual, Cold Spring Harbor Laboratory Press, Cold Spring Harbor, NY (1990), which are incorporated herein by reference. Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

### Definitions

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element.

As used herein, the twenty conventional amino acids and their abbreviations follow conventional usage. See Immunology--A Synthesis (2nd Edition, E. S. Golub and D. R. Gren, Eds., Sinauer Associates, Sunderland, Mass. (1991)), which is incorporated herein by reference.

A "conservative amino acid substitution" is one in which an amino acid residue is substituted by another amino acid residue having a side chain R group with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. See, e.g., Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine.

Alternatively, a conservative replacement is any change having a positive value in the PAM250 log-likelihood matrix disclosed in Gonnet et al., Science 256:1443-45 (1992), herein incorporated by reference. A "moderately conservative" replacement is any change having a nonnegative value in the PAM250 log-likelihood matrix.

Preferred amino acid substitutions are those which: (1) reduce susceptibility to proteolysis, (2) reduce susceptibility to oxidation, (3) alter binding affinity for forming protein complexes, and (4) confer or modify other physicochemical or functional properties of such analogs. Analogs comprising substitutions, deletions, and/or insertions can include various muteins of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts). A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al., Nature 354:105 (1991), which are each incorporated herein by reference.

Sequence similarity for polypeptides, which is also referred to as sequence identity, is typically measured using sequence analysis software. Protein analysis software matches similar sequences using measures of similarity assigned to various substitutions, deletions and other modifications, including conservative amino acid substitutions. For instance, GCG contains programs such as "Gap" and "Bestfit" which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA using default or recommended parameters, a program in GCG Version 6.1. FASTA (e.g., FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)). Another preferred algorithm when comparing a sequence of the invention to a database containing a large number of sequences from different organisms is the computer program BLAST, especially blastp or tblastn, using default parameters. *See, e.g.,* Altschul et al., J. Mol. Biol. 215:403-410 (1990); Altschul et al., Nucleic Acids Res. 25:3389-402 (1997); herein incorporated by reference.

An intact "antibody" comprises at least two heavy (H) chains and two light (L) chains inter-connected by disulfide bonds. See generally, Fundamental Immunology, Ch. 7 (Paul, W., ed., 2nd ed. Raven Press, N.Y. (1989)) (incorporated by reference in its entirety for all purposes). Each heavy chain is comprised of a heavy chain variable region (HCVR or V_{H}) and a heavy chain constant region (C_{H}). The heavy chain constant region is comprised of three domains, CH1, CH2 and CH3. Each light chain is comprised of a light chain variable region (LCVR or V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxyl-terminus in the following order: FR1, CDR1, FR2, CDR2, FR3, CDR3, FR4. The assignment of amino acids to each domain is in accordance with the definitions of Kabat, Sequences of Proteins of Immunological Interest (National Institutes of Health, Bethesda, MD (1987 and 1991)), or Chothia & Lesk, J. Mol. Biol. 196:901-917 (1987); Chothia et al., Nature 342:878-883 (1989).

The variable regions of the heavy and light chains contain a binding domain that interacts with an antigen. The constant regions of the antibodies may mediate the binding of the immunoglobulin to host tissues or factors, including various cells of the immune system (*e.g.*, effector cells) and the first component (Clq) of the classical complement system.

The term "antibody" can include antigen-binding portions of an intact antibody that retain capacity to specifically bind the antigen of the intact antibody (*e.g*., 4-1BB, CD20, or P-cadherin). Antigen-binding portions may be produced by recombinant DNA techniques or by enzymatic or chemical cleavage of intact antibodies.

Examples of antigen-binding portions include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a single domain antibody ("dAb"), which consists of a VH domain as described in Ward et al., Nature 341:544-546 (1989); and (vi) an isolated complementarity determining region (CDR). Furthermore, although the two domains of the Fv fragment, V_{H} and V_{L}, are coded for by separate genes, they can be joined, using recombinant methods, by a synthetic linker that enables them to be made as a single protein chain in which the V_{H} and V_{L} regions pair to form monovalent molecules (known as single chain Fv (scFv); *See, e.g.,* Bird et al. Science 242:423-426 (1988); and Huston et al. Proc. Natl. Acad. Sci. USA 85:5879-5883 (1988)). Such single chain antibodies are included by reference to the term "antibody".

A "bispecific antibody" has two different binding specificities, *see, e.g.,* U.S. Pat. No. 5,922,845 and U.S. Pat. No. 5,837,243; Zeilder J. Immunol. 163:1246-1252 (1999); Somasundaram Hum. Antibodies 9:47-54 (1999); Keler Cancer Res. 57:4008-4014 (1997). For example, the invention provides bispecific antibodies having one binding site for a cell surface antigen (such as human 4-1BB, CD20, or P-cadherin), and a second binding site for an Fc receptor on the surface of an effector cell. The invention also provides multispecific antibodies, which have at least three binding sites.

The term "bispecific antibodies" further includes "diabodies." Diabodies are bivalent, bispecific antibodies in which the V_{H} and V_{L} domains are expressed on a single polypeptide chain, but using a linker that is too short to allow for pairing between the two domains on the same chain, thereby forcing the domains to pair with complementary domains of another chain and creating two antigen binding sites (*See, e.g.,* Holliger et al., Proc. Natl. Acad. Sci. USA 90:6444-6448 (1993); Poljak et al., Structure 2:1121-1123(1994)).

The terms "human antibody" or "human sequence antibody", as used interchangeably herein, include antibodies having variable and constant regions (if present) derived from human germline immunoglobulin sequences. The human sequence antibodies of the invention may include amino acid residues not encoded by human germline immunoglobulin sequences (e.g., mutations introduced by random or site-specific mutagenesis in vitro or by somatic mutation in vivo). However, the term "human antibody", as used herein, is not intended to include "chimeric" antibodies in which CDR sequences derived from the germline of another mammalian species, such as a mouse, have been grafted onto human framework sequences (i.e., "humanized" or PRIMATIZED™ antibodies).

The term "chimeric antibody" as used herein means an antibody that comprises regions from two or more different antibodies. In one embodiment, one or more of the CDRs are derived from a human antibody. In another embodiment, all of the CDRs are derived from a human antibody. In another embodiment, the CDRs from more than one human antibody are combined in a chimeric human antibody. For instance, a chimeric antibody may comprise a CDR1 from the light chain of a first human anti-4-1BB antibody, a CDR2 from the light chain of a second human anti-4-1BB antibody and a CDR3 and CDR3 from the light chain of a third human anti-4-1BB antibody, and the CDRs from the heavy chain may be derived from one or more other anti-4-1BB antibodies. Further, the framework regions may be derived from one of the same anti-4-1BB antibodies or from one or more different human(s).

Moreover, as discussed previously herein, chimeric antibody includes an antibody comprising a portion derived from the germline sequences of more than one species.

By the term "effective amount", or "therapeutically effective amount," as used herein, is meant an amount that when administered to a mammal, preferably a human, mediates a detectable therapeutic response compared to the response detected in the absence of the compound. A therapeutic response, such as, but not limited to, inhibition of and/or decreased tumor growth, tumor size, metastasis, and the like, can be readily assessed by a plethora of art-recognized methods, including, e.g., such methods as disclosed herein.

The skilled artisan would understand that the effective amount of the compound or composition administered herein varies and can be readily determined based on a number of factors such as the disease or condition being treated, the stage of the disease, the age and health and physical condition of the mammal being treated, the severity of the disease, the particular compound being administered, and the like.

A "therapeutic effective amount", or "effective amount," is intended to qualify the amount of an agent required to detectably reduce to some extent one or more of the symptoms of a neoplasia disorder, including, but is not limited to: 1) reduction in the number of cancer cells; 2) reduction in tumor size; 3) inhibition (i.e., slowing to some extent, preferably stopping) of cancer cell infiltration into peripheral organs; 3) inhibition (i.e., slowing to some extent, preferably stopping) of tumor metastasis; 4) inhibition, to some extent, of tumor growth; 5) relieving or reducing to some extent one or more of the symptoms associated with the disorder; and/or 6) relieving or reducing the side effects associated with the administration of anticancer agents.

By the term "compete", as used herein with regard to an antibody, is meant that a first antibody, or an antigen-binding portion thereof, competes for binding with a second antibody, or an antigen-binding portion thereof, where binding of the first antibody with its cognate epitope is detectably decreased in the presence of the second antibody compared to the binding of the first antibody in the absence of the second antibody. The alternative, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope or ligand, whether to the same, greater, or lesser extent, the antibodies are said to "cross-compete" with each other for binding of their respective epitope(s). For instance, cross-competing antibodies can bind to the epitope, or portion of the epitope, to which the antibodies of the embodiments bind. Both competing and cross-competing antibodies are encompassed by the present embodiments. Regardless of the mechanism by which such competition or cross-competition occurs (e.g., steric hindrance, conformational change, or binding to a common epitope, or portion thereof, and the like), the skilled artisan would appreciate, based upon the teachings provided herein, that such competing and/or cross-competing antibodies are encompassed and can be useful for the methods disclosed herein.

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

"Instructional material," as that term is used herein, includes a publication, a recording, a diagram, or any other medium of expression which can be used to communicate the usefulness of the compound, combination, and/or composition of the invention in the kit for affecting, alleviating or treating the various diseases or disorders recited herein. Optionally, or alternately, the instructional material can describe one or more methods of alleviating the diseases or disorders in a cell, a tissue, or a mammal, including as disclosed elsewhere herein.

The instructional material of the kit may, for example, be affixed to a container that contains the compound and/or composition of the invention or be shipped together with a container which contains the compound and/or composition. Alternatively, the instructional material may be shipped separately from the container with the intention that the recipient uses the instructional material and the compound cooperatively. Except when noted, the terms "patient" or "subject" are used interchangeably and refer to mammals such as human patients and non-human primates, as well as veterinary subjects such as rabbits, rats, and mice, and other animals. Preferably, patient refers to a human.

The phrase "pharmaceutically acceptable salt(s)", as used herein, includes salts of acidic or basic groups which may be present in a compound. Compounds that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, such as the acetate, benzenesulfonate, benzoate, bicarbonate, bisulfate, bistosylate, bitartrate, borate, bromide, calcium edetate, camsylate, carbonate, chloride, clavulanate, citrate, dihydrochloride, edetate, edislyate, estolate, esylate, ethylsuccinate, fumarate, gluceptate, gluconate, glutamate, glycollylarsanilate, hexylresorcinate, hydrabamine, hydrobromide, hydrochloride, iodide, isothionate, lactate, lactobionate, laurate, malate, maleate, mandelate, mesylate, methylsulfate, mucate, napsylate, nitrate, oleate, oxalate, pamoate (embonate), palmitate, pantothenate, phosphate/diphosphate, polygalacturonate, salicylate, stearate, subacetate, succinate, tannate, tartrate, teoclate, tosylate, thiethiodode, and valerate salts. Preferred salts of compounds 1-3 are disclosed in PCT Publication No. 2003/016305, U.S. Patent Application No. 10/956,420, filed September 30, 2004, and PCT Application No. PCT/IB2004/003070, filed September 20, 2004, the disclosures of which are incorporated herein by reference in their entireties. Particularly preferred salts of compound 1 include malate salts, most preferably an L-malate salt. A particularly preferred salt of compound 3 is a maleate salt.

Conventional notation is used herein to portray polypeptide sequences: the lefthand end of a polypeptide sequence is the amino-terminus; the right-hand end of a polypeptide sequence is the carboxyl-terminus.

By the phrase "specifically binds," as used herein, is meant a compound, e.g., a protein, a nucleic acid, an antibody, and the like, which recognizes and binds a specific molecule, but does not substantially recognize or bind other molecules in a sample. For instance, an antibody or a peptide inhibitor which recognizes and binds a cognate ligand 4-1BB in a sample, but does not substantially recognize or bind other molecules in the sample. Thus, under designated assay conditions, the specified binding moiety (e.g., an antibody or an antigen-binding portion thereof) binds preferentially to a particular target molecule and does not bind in a significant amount to other components present in a test sample. A variety of assay formats may be used to select an antibody that specifically binds a molecule of interest. For example, solid-phase ELISA immunoassay, immunoprecipitation, BIAcore and Western blot analysis are used to identify an antibody that specifically reacts with 4-1BB. Typically a specific or selective reaction will be at least twice background signal or noise and more typically more than 10 times background, even more specifically, an antibody is said to "specifically bind" an antigen when the equilibrium dissociation constant (KD) is ≤ 1 µM, preferably ≤ 100 nM and most preferably ≤ 10 nM.

The term "KD" refers to the equilibrium dissociation constant of a particular antibody-antigen interaction.

The term "antibody dependent cellular cytotoxicity" or "ADCC" refers to a cell mediated reaction in which non-specific cytotoxic cells (e.g. NK cells, neutrophils, macrophages, etc.) recognize antibody bound on a target cell and subsequently cause lysis of the target cell. Such cytotoxic cells that mediate ADCC generally express Fc receptor: (FcR). The primary cells for mediating ADCC (NK cells) express FcγRIII, whereas monocytes express FcyRI, FcγRII, FcγRIII, and/or FcγRIV. To assess ADCC activity of a molecule, an *in vitro* ADCC assay, such as that described in U.S. Pat. Nos. 5,500,362 or 5,821,337 may be performed. Useful effector cells for such assays include peripheral blood mononuclear cells (PBMC) and Natural Killer (NK) cells. Alternatively, or additionally, ADCC activity of the molecules of interest may be assessed *in vivo,* e.g., in an animal model.

The term "ADCC-inducing antibody" refers to an antibody that demonstrates ADCC as measured by assay(s) known to those of skill in the art. Such activity is typically characterized by the binding of the Fc region with various FcRs. Without being limited by any particular mechanism, those of skill in the art will recognize that the ability of an antibody to demonstrate ADCC can be, for example, by virtue of it subclass (such as IgG1 or IgG3), by mutations introduced into the Fc region, or by virtue of modifications to the carbohydrate patterns in the Fc region of the antibody. Such modifications are described, for example, in U.S. Patent Publication No. 2007-0092521.

The antibody referred to herein as "rituximab" will be readily recognized by those skilled in the art, and is sold under the tradenames Rituxan® and MabThera®. Rituximab is a genetically engineered chimeric monoclonal antibody directed against the human CD20 antigen. This chimeric antibody contains a human IgG1 constant domain and is identified by the name "C2B8" in U.S. Pat. No. 5,736,137 (Andersen, K. C., et. al.) issued on Apr 17,1998. Rituximab is approved for the treatment of patients with relapsed or refracting low-grade or follicular, CD20 positive, B cell non-Hodgkin's lymphoma. In vitro mechanism of action studies have shown that rituximab exhibits human complement dependent cytotoxicity (CDC) as well as significant activity in assays that measure antibody-dependent cellular cytotoxicity (ADCC).

As used herein, "substantially pure" means an object species is the predominant species present (i.e., on a molar basis it is more abundant than any other individual species in the composition), and preferably a substantially purified fraction is a composition wherein the object species (e.g., an anti-4-1BB antibody) comprises at least about 50 percent (on a molar basis) of all macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

As used herein, to "treat" means reducing the frequency with which symptoms of a disease (i.e., tumor growth and/or metastasis, or other effect mediated by the numbers and/or activity of immune cells, and the like) are experienced by a patient. The term includes the administration of the compounds or agents of the present invention to prevent or delay the onset of the symptoms, complications, or biochemical indicia of a disease (e.g., elevation of PSA level), alleviating the symptoms or arresting or inhibiting further development of the disease, condition, or disorder. Treatment may be prophylactic (to prevent or delay the onset of the disease, or to prevent the manifestation of clinical or subclinical symptoms thereof) or therapeutic suppression or alleviation of symptoms after the manifestation of the disease.

"Combination therapy" embraces the administration of an ADCC-inducing antibody and a 4-1BB antibody as part of a specific treatment regimen intended to provide a beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" generally is not intended to encompass the administration of two or more of these therapeutic agents as part of separate monotherapy regimens that incidentally and arbitrarily result in the combinations of the present invention. "Combination therapy" embraces administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, both the therapeutic agents may be administered orally or both therapeutic agents may be administered by intravenous injection. The sequence in which the therapeutic agents are administered is not narrowly critical. "Combination therapy" also can embrace the administration of the therapeutic agents as described above in further combination with other biologically active ingredients (such as, but not limited to, a second and different antineoplastic agent) and non-drug therapies (such as, but not limited to, surgery or radiation treatment). Where the combination therapy further comprises radiation treatment, the radiation treatment may be conducted at any suitable time so long as a beneficial effect from the co-action of the combination of the therapeutic agents and radiation treatment is achieved. For example, in appropriate cases, the beneficial effect is still achieved when the radiation treatment is temporally removed from the administration of the therapeutic agents, perhaps by days or even weeks.

### Description

Embodiments disclosed herein relate to novel therapeutic methods comprising co-administering a combination of an anti-4-1BB antibody and an ADCC-inducing antibody (*e.g.,* an anti-CD20 such as rituximab or an anti-P-cadherin antibody) for the treatment of cancer in a patient in need of such treatment. In one embodiment, the method comprises administering an anti-4-1BB antibody (*e.g.,* MOR-7480.1) in combination with an anti-CD20 antibody (*e.g.,* rituximab). In another embodiment, the method comprises administering an anti-4-1BB antibody (*e.g.,* MOR-7480.1) in combination with an anti-P-cadherin antibody.

### I. Anti-4-1BB Antibodies

The preferred anti-4-1BB antibody is an antibody that specifically binds to human 4-1BB. Such antibodies include, but are not limited to, MOR-6032, MOR-7361, MOR-7480, MOR-7480.1, MOR-7480.2, MOR-7483, MOR-7483.1, and MOR-7483.2. These exemplary anti-4-1BB antibodies are described in Table 1 and Table 2 below.

**Table: 1. Index of SEQ ID NOs for Exemplary 4-1BB Antibodies**

| **Antibody** | **Chain** | **Full length** | | **Variable Region** | |
|---|---|---|---|---|---|
| | | **Amino Acid** | **Nucleotide** | **Amino Acid** | **Nucleotide** |
| | | **SEQ ID NO** | **SEQ ID NO** | **SEQ ID NO** | **SEQ ID NO** |
| MOR-6032 | Heavy | 5 | 13 | 4 | 11 |
| | Light | 10 | 14 | 9 | 12 |
| MOR-7361 | Heavy | 19 | 27 | 18 | 25 |
| | Light | 24 | 28 | 23 | 26 |
| MOR-7480 | Heavy | 33 | 41 | 32 | 39 |
| | Light | 38 | 42 | 37 | 40 |
| MOR-7480.1 | Heavy | 44 | 49 | 43 | 47 |
| | Light | 46 | 50 | 45 | 48 |
| MOR-7480.2 | Heavy | 44 | 49 | 43 | 47 |
| | Light | 52 | 54 | 51 | 53 |
| MOR-7483 | Heavy | 33 | 41 | 32 | 39 |
| | Light | 57 | 59 | 56 | 58 |
| MOR-7483.1 | Heavy | 44 | 49 | 43 | 47 |
| | Light | 61 | 63 | 60 | 62 |
| MOR-7483.2 | Heavy | 44 | 49 | 43 | 47 |
| | Light | 65 | 67 | 64 | 66 |

**Table 2: Amino Acid Sequence of CDRs for Exemplary 4-1BB Antibodies**

| **Antibody** | **CDR** | **Sequence** | **SEQ ID NO** |
|---|---|---|---|
| MOR-6032 | H-CDR1 | NSYAIS | 1 |
| | H-CDR2 | GIIPGFGTANYAQKFQG | 2 |
| | H-CDR3 | RKNEEDGGFDH | 3 |
| | L-CDR1 | SGDNLGDYYAS | 6 |
| | L-CDR2 | DDSNRPS | 7 |
| | L-CDR3 | QTWDGTLHFV | 8 |
| MOR-7361 | H-CDR1 | SDYYMH | 15 |
| | H-CDR2 | VISGSGSNTYYADSVKG | 16 |
| | H-CDR3 | RLYAQFEGDF | 17 |
| | L-CDR1 | SGDNIGSKYVS | 20 |
| | L-CDR2 | SDSERPS | 21 |
| | L-CDR3 | QSWDGS-ISRV | 22 |
| MOR-7480; MOR-7480.1; MOR-7480.2 | H-CDR1 | STYWIS | 29 |
| | H-CDR2 | KIYPGDSYTNYSPSFQG | 30 |
| | H-CDR3 | RGYGIFDY | 31 |
| | L-CDR1 | SGDNIGDQYAH | 34 |
| | L-CDR2 | QDKNRPS | 35 |
| | L-CDR3 | ATYTGFGSLAV | 36 |
| MOR-7483; MOR-7483.1; MOR-7483.2 | H-CDR1 | STYWIS | 29 |
| | H-CDR2 | KIYPGDSYTNYSPSFQG | 30 |
| | H-CDR3 | RGYGIFDY | 31 |
| | L-CDR1 | SGDNIGDQYAH | 34 |
| | L-CDR2 | QDKNRPS | 35 |
| | L-CDR3 | STYTFVGFTTV | 55 |

The amino and nucleic acid sequences of MOR-6032, MOR-7361, MOR-7480, MOR-7480.1, MOR-7480.2, MOR-7483, MOR-7483.1, and MOR-7483.2 are set forth herein. Briefly, the anti-4-1BB antibodies of the embodiments include antibodies having amino acid sequences of the heavy and light chains of an antibody such as, but not limited to, MOR-6032, MOR-7361, MOR-7480, MOR-7480.1, MOR-7480.2, MOR-7483, MOR-7483.1, and MOR-7483.2. Embodiments disclosed herein also relate to antibodies having the amino acid sequences of the CDRs of the heavy and light chains of these antibodies. Further embodiments concern anti-4-1BB antibodies having the variable regions of the heavy and light chains of those antibodies. In another embodiment, the antibody is selected from an antibody having the full length, variable region, or CDR, amino acid sequences of the heavy and light chains of antibodies MOR-6032, MOR-7361, MOR-7480, MOR-7480.1, MOR-7480.2, MOR-7483, MOR-7483.1, and MOR-7483.2. In some embodiments, the anti-4-1BB antibody can comprise one or more of the sequences described above with one or more conservative substitutions. In other embodiments, the anti-4-1BB antibody can be an antibody that cross-competes with MOR-6032, MOR-7361, MOR-7480, MOR-7480.1, MOR-7480.2, MOR-7483, MOR-7483.1, or MOR-7483.2.

In another embodiment, the methods described herein are practiced using an anti-4-1BB antibody that comprises a heavy chain comprising the amino acid sequences of H-CDR1, H-CDR2, and H-CDR3, and a light chain comprising the amino acid sequences of L-CDR1, L-CDR2, and L-CDR3, of an antibody selected from the group consisting of MOR-6032, MOR-7361, MOR-7480, MOR-7480.1, MOR-7480.2, MOR-7483, MOR-7483.1, or MOR-7483.2, or sequences having changes from said CDR sequences selected from the group consisting of conservative changes, wherein the conservative changes are selected from the group consisting of replacement of nonpolar residues by other nonpolar residues, replacement of polar charged residues other polar uncharged residues, replacement of polar charged residues by other polar charged residues, and substitution of structurally similar residues; non-conservative substitutions, wherein the non-conservative substitutions are selected from the group consisting of substitution of polar charged residue for polar uncharged residues and substitution of nonpolar residues for polar residues, additions and deletions.

In a further embodiment, the anti-4-1BB antibody contains fewer than 10, 7, 5, or 3 amino acid changes from the germline sequence in the framework or CDR regions. In another embodiment, the antibody contains fewer than 5 amino acid changes in the framework regions and fewer than 10 changes in the CDR regions. In one preferred embodiment, the antibody contains fewer than 3 amino acid changes in the framework regions and fewer than 7 changes in the CDR regions. In a preferred embodiment, the changes in the framework regions are conservative and those in the CDR regions are somatic mutations.

In another embodiment, the anti-4-1BB antibody has at least 80%, more preferably, at least 85%, even more preferably, at least 90%, yet more preferably, at least 95%, more preferably, at least 99%, sequence identity over the heavy and light chain CDR-1, CDR-2 and CDR-3 sequences with the CDR sequences of MOR-6032, MOR-7361, MOR-7480, MOR-7480.1, MOR-7480.2, MOR-7483, MOR-7483.1, or MOR-7483.2. Even more preferably, the antibody shares 100% sequence identity over the heavy and light chain CDR-1, CDR-2 and CDR-3 with the CDR sequences of MOR-6032, MOR-7361, MOR-7480, MOR-7480.1, MOR-7480.2, MOR-7483, MOR-7483.1, or MOR-7483.2.

In yet another embodiment, the anti-4-1BB antibody has at least 80%, more preferably, at least 85%, even more preferably, at least 90%, yet more preferably, at least 95%, more preferably, at least 99%, sequence identity over the heavy and light chain variable region sequences with the variable region sequences of MOR-6032, MOR-7361, MOR-7480, MOR-7480.1, MOR-7480.2, MOR-7483, MOR-7483.1, or MOR-7483.2. Even more preferably, the antibody shares 100% sequence identity over the heavy and light chain variable region sequences with the variable region sequences of MOR-6032, MOR-7361, MOR-7480, MOR-7480.1, MOR-7480.2, MOR-7483, MOR-7483.1, or MOR-7483.2.

### II. ADCC Inducing Antibodies

Embodiments disclosed herein encompass the use of any ADCC-inducing antibody. The preferred ADCC-inducing antibodies include, but are not limited to, anti-CD20 antibodies (such as rituximab) and P-cadherin antibodies (such as g-194-g09). Suitable P-cadherin antibodies are disclosed, for example, in U.S. Patent No. 7,452,537, which is hereby incorporated by reference. However, the skilled artisan would appreciate that a variety of ADCC-inducing antibodies could be used in the embodiments described herein. For example, suitable antibodies can be engineered to have enhanced ADCC-inducing activity (see, for example, Lazar et al., PNAS 2006; 103; 4005-4010; Bowles et al., Blood 2006; 108:2648-2654; Shields et al., JBC 2002; 277:26733-26740; Suzuki et al., Clin Cancer Res 2007; 13(6); and Satoh et al., Expert Opin. Biol. Ther. 2006; 6(11):1161-1173). Thus, for example, anti-CD20 antibodies, such as rituximab, could be engineered according to methods known in the art to produce ADCC-antibodies suitable for use in the embodiments disclosed herein. Similarly, anti-P-cadherin antibodies, such as g-194-g09 (described herein), could be engineered according to methods known in the art to produce ADCC-antibodies suitable for use in the embodiments disclosed herein.

Embodiments disclosed herein also relate to antibodies having the amino acid sequences of the CDRs of the heavy and light chains of rituximab and g-194-g09. In other embodiments, the ADCC-inducing antibody is selected from an antibody having the full length, variable region, or CDR, amino acid sequences of the heavy and light chains of rituximab or g-194-g09.

While the anti-4-1BB antibodies and ADCC-inducing antibodies discussed herein may be preferred, the skilled artisan, based upon the disclosure provided herein, would appreciate that the embodiments encompass a wide variety of anti-4-1BB antibodies and ADCC-inducing antibodies and is not limited to the particular antibodies disclosed herein. More particularly, while human antibodies are typically preferred, the embodiments are in no way limited to human antibodies; rather, the embodiments encompass useful antibodies regardless of species origin, and includes, among others, chimeric humanized and/or primatized antibodies. The embodiments include anti-4-1BB antibodies and ADCC-inducing antibodies produced by any method, including, but not limited to, a method known in the art (e.g., screening phage display libraries, and the like) or to be developed in the future for producing an anti-4-1BB antibody of the invention.

The present embodiments encompass human antibodies produced using a transgenic non-human mammal, i.e., XenoMouse™ (Abgenix, Inc., Fremont, CA) as disclosed in the U.S. 6,682,736, to Hanson et al.

Another transgenic mouse system for production of "human" antibodies is referred to as "HuMAb-MouseTM" (Medarex, Princeton, NJ), which contain human immunoglobulin gene miniloci that encodes unrearranged human heavy (mu and gamma) and kappa light chain immunoglobulin sequences, together with targeted mutations that inactivate the endogenous mu and kappa chain loci (Lonberg et al. Nature 368:856-859 (1994), and U.S. Pat. No. 5,770,429).

However, the embodiments encompass antibodies produced using any transgenic mammal such as, but not limited to, the Kirin TC Mouse™ (Kirin Beer Kabushiki Kaisha, Tokyo, Japan) as described in, e.g., Tomizuka et al., Proc Natl Acad Sci USA 97:722 (2000); Kuroiwa et al., Nature Biotechnol 18:1086 (2000); U.S. Patent Application Publication No. 2004/0120948, to Mikayama et al.; and the HuMAb-Mouse™ (Medarex, Princeton, NJ) and XenoMouse™ (Abgenix, Inc., Fremont, CA), supra. Thus, the invention encompasses using an antibody produced using any transgenic or other non-human animal.

In another embodiment, the antibodies employed in methods disclosed herein are not fully human, but "humanized". In particular, murine antibodies or antibodies from other species can be "humanized" or "primatized" using techniques well known in the art. See, e.g., Winter and Harris Immunol. Today 14:43-46 (1993), Wright et al. Crit. Reviews in Immunol. 12:125-168 (1992), and US Patent No. 4,816,567, to Cabilly et al, and Mage and Lamoyi in Monoclonal Antibody Production Techniques and Applications pp. 79-97, Marcel Dekker, Inc., New York, NY (1987).

As will be appreciated based upon the disclosure provided herein, antibodies for use herein can be obtained from a transgenic non-human mammal, and hybridomas derived therefrom, but can also be expressed in cell lines other than hybridomas.

Mammalian cell lines available as hosts for expression are well known in the art and include many immortalized cell lines available from the American Type Culture Collection (ATCC), including but not limited to Chinese hamster ovary (CHO) cells, NSO (also referred to as NS0), HeLa cells, baby hamster kidney (BHK) cells, monkey kidney cells (COS), and human hepatocellular carcinoma cells (e.g., Hep G2). Non-mammalian prokaryotic and eukaryotic cells can also be employed, including bacterial, yeast, insect, and plant cells.

Various expression systems can be used as well known in the art, such as, but not limited to, those described in e.g., Sambrook and Russell, Molecular Cloning, A Laboratory Approach, Cold Spring Harbor Press, Cold Spring Harbor, NY (2001), and Ausubel et al., Current Protocols in Molecular Biology, John Wiley & Sons, NY (2002). These expression systems include dihydrofolate reductase (DHFR)-based systems, among many others. The glutamine synthetase system of expression is discussed in whole or part in connection with European Patents Nos. EP 216 846, EP 256 055, and EP 323 997 and European Patent Application 89303964. In one embodiment, the antibody used is made in NS0 cells using a glutamine synthetase system (GS-NS0). In another embodiment, the antibody is made in CHO cells using a DHFR system. Both systems are well-known in the art and are described in, among others, Barnes et al. Biotech & Bioengineering 73:261-270 (2001), and references cited therein.

Site directed mutagenesis of the antibody CH2 domain to eliminate glycosylation may be preferred in order to prevent changes in either the immunogenicity, pharmacokinetic, and/or effector functions resulting from non-human glycosylation. Further, the antibody can be deglycosylated by enzymatic (see, e.g., Thotakura et al. Meth. Enzymol. 138:350 (1987)) and/or chemical methods (see, e.g., Hakimuddin et al., Arch. Biochem. Biophys. 259:52 (1987)).

Further, the embodiments encompass using an antibody comprising an altered glycosylation pattern. The skilled artisan would appreciate, based upon the disclosure provided herein, that an antibody can be modified to comprise additional, fewer, or different glycosylations sites compared with the naturally-occurring antibody. Such modifications are described in, e.g., U.S. Patent Application Publication Nos. 2003/0207336, and 2003/0157108, and International Patent Publication Nos. WO 01/81405 and 00/24893.

Additionally, some embodiments comprise using one or more antibodies regardless of the glycoform, if any, present on the antibody. Moreover, methods for extensively remodeling the glycoform present on a glycoprotein are well-known in the art and include, e.g., those described in International Patent Publication Nos. WO 03/031464, WO 98/58964, and WO 99/22764, and US Patent Application Publication Nos. 2004/0063911, 2004/0132640, 2004/0142856, 2004/0072290, and US Patent No. 6,602,684 to Umaña et al.
Further, the embodiments encompass using an antibody with any art-known covalent and non-covalent modification, including, but not limited to, linking the polypeptide to one of a variety of nonproteinaceous polymers, e.g., polyethylene glycol, polypropylene glycol, or polyoxyalkylenes, in the manner set forth in, for example, U.S. Patent Application Publication Nos. 2003/0207346 and 2004/0132640, and U.S. Pat. Nos. 4,640,835; 4,496,689; 4,301,144; 4,670,417; 4,791,192; 4,179,337.

Additionally, the embodiments encompass using an antibody, or antigen-binding portion thereof, chimeric protein comprising, e.g., a human serum albumin polypeptide, or fragment thereof. Whether the chimeric protein is produced using recombinant methods by, e.g., cloning of a chimeric nucleic acid encoding the chimeric protein, or by chemical linkage of the two peptide portions, the skilled artisan would understand once armed with the teachings provided herein that such chimeric proteins are well-known in the art and can confer desirable biological properties such as, but not limited to, increased stability and serum half-life to the antibody of the invention and such molecules are therefore included herein.

Antibodies that are generated for use in the invention need not initially possess a particular desired isotype. Rather, the antibody as generated can possess any isotype and can be isotype switched thereafter using conventional techniques. These include direct recombinant techniques (see, e.g., U.S. Patent 4,816,397), and cell-cell fusion techniques (see e.g., U.S. Patent No. 5,916,771).

The effector function of the antibodies of the invention may be changed by isotype switching to an IgG1, IgG2, IgG3, IgG4, IgD, IgA, IgE, or IgM for various therapeutic uses. Furthermore, dependence on complement for cell killing can be avoided through the use of bispecifics, immunotoxins, or radiolabels, for example.

Therefore, the present embodiments are not limited in any way to the antibodies specifically disclosed herein, or any other, particular antibodies. The invention encompasses combining administration of any anti-4-1BB antibody with any ADCC-inducing antibody. Preferably, the anti-4-1BB antibody is MOR-7480.1 and the ADCC-inducing antibody is rituximab. However, any anti-4-1BB antibody or ADCC-inducing antibody, or antigen-binding portion thereof, as described elsewhere herein, or as known in the art or developed in the future, can be used in a method described herein.

### III. Combination Therapy with Anti-4-1BB Antibodies and Antibodies Capable of Inducing ADCC

Embodiments disclosed herein relate to combination therapy comprising co-administering an anti-4-1BB antibody (e.g., MOR-6032, MOR-7361, MOR-7480, MOR-7480.1, MOR-7480.2, MOR-7483, MOR-7483.1, orMOR-7483.2) and at least one ADCC-inducing antibody (*e.g*., an anti-CD20 such as rituximab or an anti-P-cadherin antibody).

In one embodiment, a combination of an anti-4-1BB antibody (*e.g*., MOR-7480.1) and an ADCC-inducing antibody is co-administered to a patient to treat cancer. The combination can be useful for treatment of, among other things, abnormal cell growth, for example, hematological malignancies, B-cell malignancies, non-Hodgkin's lymphoma, CD20-positive non-Hodgkin's lymphoma, mesothelioma, hepatobiliary (hepatic and billiary duct), a primary or secondary CNS tumor, a primary or secondary brain tumor, lung cancer (NSCLC and SCLC), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), breast cancer (e.g., triple negative breast cancer), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, non-Hodgkin's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

In some embodiments, a combination of an anti-4-1BB antibody (*e.g*., MOR-7480.1) and rituximab is co-administered to a patient to treat hematological malignancies, B-cell malignancies, non-Hodgkin's lymphoma, or CD20-positive non-Hodgkin's lymphoma.

In some embodiments, a combination of an anti-4-1BB antibody (*e.g*., MOR-7480.1) and anti-P-cadherin antibody is co-administered to a patient to treat breast cancer (*e.g*., triple negative breast cancer).

Furthermore, the invention encompasses use of an anti-4-1BB antibody in combination with at least one ADCC-inducing antibody (*e.g*., an anti-CD20 such as rituximab or an anti-P-cadherin antibody) as a neoadjuvant, adjuvant, first line treatment, second-line and/or third-line therapy for cancer (*e.g*., adjuvant therapy for breast cancer, first line therapy for metastatic lung cancer, third line therapy for germ cell tumors, and the like).

In one embodiment, the combination of the invention is administered in further combination with a standard of care therapy for one of the cancers described above. In another embodiment, the combination is administered to a patient who has failed standard of care therapy.

The skilled artisan would appreciate, once provided the teachings disclosed herein, that the methods disclosed herein can be used with, or sequentially (preceding or following) with surgery, radiotherapy, or both, to treat cancer. That is, various treatments can be combined with the antibody combination therapy, as would be understood by one skilled in the art once armed with the teachings provided herein.

In another embodiment, an anti-4-1BB antibody in combination with at least one ADCC-inducing antibody (*e.g*., an anti-CD20 such as rituximab or an anti-P-cadherin antibody) is co-administered to enhance, prolong, or both, an immune response to a tumor. This is because there may be an interaction between the anti-tumor effect of the anti-4-1BB antibody and the ADCC-inducing antibody that leads to more effective anti-tumor effect than either antibody alone. Thus, without wishing to be bound by any particular theory, the combination of the anti-4-1BB antibody and the ADCC-inducing antibody can induce a more robust immunological response within the tumor than expected. Therefore, the combination of the anti-4-1BB antibody and the ADCC-inducing antibody can provide a potential additive or synergistic effect thereby providing an important novel therapeutic treatment for cancer.

In certain embodiments, the ADCC-inducing antibody (*e.g*., an anti-CD20 such as rituximab or an anti-P-cadherin antibody) may enhance the effects of the anti-4-1BB antibody in an additive manner. In a preferred embodiment, the ADCC-inducing antibody (*e.g.,* an anti-CD20 such as rituximab or an anti-P-cadherin antibody) enhances the effects of the anti-4-1BB antibody in a synergistic manner. In another embodiment, the anti-4-1BB antibody enhances the effect of an ADCC-inducing antibody in an additive manner. Preferably, the effects are enhanced in a synergistic manner. Thus, in certain embodiments, the invention encompasses methods of disease treatment or prevention that provide better therapeutic profiles than administration of the ADCC-inducing antibody (*e.g.,* an anti-CD20 such as rituximab or an anti-P-cadherin antibody) alone and/or anti-4-1BB antibody alone.

Encompassed by the present embodiments are combination therapies that have additive potency or an additive therapeutic effect while reducing or avoiding unwanted or adverse effects. The invention also encompasses synergistic combinations where the therapeutic efficacy is greater than additive, while unwanted or adverse effects are reduced or avoided. In certain embodiments, the methods of the invention permit treatment or prevention of diseases and disorders wherein treatment is improved by an enhanced anti-tumor response using lower and/or less frequent doses of anti-4-1BB antibody and/or ADCC-inducing antibody to reduce the incidence of unwanted or adverse effects caused by the administration of anti-4-1BB antibody and/or ADCC-inducing antibody alone, while maintaining or enhancing efficacy of treatment, preferably increasing patient compliance, improving therapy and/or reducing unwanted or adverse effects.

The methods and compositions of the invention are useful not only in untreated patients but are also useful in the treatment of patients partially or completely unresponsive to the ADCC-inducing antibody administered alone or anti-4-1BB antibody administered alone. Various embodiments provide methods and compositions useful for the treatment of diseases or disorders in patients that have been shown to be or may be refractory or non-responsive to therapies comprising the administration of either or both anti-4-1BB antibody and/or ADCC-inducing antibodies, and wherein treatment is improved by an enhanced immune response. In one embodiment, the method comprises combining an anti-CD20 antibody (preferably, rituximab) and an anti-4-1BB antibody (preferably, antibody MOR-7480.1).

The antibodies can be administered in doses, compositions, by dosage regimens, and by administration routes described herein. The skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a patient can also be determined, as can the temporal requirements for administering each agent to provide a detectable therapeutic benefit to the patient. Accordingly, while certain dose and administration regimens are exemplified herein, these examples in no way limit the dose and administration regimen that can be provided to a patient in practicing the present invention. Further, one skilled in the art would understand, once armed with the teachings provided herein, that a therapeutic benefit, such as, but not limited to, detectable decrease in tumor size and/or metastasis, decreased level of PSA in prostate cancer, and increased time to recurrence, among many other parameters, can be assessed by a wide variety of methods known in the art for assessing the efficacy of treatment of cancer, and these methods are encompassed herein, as well as methods to be developed in the future.

Some embodiments disclosed herein relate to neoadjuvant, adjuvant, first-line and/or second-line therapy comprising administering a combination of antibodies. Other embodiments encompass use of the combination along the entire disease and treatment continuum. More specifically, the novel methods disclosed herein can provide a therapeutic benefit before and after metastasis, as well as to patients that have become refractory to a chemotherapeutic agent, in that the antibody combination can enhance an immune response, including any response mediated by therapy. The data disclosed herein suggest that immunotherapy comprising an anti-4-1BB antibody in combination with at least one ADCC-inducing antibody (e.g., an anti-CD20 such as rituximab or an anti-P-cadherin antibody) can provide a therapeutic benefit either alone or combined with at least one additional agent, at any point during treatment. Indeed, the data disclosed herein further suggest that a synergistic effect is mediated by combined administration of an anti-4-1BB antibody and at least one ADCC-inducing antibody (e.g., an anti-CD20 such as rituximab or an anti-P-cadherin antibody) for treatment of cancer. Therefore, the present embodiments provide important novel therapeutics for treatment of cancer whereby the patient's immune system is enhanced to provide an anti-tumor effect.

### IV. Additional Combination Therapy

Based upon the disclosure provided herein, including the combined additive or synergistic effect of co-administering an anti-4-1BB antibody in combination with an ADCC-inducing antibody (*e.g*., an anti-CD20 such as rituximab or an anti-P-cadherin antibody), it would be appreciated by the skilled artisan that the invention encompasses numerous combination therapies wherein the antibodies are administered to the patient in combination with at least one other therapeutic agent thereby providing a therapeutic benefit. Although many such combinations will be readily apparent to one skilled in the art once armed with the teachings provided herein, several combinations are discussed herein. However, the embodiments disclosed herein are in no way limited to these combinations, which are set forth herein merely for illustrative purposes.

Co-administration of the antibodies with an additional therapeutic agent encompasses co-administering the anti-4-1BB antibody, an ADCC-inducing antibody (*e.g*., an anti-CD20 such as rituximab or an anti-P-cadherin antibody), and one or more additional therapeutic agents, and also encompasses co-administering two or more separate pharmaceutical compositions, one comprising the anti-4-1BB antibody and the other(s) comprising the ADCC-inducing antibody, and other(s) comprising at least one additional therapeutic agent. Further, although co-administration or combination (conjoint) therapy generally mean that the antibodies and additional therapeutic agents are administered at the same time as one another, it also encompasses simultaneous, sequential or separate dosing of the individual components of the treatment. Additionally, where an antibodies are administered intravenously and the additional therapeutic agent(s) is administered orally, or by subcutaneous or intramuscular injection, it is understood that the combination is preferably administered as two, three, or more separate pharmaceutical compositions.

When a mammal is subjected to additional chemotherapy, chemotherapeutic agents well-known in the art can be used in combination with the methods described herein. Additionally, growth factor inhibitors, biological response modifiers, alkylating agents, intercalating antibiotics, vinca alkaloids, immunomodulators, taxanes, selective estrogen receptor modulators (SERMs), such as, but not limited to, lasofoxifene, angiogenesis inhibitors, among many therapeutic agents, some of which are described below, can be used.

### Angiogenesis inhibitors

An angiogenesis inhibitor can be used in the methods described herein. An angiogenesis inhibitor includes, but is not limited to, bevacizumab (AVASTIN; Genentech), a humanized antibody to VEGF. It can be used in combination with 5FU, and is indicated as a first-line treatment of patients with metastatic carcinoma of the colon or rectum. Agents that directly target angiogenic factors or their receptors offer the prospect for greater activity in receptor-competent hematologic malignancies by interrupting autocrine receptor signaling. Bevacizumab produces sustained neutralization of circulating VEGF and may be useful for treatment of myelodysplastic syndrome (MDS), lymphoma, acute myeloid leukemia (AML), and solid tumors. RTKI small molecule inhibitors of angiogenic receptor signaling (*e.g*., indolinone) are encompassed in the embodiments described herein. The first receptor antagonist to enter clinical testing in hematologic malignancies is SU5416 (Sugen), which impairs ligand-induced autophosphorylation of the VEGFR-1 and VEGFR-2 receptors and c-Kit. SU5416 inhibits VEGF-induced clonogenic response in leukemia cell lines and promotes apoptosis in myeloblasts from AML patients. Other RTKIs, including PTK787/ZK222584 (Novartis), and AG-13736 (Agouron/Pfizer), are being assessed to treat AML and other receptor-competent hematologic malignancies. The embodiments disclosed herein also include treatment of cancer, e.g., lymphomas, colon carcinomas, renal carcinoma, gastrointestinal stromal tumors, and the like, using a combination of an anti-4-1BB antibody, at least one ADCC-inducing antibody (e.g., an anti-CD20 such as rituximab or an anti-P-cadherin antibody), and at least one additional angiogenesis inhibitor, e.g., AG-13736, AG-26,798, and the like, as well as other angiogenesis inhibitors that are well-known in the art or developed in the future.

Thus, anti-angiogenesis agents, such as MMP-2 (matrix-metalloproteinase 2) inhibitors, MMP-9 (matrix-metalloproteinase 9) inhibitors, and COX-II (cyclooxygenase II) inhibitors, can be used in the methods describe herein. Examples of useful COX-II inhibitors include CELEBREXTM (celecoxib), valdecoxib, rofecoxib, parecoxib, deracoxib, SD-8381, ABT-963, etoricoxib, lumiracoxib, BMS-347070, NS-398, RS 57067, meloxicam. Examples of useful matrix metalloproteinase inhibitors are described in International Patent Publication Nos. WO 96/33172; WO 96/27583; WO 98/07697, WO 98/03516, WO 98/34918, WO 98/34915, WO 98/33768, WO 98/30566, WO 90/05719, WO 99/52910, WO 99/52889, WO 99/29667, European Patent Application Nos. 780386 (published June 25, 1997), 97304971.1 (filed July 8, 1997), 99308617.2 (filed October 29, 1999), 606046 (published July 13, 1994), 931788 (published July 28, 1999), 99302232.1 (filed March 25, 1999), International Application PCT/IB98/01113 (filed July 21, 1998), Great Britain patent application number 9912961.1 (filed June 3, 1999), United States Provisional Patent Application No. 60/148,464 (filed August 12, 1999), and U.S. Patent Nos. 5,863,949, and 5,861,510.

Preferred MMP-2 and MMP-9 inhibitors are those that have little or no activity inhibiting MMP-1. More preferred are those that selectively inhibit MMP-2 and/or MMP-9 relative to the other matrix-metalloproteinases (i.e. MMP-1, MMP-3, MMP-4, MMP-5, MMP-6, MMP-7, MMP-8, MMP-10, MMP-11, MMP-12, and MMP-13).

### Signal transduction inhibitors

The treatments described herein can also be used with signal transduction inhibitors other, such as agents that can inhibit EGFR (epidermal growth factor receptor) responses, such as EGFR antibodies, EGF antibodies, and molecules that are EGFR inhibitors; VEGF (vascular endothelial growth factor) inhibitors, such as VEGF receptors and molecules that can inhibit VEGF; and erbB2 receptor inhibitors, such as organic molecules or antibodies that bind to the erbB2 receptor, for example, HERCEPTIN (Genentech, Inc., San Francisco, CA).

EGFR inhibitors are described in, for example in International Patent Publication Nos. WO 95/19970, WO 98/14451, WO 98/02434, and U.S. Patent No. 5,747,498, and such substances can be used in the present invention as described herein. EGFR-inhibiting agents include, but are not limited to, the monoclonal antibodies C225 (ERBITUX), anti-EGFR 22Mab (ImClone Systems Inc., New York, NY), and ABX-EGF (panitumumab, Abgenix Inc., Fremont, CA), the compounds ZD-1839 (AstraZeneca), BIBX-1382 (Boehringer Ingelheim), MDX-447 (Medarex,Inc., Annandale, NJ), and OLX-103 (Merck & Co., Whitehouse Station, NJ), VRCTC-310 (Ventech Research) and EGF fusion toxin (Seragen Inc., Hopkinton, MA). These and other EGFR-inhibiting agents can be used in the present embodiments.

Compounds directed at inhibition of epidermal growth factor receptor (EGFR) tyrosine kinase (TK) represent a relatively new class of antineoplastic drugs that are useful in the method of the present invention. Many human cancers express members of the EGFR family on the cell surface. When a ligand binds to EGFR, it sets off a cascade of cellular reactions that result in increased cell division and influence other aspects of cancer development and progression, including angiogenesis, metastatic spread, and inhibition of apoptosis. EGFR-TK inhibitors may selectively target one of the members of the EGFR family (EGFR (also known as HER1 or ErbB-1), HER2/neu (also known as ErbB-2), HER3 (also known as ErbB-3), or HER4 (also known as ErbB-4)), or may target two or more of them. EGFR-TK inhibitors suitable for use in the present invention include gefitinib (IRESSA), erlotinib (TARCEVA), CI-1033 (Pfizer), GW2016 (GlaxoSmithKline), EKB-569 (Wyeth), PKI-166 (Novartis), CP-724,714 (Pfizer), and BIBX-1382 (Boeringer-Ingelheim). Additional EGFR-TK inhibitors are described in United States Patent Application No. 09/883,752, filed June 18, 2001.
VEGF inhibitors, for example SU-5416 and SU-6668 (Sugen Inc., San Francisco, CA), can also be employed in combination with the methods described herein. VEGF inhibitors are described for example in International Patent Application No. PCT/IB99/00797 (filed May 3, 1999), International Patent Publication Nos. WO 99/24440; WO 95/21613; WO 99/61422; WO 98/50356; WO 99/10349; WO 97/32856; WO 97/22596; WO 98/54093; WO 98/02438; WO 99/16755; WO 98/02437; U.S. Patent Nos. 5,834,504; 5,883,113; 5,886,020; and 5,792,783. Other examples of some specific VEGF inhibitors useful in the present invention are IM862 (Cytran Inc., Kirkland, WA); IMC-1C11 Imclone antibody, anti-VEGF monoclonal antibody of Genentech, Inc., San Francisco, CA; and angiozyme, a synthetic ribozyme from Ribozyme (Boulder, CO) and Chiron (Emeryville, CA).

ErbB2 receptor inhibitors, such as GW-282974 (Glaxo Wellcome pic), and the monoclonal antibodies AR-209 (Aronex Pharmaceuticals Inc., Woodlands, TX) and 2B-1 (Chiron), can furthermore be combined with the methods described herein, for example those indicated in International Patent Publication Nos. WO 98/02434; WO 99/35146; WO 99/35132; WO 98/02437; WO 97/13760; WO 95/19970; U.S. Patent Nos. 5,587,458, and 5,877,305. ErbB2 receptor inhibitors useful in the present embodiments are also described in EP1029853 (published August 23, 2000) and in International Patent Publication No. WO 00/44728, (published August 3, 2000). The erbB2 receptor inhibitor compounds and substance described in the aforementioned PCT applications, U.S. patents, and U.S. provisional applications, as well as other compounds and substances that inhibit the erbB2 receptor, can be used with the antibodies in accordance with the present embodiments.

The treatments described herein also be used with other agents useful in treating abnormal cell growth or cancer, including, but not limited to other agents capable of enhancing antitumor immune responses; and anti-proliferative agents such as farnesyl protein transferase inhibitors (e.g., BMS 214662), and αvβ3 inhibitors, such as the αvβ3 antibody VITAXIN, αvβ5 inhibitors, p53 inhibitors, and the like.

Where the antibodies of the methods described herein are administered in combination with another immunomodulatory agent, the immunomodulatory agent can be selected for example from the group consisting of a dendritic cell activator such as CD40 ligand and anti-CD40 agonist antibodies, as well as enhancers of antigen presentation, enhancers of T-cell tropism, inhibitors of tumor-related immunosuppressive factors, such as TGF-β (transforming growth factor beta), and IL-10. Preferred anti-CD40 agonist antibodies encompass antibodies disclosed in International Patent Application No. PCT/US02/36107, filed November 8, 2002 (published as WO 03/040170 on May 15, 2003), and U.S. Patent Application No. 10/292,088, filed November 8, 2002 (published as U.S. Patent Publication No. US2003/0211100 on Nov. 13, 2003), including, but not limited to, an antibody having the heavy and light chain amino acid sequence of antibody 3.1.1, 3.1.1.H-A78T, 3.1.1H-A78T-V88A-V97A, 3.1.1L-L4M-L83V, 3.1.1H-A78T-V88A-V97A/3.1.1L-L4M-L83V, 7.1.2, 10.8.3, 15.1.1, 21.2.1, 21.4.1, 22.1.1, 22.1.1H-C109A, 23.5.1, 23.25.1, 23.28.1, 23.28.1H-D16E, 23.29.1, and 24.2.1.

### IGF-1R inhibitors

The present treatment regimens may also be combined with antibodies or other ligands that inhibit tumor growth by binding to IGF-1R (insulin-like growth factor 1 receptor). Example of anti-IGF-1R antibodies that can be used include those described in International Patent Application No. PCT/US01/51113, filed 12/20/01 (published as WO 02/053596 on July 11, 2002), and International Patent Application No. PCT/IB2004/002555, filed August 3, 2004 (published as WO 2005/016967 on Feb. 24, 2005). Preferred anti-IGFR-1R antibodies encompass an antibody having the heavy and light chain amino acid sequence of, e.g., antibody 2.12.1, 2.13.2, 2.14.3, 3.1.1, 4.9.2 and 4.17.3.

Ligands that inhibit signaling via the IGF-1R also encompass small molecules, and other ligands including, inter alia, somavert (PEGVISOMANT), which is a growth hormone analog that inhibits IGF-1 signaling. PEGVISOMANT is conjugated with polyethylene glycol and can be used, among other things, to treat acromegaly. PEGVISOMANT can be co-administered with antibodies of the methods described herein to treat cancer in that the combination can inhibit tumor growth. Thus, PEGVISOMANT, similarly with anti-IGF-1R antibodies, can be used to treat cancer as disclosed herein.

The present embodiments encompass therapies that can be further combined with a wide plethora of therapeutic, surgical, radiation, and other therapeutics, to treat a patient. Therapeutic agents are numerous and have been described in, for instance, U.S. Patent Application Publication No. 2004/0005318, No. 2003/0086930, No. 2002/0086014, and International Publication No. WO 03/086459, all of which are incorporated by reference herein, among many others. Such therapeutic agents include, but are not limited to, topoisomerase I inhibitors; other antibodies (trastuzumab, anti-IGF-1R, and the like); chemotherapeutic agents such as, but not limited to, imatinib (GLEEVEC, GLIVEC, or STI571; Novartis), sorafenib (BAY 43-9006; Bayer Pharmaceuticals Corp./Onyx Pharmaceuticals), selective estrogen receptor modulators (SERMs), taxanes, vinca alkaloids, temozolomide, angiogenesis inhibitors, EGFR inhibitors, VEGF inhibitors, ErbB2 receptor inhibitors, anti-proliferative agents (e.g., farnesyl protein transferase inhibitors, and αvβ3 inhibitors, αvβ5 inhibitors, p53 inhibitors, and the like), immunomodulators, cytokines, tumor vaccines; tumor-specific antigens; dendritic and stem cell therapies; alkylating agents, folate antagonists; pyrimidine antagonists; anthracycline antibiotics; platinum compounds; costimulatory molecules (e.g., CD4, CD25, PD-1, B7-H3, 4-1BB, OX40, ICOS, CD30, HLA-DR, MHCII, and LFA).

### Radiotherapy

Radiation therapy can be co-administered with the antibody combination therapies described herein. Radiotherapy is administered in accordance to well-known radiotherapy methods for treatment of cancer, such as breast cancer. The dose and regimen for radiotherapy can be readily determined by one skilled in the art and is based on the stage of the disease, and other factors well-known in the art.

### Palliative agents

The present embodiments also encompass the administration of other therapeutic agents. Such therapeutic agents include analgesics, cancer vaccines, anti-vascular agents, anti-proliferative agents, anti-emetic agents, and anti-diarrheal agents. Preferred anti-emetic agents include ondansetron hydrochloride, granisetron hydrochloride, and metoclopramide. Preferred anti-diarrheal agents include diphenoxylate and atropine (LOMOTIL), loperamide (IMMODIUM), and octreotide (SANDOSTATIN).

In another embodiment, the methods described herein include administering an agent with anti-diarrheal effect wherein the agent is indicated in the treatment of chronic inflammatory conditions of the gastrointestinal tract. Such agents include, among others, steroids with topical activity (e.g., budesonide [ENTOCORT]), and anti-tumor necrosis factor (TNF) drugs (e.g., infliximab [REMICADE], etanercept [ENBREL], and adalimumab [HUMIRA]).

### Stem cell-based therapy

The antibody combination therapy disclosed herein can be combined with stem cell transplantation to provide a therapeutic benefit to a patient afflicted with cancer. Stem cell transplantation may be performed according to the methods known in the art. Some such methods are described in Appelbaum in Harrison's Principles of Internal Medicine, Chapter 14, Braunwald et al., Eds., 15th ed., McGraw-Hill Professional (2001), which is hereby incorporated herein by reference. Thus, the methods of the present invention relate to the treatment of cancer in a mammal who has undergone stem cell transplantation, which methods comprise administering to the mammal an amount of an anti-4-1BB antibody in combination with at least one ADCC-inducing antibody (e.g., an anti-CD20 such as rituximab or an anti-P-cadherin antibody), which is effective in treating the cancer in further combination with stem cell transplantation.

Where the method comprises stem cell transplant, the first dose of the antibody combination therapy can be administered after the immune system of the mammal has recovered from transplantation, for example, in the period of from one to 12 months post transplantation. In certain embodiments, the first dose is administered in the period of from one to three, or one to four months post transplantation. The patient may undergo stem cell transplantation and preparatory treatment(s).

The methods described herein also relate to methods for the treatment of cancer in a mammal comprising the steps of (i) performing stem cell transplantation in the mammal, and (ii) administering an effective amount of a anti-4-1BB antibody in combination with an effective amount of at least one ADCC-inducing antibody. Preferably, the mammal is a human. Stem cell transplantation may be allogeneic or autologous stem cell transplantation. Further, cell transplantation encompasses adoptive transfer of lymphocytes, either from the same patient and/or from a HLA-matched donor.

Further, the methods of the invention can be combined with radiation therapy and stem cell transplant, and any combination of any of the treatments described herein, known in the art, or to be developed in the future.

Where the antibody combination treatment is combined with a standard cancer treatment, such as, inter alia, chemotherapeutic regimes, it may be possible to reduce the dose of chemotherapeutic reagent administered (Mokyr, M. et al. Cancer Research 58: 5301-5304 (1998)). The combination therapies disclosed herein can provide an increased source of tumor-specific antigens thereby providing an increased immune response to the tumor which, in turn, provides a therapeutic benefit to the patient.

### V. Dosage Regimens

For administration of one or more antibodies described herein, dosage regimens can be adjusted to provide the optimum desired response. For example, a single bolus can be administered, several divided doses can be administered over time or the dose may be proportionally reduced or increased as indicated by the exigencies of the therapeutic situation. It can be especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are typically dictated by and directly dependent on (a) the unique characteristics of the antibody and the particular therapeutic or prophylactic effect to be achieved, and (b) the limitations inherent in the art of compounding such an active compound for the treatment of sensitivity in individuals.

For administration of antibodies or combinations of antibodies, the dosage can range from about 0.0001 to 100 mg/kg, and more usually 0.01 to 20 mg/kg, of the host body weight. An exemplary, non-limiting range for a therapeutically effective amount of an antibody or combination of antibodies administered according to the methods described herein is at least about 0.01 mg/kg, at least about 0.1 mg/kg, at least about 0.3 mg/kg, at least about 1 mg/kg, at least about 5 mg/kg, at least about 6 mg/kg, at least about 10 mg/kg, at least about 15 mg/kg, or at least about 20 mg/kg. For example, a therapeutically effective amount of antibody or combination of antibodies can range from about 0.01-15 mg/kg, or for example about 0.03-3 mg/kg, or for example about 0.1-1 mg/kg. In addition, a therapeutically effective amount of antibody can range from about 0.1-30 mg/kg, or for example about 0.3-25 mg/kg, or for example about 1-20 mg/kg, or for example about 3-20 mg/kg, or for example about 5-20 mg/kg, or for example about 10-20 mg/kg, or about 3-15 mg/kg, or about 5-15 mg/kg, or about 10-15 mg/kg.

Further, an exemplary dose escalation protocol can be used to determine the maximum tolerated dose (MTD), to assess dose limiting toxicity (DLT), if any, associated with administration of the combination therapy described herein, and the like, comprises administering increasing doses, such as, but not limited to about 0.01 mg/kg, 0.03 mg/kg, 0.06 mg/kg, 0.1 mg/kg, 0.12 mg/kg, 0.18 mg/kg, 0.24 mg/kg, 0.3 mg/kg, 1 mg/kg, 3 mg/kg, 6 mg/kg, 7 mg/kg, 10 mg/kg, 12 mg/kg, 15 mg/kg, or more than 15 mg/kg, or any combination thereof. In some embodiments, successive doses of 0.1 mg/kg, 0.3 mg/kg, 1 mg/kg, 3 mg/kg, 6 mg/kg, 10 mg/kg, 15 mg/kg or 20 mg/kg are administered and the patient is assessed for toxicity, if any, as well as for efficacy of treatment, among other parameters. In some embodiments, successive doses of 0.03 mg/kg, 0.06 mg/kg, 0.12 mg/kg, 0.18 mg/kg, 0.24 mg/kg, and 0.3 mg/kg, are administered and the patient is assessed for toxicity, if any, as well as for efficacy of treatment, among other parameters. Such studies to determine toxicity and efficacy of dose regimens are well-known in the art.

It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. Determining appropriate dosages and regimens for administration of the antibody are well-known in the relevant art and would be understood to be encompassed by the skilled artisan once provided the teachings disclosed herein.

In one embodiment, the antibodies are administered in an intravenous formulation as a sterile aqueous solution containing about 5 to 20 mg/ml of antibodies, in an appropriate buffer system.

In one embodiment, part of the dose is administered by an intraveneous bolus and the rest by infusion of the antibody formulation. For example, an intravenous injection of antibody may be given as a bolus, and the rest of a predetermined antibody dose may be administered by intravenous injection. A predetermined dose of the antibodies may be administered, for example, over a period of about an hour and a half to about five hours.

Some embodiments disclosed herein relate to administering a combination of an anti-4-1BB antibody and at least one ADCC-inducing antibody (e.g., an anti-CD20 such as rituximab or an anti-P-cadherin antibody). The skilled artisan would appreciate that the combination can be administered simultaneously or the antibodies and various agents can be administered at different times. However, the present embodiments are not limited to these or any particular dosage or administration regimens for administering an anti-4-1BB antibody in combination with at least one ADCC-inducing antibody (e.g., an anti-CD20 such as rituximab or an anti-P-cadherin antibody). Rather, the optimal dose, route and regimen for administration of the antibodies can be readily determined by one of ordinary skill in the relevant art using well-known methods.

For instance, a single dose or multiples doses of each antibody (or combination of antibodies) may be administered. Alternatively, at least one dose, or at least three, six or 12 doses may be administered. The doses may be administered, for example, daily, weekly, every two weeks, monthly, every twenty days, every 25 days, every 28 days, every 30 days, every 40 days, every 50 days, every two months, every 70 days, every 80 days, every three months, every six months or yearly, or any other period that provides a therapeutic benefit to the patient as determined by the skilled practitioner.

In one embodiment, a single bolus injection comprising the anti-4-1BB antibody is administered to a patient intravenously at a dose ranging from about 0.1 mg/kg to 20 mg/kg approximately every twenty-eight days. A dose of an ADCC-inducing antibody is administered on that first day. In some embodiments, the antibodies are co-administered on the same starting day of each dose cycle. In other embodiments, the ADCC-inducing antibody is administered at any point during administration of the anti-4-1BB antibody, or vice-α-versa, and the invention is not limited in any way with respect to the relative administration of the antibodies. Thus, the ADCC-inducing antibody can be administered either before, during and/or after administration of the anti-4-1BB antibody.

The antibody combination can be administered as a neoadjuvant therapy prior to surgery, radiation therapy, or any other treatment, in order to sensitize the tumor cells or to otherwise confer a therapeutic benefit to the patient. Additionally, the combination can be co-administered as neoadjuvant therapy following localized treatment (e.g., surgery, radiation, or both).

Further, the combination can be administered as a second line therapy, such as, but not limited to, once first line therapy has failed. Alternatively, the combination can be administered concurrently with first line therapy, and or at any point during first line therapy, which can be administered following initial treatment.

The methods described herein encompass administration of a antibody combination, with or without additional therapy, including, but not limited to, hormonal, radiotherapy, and any additional therapeutic agent (chemotherapy, signal inhibition therapy, among others), and the like, as would be appreciated by one skilled in the art based upon the disclosure provided herein.

The invention also relates to an article of manufacture (e.g., dosage form adapted for i.v. administration) comprising an anti-4-1BB antibody in the amount effective to treat cancer (e.g., at least 0.01 mg/kg, at least 0.03 mg/kg, at least 0.06 mg/kg, at least 0.1 mg/kg, at least 0.12 mg/kg, at least 0.18 mg/kg, at least 0.24 mg/kg, at least 0.3 mg/kg, at least 0.5 mg/kg, at least 0.7 mg/kg, at least 1 mg/kg, at least 3 mg/kg, at least 5 mg/kg, at least 10 mg/kg, at least 15 mg/kg, or at least 20 mg/kg) and a therapeutically effective amount of at least one ADCC-inducing antibody. In certain embodiments, the article of manufacture comprises a container or containers comprising a anti-4-1BB antibody, at least one ADCC-inducing antibody (e.g., an anti-CD20 such as rituximab or an anti-P-cadherin antibody), and a label and/or instructions for use to treat cancer.

### VI. Pharmaceutical Compositions

Embodiments disclosed herein encompass the preparation and use of pharmaceutical compositions comprising a human anti-4-1BB antibody of the invention as an active ingredient in combination with at least one ADCC-inducing antibody (e.g., an anti-CD20 such as rituximab or an anti-P-cadherin antibody). Such a pharmaceutical composition may consist of each active ingredient alone, as a combination of at least one active ingredient (e.g., an effective dose of an anti-4-1BB antibody, an effective dose of at least one ADCC-inducing antibody such as rituximab) in a form suitable for administration to a subject, or the pharmaceutical composition may comprise the active ingredient and one or more pharmaceutically acceptable carriers, one or more additional (active and/or inactive) ingredients, or some combination of these.

In some embodiments, one or more antibodies are administered parenterally (e.g., intravenously) in an aqueous solution. In other embodiments, one or more antibodies are administered orally in pill/capsule form. However, the skilled artisan would understand, based upon the disclosure provided herein, that the invention is not limited to these, or any other, formulations, doses, routes of administration, and the like. Rather, the invention encompasses any formulation or method of administering an anti-4-1BB antibody in combination with an ADCC-inducing antibody, including, but not limited to, administering each agent separately in a different formulation via a different route of administration (e.g., administering one antibody i.v., while co-administering the other antibody orally), among many others. Thus, the following discussion describes various formulations for practicing the methods described herein comprising administration of any anti-4-1BB antibody in combination with an ADCC-inducing antibody, but the present embodiments are not limited to these formulations, but comprises any formulation as can be readily determined by one skilled in the art once armed with the teachings provided herein for use in the described methods.

The antibodies employed in the embodiments described herein can be incorporated into pharmaceutical compositions suitable for administration to a subject. Typically, the pharmaceutical composition comprises the antibody and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, trehalose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable substances such as wetting or minor amounts of auxiliary substances such as wetting or emulsifying agents, preservatives or buffers, which enhance the shelf life or effectiveness of the antibody or antibody portion.

The antibodies may be in a variety of forms. These include, for example, liquid, semi solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. The preferred mode of administration is parenteral (e.g., intravenous, subcutaneous, intraperitoneal, intramuscular). In a preferred embodiment, the antibody is administered by intravenous infusion or injection. In another preferred embodiment, the antibody is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the antibody in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze drying that yields a powder of the active ingredient plus any additional desired ingredient from a previously sterile filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The antibodies can be administered by a variety of methods known in the art, including, without limitation, oral, parenteral, mucosal, by-inhalation, topical, buccal, nasal, and rectal. For many therapeutic applications, the preferred route/mode of administration is subcutaneous, intramuscular, intravenous or infusion. Non-needle injection may be employed, if desired. As will be appreciated by the skilled artisan, the route and/or mode of administration will vary depending upon the desired results.

Dosage regimens may be adjusted to provide the optimum desired response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the antibody and the particular therapeutic or prophylactic effect to be achieved, and (b) the limitations inherent in the art of compounding such an active compound for the treatment of sensitivity in individuals.

It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

In one embodiment, one or more antibodies is administered in an intravenous formulation as a sterile aqueous solution containing 5 or 10 mg/ml of antibody, with sodium acetate, polysorbate 80, and sodium chloride at a pH ranging from about 5 to 6. Preferably, the intravenous formulation is a sterile aqueous solution containing 5 or 10 mg/ml of antibody, with 20 mM sodium acetate, 0.2 mg/ml polysorbate 80, and 140 mM sodium chloride at pH 5.5.

In another embodiment of the invention, one or more antibodies is administered in a sterile solution comprising 20 mM histidine buffer, pH 5.5, 84 mg/ml trehalose dihydrate, 0.2 mg/ml polysorbate 80, and 0.1 mg/ml disodium ethylenediaminetetraacetic acid dihydrate. In one aspect, the formulation is packaged in clear glass vials with a rubber stopper and an aluminum seal. In another aspect, the vial contains about 20 mg/ml of antibody with a nominal fill of about 400 mg per vial.

In one embodiment, part of the dose is administered by an intraveneous bolus and the rest by infusion of the antibody formulation. For example, a 0.01 mg/kg intravenous injection of the antibody may be given as a bolus, and the rest of a predetermined antibody dose may be administered by intravenous injection. A predetermined dose of the antibody may be administered, for example, over a period of an hour and a half to two hours to five hours.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multidose unit.

A pharmaceutical composition of the invention may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition will vary, depending upon the identity, size, and condition of the subject treated and further depending upon the route by which the composition is to be administered. By way of example, the composition may comprise between 0.1% and 100% (w/w) active ingredient.

In addition to the active ingredient, a pharmaceutical composition of the invention may further comprise one or more additional pharmaceutically active agents. Particularly contemplated additional agents include anti-emetics, anti-diarrheals, chemotherapeutic agents, cytokines, and the like.

Controlled- or sustained-release formulations of a pharmaceutical composition of the invention may be made using conventional technology.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi dose containers containing a preservative. Formulations for parenteral administration include, but are not limited to, suspensions, solutions, emulsions in oily or aqueous vehicles, pastes, and implantable sustained-release or biodegradable formulations as discussed below. Such formulations may further comprise one or more additional ingredients including, but not limited to, suspending, stabilizing, or dispersing agents. In one embodiment of a formulation for parenteral administration, the active ingredient is provided in dry (i.e. powder or granular) form for reconstitution with a suitable vehicle (e.g. sterile pyrogen free water) prior to parenteral administration of the reconstituted composition.

A composition of the present invention can be administered by a variety of methods known in the art. The route and/or mode of administration vary depending upon the desired results. The active ingredients can be prepared with carriers that protect the active ingredient against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are described by e.g., Sustained and Controlled Release Drug Delivery Systems, J. R. Robinson, ed., Marcel Dekker, Inc., New York, (1978). Pharmaceutical compositions are preferably manufactured under GMP conditions.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non toxic parenterally acceptable diluent or solvent, such as water or 1,3 butane diol, for example. Other acceptable diluents and solvents include, but are not limited to, Ringer's solution, isotonic sodium chloride solution, and fixed oils such as synthetic mono or di-glycerides. Other parentally-administrable formulations which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt.

The anti-4-1BB antibody/ADCC-inducing antibody active ingredient combination can be administered to an animal, preferably a human. The precise dosage administered of each active ingredient will vary depending upon any number of factors, including but not limited to, the type of animal and type of disease state being treated, the age of the animal and the route(s) of administration.

The anti-4-1BB antibody may be administered to an animal as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as, but not limited to, the type and severity of the disease being treated, the type and age of the animal, etc.

The ADCC-inducing antibody (e.g., an anti-CD20 such as rituximab or an anti-P-cadherin antibody) can be administered to an animal as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as, but not limited to, the ADCC-inducing antibody itself, as well as the type and severity of the disease being treated, the type and age of the animal, etc.

The anti-4-1BB antibody and ADCC-inducing antibody (e.g., an anti-CD20 such as rituximab or an anti-P-cadherin antibody) can be co-administered in that they can be administered separately, on different dates or at different times of the day, as well as simultaneously or on the same date. Co-administration thus encompasses any temporal combination of administration of the antibodies such that administration of the two mediates a therapeutic benefit to the patient that is detectably greater than administration of either agent in the absence of the other.

An anti-4-1BB antibody and ADCC-inducing antibody combination may be co-administered with numerous other compounds (antihormonal therapy agents, cytokines, chemotherapeutic and/or antiviral drugs, among many others). Alternatively, the compound(s) may be administered an hour, a day, a week, a month, or even more, in advance of the anti-4-1BB antibody and ADCC-inducing antibody combination, or any permutation thereof. Further, the compound(s) may be administered an hour, a day, a week, or even more, after administration of radiation, stem cell transplant, or administration of any therapeutic agent (e.g., cytokine, chemotherapeutic compound, and the like), or any permutation thereof. The frequency and administration regimen will be readily apparent to the skilled artisan and will depend upon any number of factors such as, but not limited to, the type and severity of the disease being treated, the age and health status of the animal, the identity of the compound or compounds being administered, the route of administration of the various compounds, and the like. Several instructive examples demonstrating methods of co-administering an anti-4-1BB antibody and ADCC-inducing antibody to treat cancer are provided, but the invention is not limited in any way to these examples, which merely serve to illustrate methods encompassed by the invention.

### VII. Kits

Further embodiments disclosed herein include various kits for treatment of cancer. The kits typically include a therapeutically effective amount of a human anti-4-1BB antibody of the invention and a therapeutically effective amount of at least one ADCC-inducing antibody (e.g., an anti-CD20 such as rituximab or an anti-P-cadherin antibody). The kit can further include an applicator, including, but not limited to, a syringe, for administration of the components of the kit to a patient. In addition, the kit can include instructional materials setting forth the pertinent information for the use of the kit to treat cancer in the patient. Further, the kit can include a wide plethora of additional agents for treatment of cancer. Such agents are set forth herein and include chemotherapeutic compounds, cancer vaccines, signal transduction inhibitors, agents useful in treating abnormal cell growth or cancer, antibodies or other ligands that inhibit tumor growth, a chemotherapeutic agent (taxane, vinca alkaloid, platinum compound, intercalating antibiotics, among many others), and cytokines, among many others, as well as palliative agents to treat, e.g., any toxicities that arise during treatment such as, but not limited to, an anti-diarrheal, an anti-emetic, and the like. Although exemplary kits are described herein, the contents of other useful kits will be apparent to the skilled artisan in light of the present disclosure.

In some embodiments, the kit comprises at least one anti-4-1BB antibody selected from MOR-6032, MOR-7361, MOR-7480, MOR-7480.1, MOR-7480.2, MOR-7483, MOR-7483.1, MOR-7483.2. In some embodiment, the kit comprises MOR-7480.1 and rituximab. In further embodiments, the kit comprises MOR-7480.1 and anti-P-cadherin1. In additional embodiments, the kit comprises MOR-7480.1 and anti-P-cadherin2. While such kits described above are preferred, the invention is not limited to these particular combinations.

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

### Examples

### Example 1: Combinatorial Effects of ADCC inducing mAb (anti-CD20 mAb) with 4-1BB agonist mAb (MAB9371) in a Transplantable Tumor Model in Mice

The combinatorial efficacy of 4-1BB and CD20 surrogate mAbs in a lymphoma model was tested. Tumor growth inhibition experiments were conducted in the A20 B cell lymphoma model, which expresses the B cell protein CD20, testing the efficacy of rat-anti-mouse 4-1BB agonist mAb (MAB9371 (R&D Systems (Minneapolis MN)) in combination with a mouse-anti-mouse CD20 antibody which served as a rituximab surrogate.

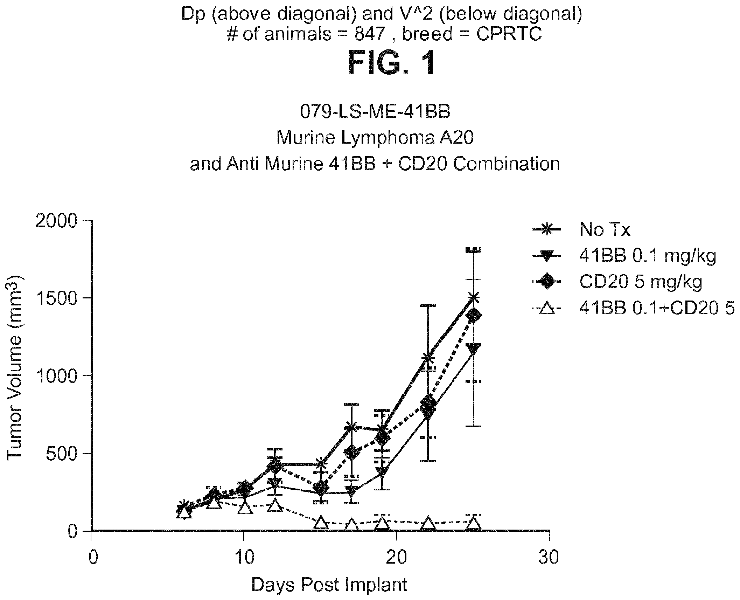

One million A20 cells were injected subcutaneously on the right flank of Balb/c mice. The tumors were allowed to grow for 7 days and the animals were randomized based upon tumor volume. A single dose of MAB9371, anti-mouse CD20 or MAB9371 in combination with anti-mouse CD20 was delivered i.p. on the day of randomization. MAB9371 was dosed sub-optimally at 0.1 mg/kg or in combination with anti-mouse CD20 at 5 mg/kg diluted in PBS. Improved tumor growth inhibition was obtained when both MAB9371 and CD20 were used together. Tumor size ([length x {width x width}] x 0.5 = volume in mm3) was assessed every 2-3 days using a digital caliper. Mean ± SEM is shown in Figure 1.

Treatment with the combination had increased the efficacy of tumor growth inhibition as compared to either CD20 or anti 4-1BB alone. In addition, the required dosage of 4-1BB in the combination group was reduced and achieved better tumor growth inhibition than dosage with anti 4-1BB alone (Figure 1).

### Example 2: Combinatorial Effects of ADCC inducing mAb (anti-P-cadherin1 mAb (g-194-g09)) with 4-1BB agonist mAb (MAB9371) in a Colon Carcinoma Model in Mice

The combinatorial efficacy of 4-1BB and P-cadherin mAbs in a colon carcinoma model was tested. Tumor growth inhibition experiments were conducted, testing the efficacy of rat-anti-mouse 4-1BB agonist mAb (MAB9371; R&D Systems (Minneapolis MN)) in combination with an anti-P-cadherin antibody (anti-P-cadherin1 or g-194-g09).

One million CT-26 cells were injected subcutaneously on the right flank of Balb/c mice. The tumors were allowed to grow for 7 days and the animals were randomized based upon tumor volume. A single dose of anti-murine 4-1BB (MAB9371), and anti P-cadherin1 or MAB9371 in combination with anti-P-cadherin1 MAB9371 was dosed sub-optimally at 0.1 mg/kg or in combination with anti-P-cadherin1 at 10 mg/kg diluted in PBS. Animals were dosed i.p. on the day of randomization. Tumor growth inhibition was improved by approximately 35% when both MAB9371 and anti P-cadherin1 (g-194-g09) were used together.

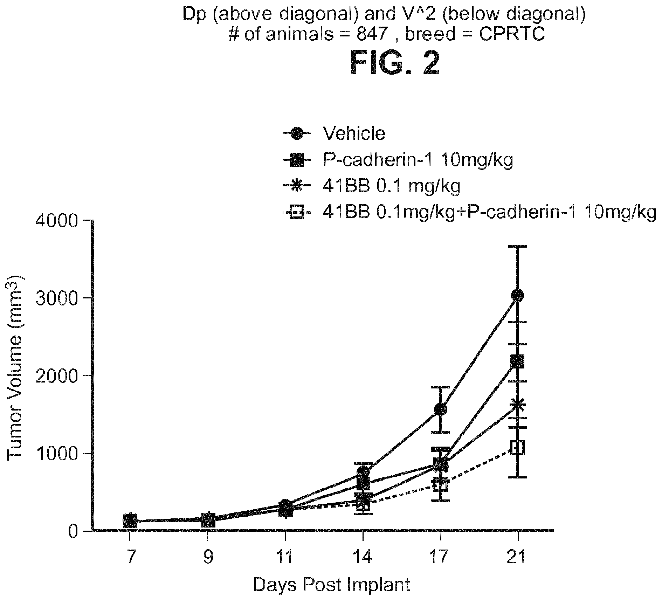

The results demonstrate that 4-1BB mAb (MAB9371), when used in combination with the anti P-cadherins mAb (g-194-g09), improved tumor growth inhibition (Figure 2).

### Example 3: Combinatorial Effects of ADCC inducing mAb (anti-P-cadherin2 mAb) with 4-1BB agonist mAb (MAB9371) in a Colon Carcinoma Model in Mice

The combinatorial efficacy of 4-1BB and P-cadherin mAbs in a colon carcinoma model was tested. Tumor growth inhibition experiments were conducted, testing the efficacy of rat-anti-mouse 4-1BB agonist mAb (MAB9371; R&D Systems (Minneapolis MN)) in combination with an anti-P-cadherin antibody (anti-P-cadherin2). Anti-P-cadherin2 was engineered from anti-P-cadherin1 (g-194-g09) to have enhanced ADCC-inducing activity.

One million CT-26 cells were injected subcutaneously on the right flank of Balb/c mice. The tumors were allowed to grow for 7 days and the animals were randomized based upon tumor volume. A single dose of anti-murine 4-1BB (MAB9371) and anti P-cadherin ADCC enhanced (anti-P-cadherin2) or MAB9371 in combination with anti P-cadherin ADCC enhanced was dosed sub-optimally at 0.1 mg/kg or in combination with anti P cadherin2 at 10 mg/kg diluted in PBS. Animals were dosed i.p. on the day of randomization. Tumor growth inhibition was improved by approximately 35% when both MAB9371 and anti P-cadherin2 were used together.

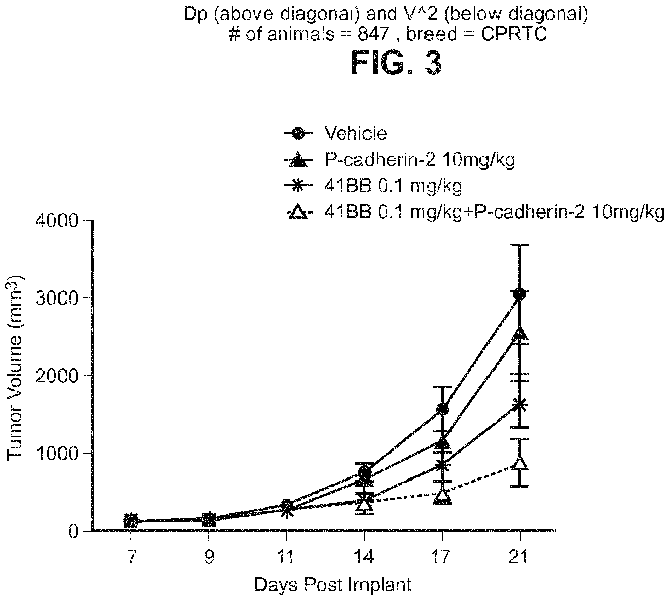

The results demonstrate 4-1BB mAb (MAB9371) when used in combination with the anti P-cadherin2 mAb, significantly improved tumor growth inhibition (Figure 3).

### Example 4: Treatment of Primary Lymphoma Positive Animals with 4-1BB Agonist Antibody and/or CD20 ADCC Inducing Antibody

The combinatorial efficacy of 4-1BB and CD20 mAbs were tested in Eµ-myc mice.

Eµ-myc (C57BL/6J-Tg(IghMyc)22Bri/J) mice were obtained (Jackson Laboratories) and blood samples monitored weekly by FACS. Using established enrollment criteria, animals were randomly assigned to 6 treatment groups; 1 mg/kg rat IgG2a control qwx2 (Group 1), 1 mg/kg MAB9371 qwx2 (Group 2), 10 mg/kg anti-mouse CD20 qwx4 (Group 3), 1 mg/kg MAB9371 qwx2 + 10 mg/kg anti-mouse CD20 qwx4 (Group 4), 0.1 mg/kg MAB9371 qwx2 + 10 mg/kg anti-mouse CD20 qwx4 (Group 5), and 1 mg/kg MAB9371 qwx1 + 10 mg/kg anti-mouse CD20 qwx4 (Group 6). Animals continued to be monitored via weekly saphenous vein bleeds and subsequent FACS analysis along with gross observation and body weight monitoring. Animals demonstrating overt signs of disease were euthanized according to humane practices, tissues from groups 1-4 were collected for histopathology, and survival post dosing was recorded.

A summary of the groups is depicted Table 3.

**Table 3. Summary Table; Eµ-myc Study**

| **Group** | **Treatment** | **Median Age @ Day 0 [weeks ±SD (range)]** | **Median Survival [days ±SD (range)]** | **Group1 Comparison P value summary** | **Group 3 Comparison P value summary** |
|---|---|---|---|---|---|
| Group 1, n=9 | 1mpk rat IgG2a qwx2 | 15.7 ±5.5 (7.3-25) | 13 ±11 (4-39) | | ns |
| Group 2, n=10 | 1mpk MAB9371 qwx2 | 10.9 ±9.5 (6.3-38.4) | 23.5 ±14.5 (11-63) | ns | *p = 0.0227 |
| Group 3, n=10 | 10mpk anti-CD20 qwx4 | 12.9 ±5.5 (7.9-25.3) | 15 ±5.8 (5-22) | ns | |
| Group 4, n=10 | 1 mpk MAB9371 qwx2 + 10mpk anti-CD20 qwx4 | 14.9 ±7.4 (8.4-32.9) | 34.5 ±46.1 (25-175) | **p = 0.0014 | ***p <0.0001 |
| Group 5, n=10 | 0.1 mpk MAB9371 qwx2 + 10mpk anti-CD20 qwx4 | 11.9 ±4.2 (9.4-22.7) | 25.5 ±21.1 (6-57) | ns | ns |
| Group 6, n=10 | 1mpk MAB9371 qwx1 + 10mpkanti-CD20 qwx4 | 16.6 ±7.4 (8.7-32.4) | 25 ±12.2 (11-56) | ns | **p = 0.0056 |

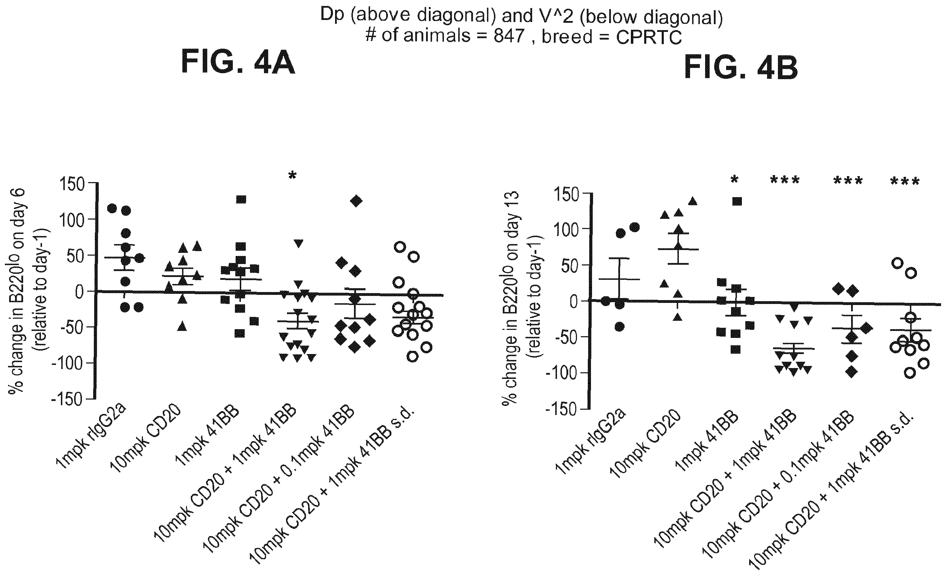

The median age at enrollment was 13.6 ± 6.7 weeks for the entire study with no significant difference in ages between the 6 groups. Significant survival benefit was observed for Group 4 (1 mg/kg MAB9371 qwx2 + 10 mg/kg anti-mouse CD20 qwx4) versus Group 1 (1 mg/kg rat IgG2a control qwx2, Table 1) and Group 3 (10 mg/kg anti-mouse CD20 qwx4, (Figure 4, Table 1).

Peripheral blood samples were stained with fluorochrome labeled Abs to B220, CD20, CD3, IgM, and IgD. Samples were analyzed by flow cytometry using a FACS Canto and FACS Diva software followed by data analysis using FlowJo software. Animals demonstrating an increase in B220lo+ population above 25% of the total lymphocytes combined with an increase in forward scatter and a week to week variation in B220lo+ of >5% were randomly assigned to treatment groups. The day that animals were assigned to the group is considered study day 0 (n=10 animals/group). Dosing was based on an average body weight of 22.5 grams/animal. Animals continued to be monitored via flow cytometry, body weight, and gross observation. Animals demonstrating overt signs of advanced lymphoma were euthanized according to humane practices. Survival of the test groups was compared to Group 1 or Group 3 and significance of the survival plots determined using a Log-rank (Mantel-Cox) test using Prism Graph software.

Animals were treated on day 0 (Figure 4A) or day 0 and day 7 (Figure 4B) with the indicated antibody or antibody combination at the indicated concentration and assessed via flow cytometry on day 6 (Figure 4A) or day 13 (Figure 4B). The exception was that animals in panel B group "10mpk CD20+1 mpk 4-1BB s.d." received CD20 antibody on days 0 and 7 but 4-1BB only on day 0. The data is represented as intra-animal change in the percent of B220 low cells relative to the day prior to enrollment (day -1) and is calculated using the following formula ((%B220low cells on indicated study day - %B220low cells day prior to enrollment)/ %B220low cells at day prior to enrollment)*100). Statistical significance of the treatment groups vs. the 10mpk CD20 group was determined using 1 way ANOVA analysis and Dunnet's Multiple Comparison Test using Graph Pad Prism software; n≥5/group *p<0.05, *** p<0.005.

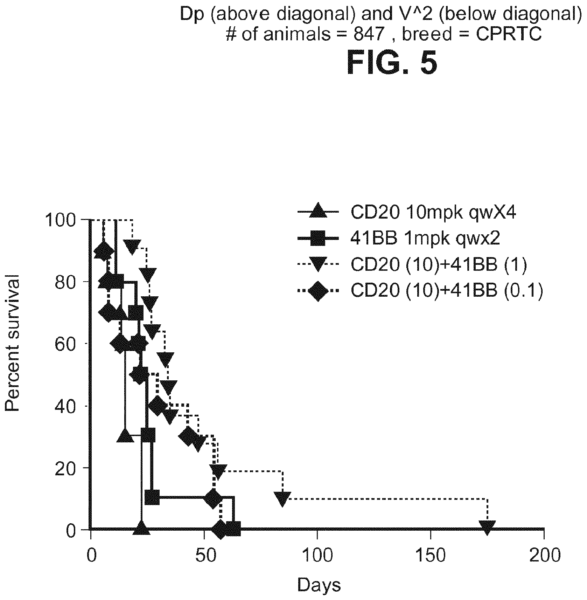

Animals were treated with 10mg/kg CD20 weekly for 4 weeks, 1mg/kg 4-1BB weekly for 2 weeks or a combination of CD20 and the indicated 4-1BB concentration according to the indicated schedule. Survival days post treatment of the two groups is shown in Figure 5. Median survival was calculated using Graph Pad Prism CD20=15 days, 1mpk 4-1BB =23.5 days, CD20+0.1mpk 4-1BB=25.5 days, CD20+1mpk 4-1BB= 34 days. Statistical significance of the 4-1BB treatment or combination treatment versus CD20 group was determined using a Log Rank (Mantel-Cox) test using Graph Pad Prism software, CD20 vs. 4-1BB *p<0.025, CD20 vs. CD20 + 0.1mpk 4-1BB p= not significant, CD20 vs. CD20 + 1mpk 4-1BB ***□□□p<0.0001.

The results indicate that treatment of animals with MAB9371 alone demonstrated a stabilization of disease by FACS analysis. However, significant reduction of circulating tumor was observed for all combination dose groups following treatment (Figure 4).

### RAW SEQUENCE LISTING

(In amino acid sequences, CDRs are underlined, variable regions in upper case, and constant regions in lower case)

### A. ANTIBODY MOR-6032

**Amino Acid Sequence of V_{H} (SEQ ID NO.: 4):** 
QVQLVQSGAEVKKPGSSVKVSCKASGGTFNSYAISWVRQAPGQGLEWMGGIIP
GFGTANYAQKFQGRVTITADESTSTAYMELSSLRSEDTAVYYCARKNEEDGGFDHWG
QGTLVTVSS
**Amino Acid Sequence of Full Length Heavy Chain (IgG2) (SEQ ID NO.: 5):** 
QVQLVQSGAEVKKPGSSVKVSCKASGGTFNSYAISWVRQAPGQGLEWMGGIIP
GFGTANYAQKFQGRVTITADESTSTAYMELSSLRSEDTAVYYCARKNEEDGGFDHWG
QGTLVTVSSastkgpsvfplapcsrstsestaalgclvkdyfpepvtvswnsgaltsgvhtfpavlqssglyslssvvtvp
ssnfgtqtytcnvdhkpsntkvdktverkccvecppcpappvagpsvflfppkpkdtlmisrtpevtcvvvdvshedpevqf
nwyvdgvevhnaktkpreeqfnstfrvvsvltvvhqdwlngkeykckvsnkglpapiektisktkgqprepqvytlppsree
mtknqvsltclvkgfypsdiavewesngqpennykttppmldsdgsfflyskltvdksrwqqgnvfscsvmhealhnhytq
kslslspgk
**Amino Acid Sequence of V_{L} (SEQ ID NO.: 9):** 
DIELTQPPSVSVAPGQTARISCSGDNLGDYYASWYQQKPGQAPVLVIYDDSNRP
SGIPERFSGSNSGNTATLTISGTQAEDEADYYCQTWDGTLHFVFGGGTKLTVL
**Amino Acid Sequence of Full Length Light Chain (SEQ ID NO.: 10):** 
DIELTQPPSVSVAPGQTARISCSGDNLGDYYASWYQQKPGQAPVLVIYDDSNRPSGIPE
RFSGSNSGNTATLTISGTQAEDEADYYCQTWDGTLHFVFGGGTKLTVLgqpkaapsvtlfpp
sseelqankatlvclisdfypgavtvawkadsspvkagvetttpskqsnnkyaassylsltpeqwkshrsyscqvthegstv
ektvaptecs
**Nucleic Acid Sequence of V_{H} (SEQ ID NO.: 11):** 
caggtgcaattggttcagtctggcgcggaagtgaaaaaaccgggcagcagcgtgaaagtgagctgcaaagcct
ccggaggcacttttaattcttatgctatttcttgggtgcgccaagcccctgggcagggtctcgagtggatgggcggtatcattcc
gggttttggcactgcgaattacgcgcagaagtttcagggccgggtgaccattaccgcggatgaaagcaccagcaccgcgt
atatggaactgagcagcctgcgtagcgaagatacggccgtgtattattgcgcgcgtaagaatgaggaggatggtggttttga
tcattggggccaaggcaccctggtgacggttagctca
**Nucleic Acid Sequence of Full Length Heavy Chain (SEQ ID NO.: 13):** 
caggtgcaattggttcagtctggcgcggaagtgaaaaaaccgggcagcagcgtgaaagtgagctgcaaagcct
ccggaggcacttttaattcttatgctatttcttgggtgcgccaagcccctggcagggtctcgagtggatgggcggtatcattcc
gggttttggcactgcgaattacgcgcagaagtttcagggccgggtgaccattaccgcggatgaaagcaccagcaccgcgt
atatggaactgagcagcctgcgtagcgaagatacggccgtgtattattgcgcgcgtaagaatgaggaggatggtggttttga
tcattggggccaaggcaccctggtgacggttagctcagcctccaccaagggcccatcggtcttccccctggcgccctgctcc
aggagcacctccgagagcacagcggccctgggctgcctggtcaaggactacttccccgaaccggtgacggtgtcgtgga
actcaggcgctctgaccagcggcgtgcacaccttcccggctgtcctacagtcctcaggactctactccctcagcagcgtagt
gaccgtgccctccagcaacttcggcacccagacctacacctgcaacgtagatcacaagcccagcaacaccaaggtgga
caagacagttgagcgcaaatgttgtgtcgagtgcccaccgtgcccagcaccacctgtggcaggaccgtcagtcttcctcttcc
ccccaaaacccaaggacaccctcatgatctcccggacccctgaggtcacgtgcgtggtggtggacgtgagccacgaaga
ccccgaggtccagttcaactggtacgtggacggcgtggaggtgcataatgccaagacaaagccacgggaggagcagttc
aacagcacgttccgtgtggtcagcgtcctcaccgtcgtgcaccaggactggctgaacggcaaggagtacaagtgcaaggt
ctccaacaaaggcctcccagcccccatcgagaaaaccatctccaaaaccaaagggcagccccgagaaccacaggtgt
acaccctgcccccatcccgggaggagatgaccaagaaccaggtcagcctgacctgcctggtcaaaggcttctaccccag
cgacatcgccgtggagtgggagagcaatgggcagccggagaacaactacaagaccacacctcccatgctggactccga
cggctccttcttcctctacagcaagctcaccgtggacaagagcaggtggcagcaggggaacgtcttctcatgctccgtgatg
catgaggctctgcacaaccactacacacagaagagcctctccctgtctccgggtaaa
**Nucleic Acid Sequence of V_{L} (SEQ ID NO.: 12):** 
Gatatcgaactgacccagccgccttcagtgagcgttgcaccaggtcagaccgcgcgtatctcgtgtagcggcgat
aatcttggtgattattatgcttcttggtaccagcagaaacccggcaggcgccagttcttgtgatttatgatgattctaatcgtccct
caggcatcccggaacgctttagcggatccaacagcggcaacaccgcgaccctgaccattagcggcactcaggcggaag
acgaagcggattattattgccagacttgggatggtactcttcatttgtgtttggcggcggcacgaagttaaccgttctt
**Nucleic Acid Sequence of Full Length Light Chain (SEQ ID NO.: 14):**

Gatatcgaactgacccagccgccttcagtgagcgttgcaccaggtcagaccgcgcgtatctcgtgtagcggcgataatcttg
gtgattattatgcttcttggtaccagcagaaacccggcaggcgccagttcttgtgatttatgatgattctaatcgtccctcaggc
atcccggaacgctttagcggatccaacagcggcaacaccgcgaccctgaccattagcggcactcaggcggaagacgaa
gcggattattattgccagacttgggatggtactcttcattttgtgtttggcggcggcacgaagttaaccgttcttggtcagcccaag
gctgcccccctcggtcactctgttcccaccctcctctgaggagcttcaagccaacaaggccacactggtgtgtctcataagtga
cttctacccgggagccgtgacagtggcctggaaggcagatagcagccccgtcaaggcgggagtggagaccaccacacc
ctccaaacaaagcaacaacaagtacgcggccagcagctacctgagcctgacgcctgagcagtggaagtcccacagaa
gctacagctgccaggtcacgcatgaagggagcaccgtggagaagacagtggcccctacagaatgttca

### B. ANTIBODY MOR 7361

**Amino Acid Sequence of V_{H} (SEQ ID NO.: 18)** 
QVQLVESGGGLVQPGGSLRLSCAASGFTFSDYYMHWVRQAPGKGLEWVSVIS
GSGSNTYYADSVKGRFTISRDNSKNTLYLQMNSLRAEDTAVYYCARLYAQFEGDFWG
QGTLVTVSS
**Amino Acid Sequence of Full Length Heavy Chain (IgG2) (SEQ ID NO.: 19):** 
QVQLVESGGGLVQPGGSLRLSCAASGFTFSDYYMHWVRQAPGKGLEWVSVIS
GSGSNTYYADSVKGRFTISRDNSKNTLYLQMNSLRAEDTAVYYCARLYAQFEGDFWG
QGTLVTVSSastkgpsvfplapcsrstsestaalgclvkdyfpepvtvswnsgaltsgvhtfpavlqssglyslssvvtvp
ssnfgtqtytcnvdhkpsntkvdktverkccvecppcpappvagpsvflfppkpkdtlmisrtpevtcvvvdvshedpevqf
nwyvdgvevhnaktkpreeqfnstfrvvsvltvvhqdwlngkeykckvsnkglpapiektisktkgqprepqvytlppsree
mtknqvsltclvkgfypsdiavewesngqpennykttppmldsdgsfflyskltvdksrwqqgnvfscsvmhealhnhytq
kslslspgk
**Amino Acid Sequence of V_{L} (SEQ ID NO.: 23):** 
DIELTQPPSVSVAPGQTARISCSGDNIGSKYVSWYQQKPGQAPVLVIYSDSERP
SGIPERFSGSNSGNTATLTISGTQAEDEADYYCQSWDGS-ISRVFGGGTKLTVL
**Amino Acid Sequence of Full Length Light Chain (SEQ ID NO: 24):** 
DIELTQPPSVSVAPGQTARISCSGDNIGSKYVSWYQQKPGQAPVLVIYSDSERP
SGIPERFSGSNSGNTATLTISGTQAEDEADYYCQSWDGSISRVFGGGTKLTVLgqpkaap
svtlfppsseelqankatlvclisdfypgavtvawkadsspvkagvetttpskqsnnkyaassylsltpeqwkshrsyscqvt
hegstvektvaptecs
**Nucleic Acid Sequence of V_{H} (SEQ ID NO.: 25):** 
Caggtgcaattggtggaaagcggcggcggcctggtgcaaccgggcggcagcctgcgtctgagctgcgcggcc
tccggatttaccttttctgattattatatgcattgggtgcgccaagcccctggaagggtctcgagtgggtgagcgttatctctggtt
ctggtagcaatacctattatgcggatagcgtgaaaggccgttttaccatttcacgtgataattcgaaaaacaccctgtatctgca
aatgaacagcctgcgtgcggaagatacggccgtgtattattgcgcgcgtctttatgctcagtttgagggtgatttttggggccaa
ggcaccctggtgacggttagctca
**Nucleic Acid Sequence of Full Length Heavy Chain (SEQ ID NO.: 27):** 
caggtgcaattggtggaaagcggcggcctggtgcaaccgggcggcagcctgcgtctgagctgcgcggcctccggatt
taccttttctgattattatatgcattgggtgcgccaagcccctggaagggtctcgagtgggtgagcgttatctctggttctggtag
caatacctattatgcggatagcgtgaaaggccgttttaccatttcacgtgataattcgaaaaacaccctgtatctgcaaatgaa
cagcctgcgtgcggaagatacggccgtgtattattgcgcgcgtctttatgctcagtttgagggtgatttttggggccaaggcacc
ctggtgacggttagctcagcctccaccaagggcccatcggtcttccccctggcgccctgctccaggagcacctccgagagc
acagcggccctgggctgcctggtcaaggactacttccccgaaccggtgacggtgtcgtggaactcaggcgctctgaccag
cggcgtgcacaccttcccggctgtcctacagtcctcaggactctactccctcagcagcgtagtgaccgtgccctccagcaact
tcggcacccagacctacacctgcaacgtagatcacaagcccagcaacaccaaggtggacaagacagttgagcgcaaat
gttgtgtcgagtgcccaccgtgcccagcaccacctgtggcaggaccgtcagtcttcctcttccccccaaaacccaaggacac
cctcatgatctcccggacccctgaggtcacgtgcgtggtggtggacgtgagccacgaagaccccgaggtccagttcaactg
gtacgtggacggcgtggaggtgcataatgccaagacaaagccacgggaggagcagttcaacagcacgttccgtgtggtc
agcgtcctcaccgtcgtgcaccaggactggctgaacggcaaggagtacaagtgcaaggtctccaacaaaggcctcccag
cccccatcgagaaaaccatctccaaaaccaaagggcagccccgagaaccacaggtgtacaccctgcccccatcccggg
aggagatgaccaagaaccaggtcagcctgacctgcctggtcaaaggcttctaccccagcgacatcgccgtggagtggga
gagcaatgggcagccggagaacaactacaagaccacacctcccatgctggactccgacggctccttcttcctctacagca
agctcaccgtggacaagagcaggtggcagcaggggaacgtcttctcatgctccgtgatgcatgaggctctgcacaaccact
acacaca
gaagagcctctccctgtctccgggtaaa
**Nucleic Acid Sequence of V_{L} (SEQ ID NO.: 26)** 
gatatcgaactgacccagccgccttcagtgagcgttgcaccaggtcagaccgcgcgtatctcgtgtagcggcgat
aatattggttctaagtatgtttcttggtaccagcagaaacccgggcaggcgccagttcttgtgatttattctgattctgagcgtccct
caggcatcccggaacgctttagcggatccaacagcggcaacaccgcgaccctgaccattagcggcactcaggcggaag
acgaagcggattattattgccagtcttgggatggttctatttctcgtgtgtttggcggcggcacgaagttaaccgtcctaggtcag
**Nucleic Acid Sequence of Full Length Light Chain (SEQ ID NO.: 28)**

gatatcgaactgacccagccgccttcagtgagcgttgcaccaggtcagaccgcgcgtatctcgtgtagcggcgat
aatattggttctaagtatgtttcttggtaccagcagaaacccgggcaggcgccagttcttgtgatttattctgattctgagcgtccct
caggcatcccggaacgctttagcggatccaacagcggcaacaccgcgaccctgaccattagcggcactcaggcggaag
acgaagcggattattattgccagtcttgggatggttctatttctcgtgtgtttggcggcggcacgaagttaaccgtcctaggtcag

### C. ANTIBODY MOR 7480

**Amino Acid Sequence of V_{H} (SEQ ID NO.: 32)** 
QVQLVQSGAEVKKPGESLKISCKGSGYSFSTYWISWVRQMPGKGLEWMGKIYP
GDSYTNYSPSFQGQVTISADKSISTAYLQWSSLKASDTAMYYCARGYGIFDYWGQGTL
VTVSS
**Amino Acid Sequence of Full Length Heavy Chain (IgG2)(SEQ ID NO.:33)** 
QVQLVQSGAEVKKPGESLKISCKGSGYSFSTYWISWVRQMPGKGLEWMGKIYP
GDSYTNYSPSFQGQVTISADKSISTAYLQWSSLKASDTAMYYCARGYGIFDYWGQGTL
VTVSSastkgpsvfplapcsrstsestaalgclvkdyfpepvtvswnsgaltsgvhtfpavlqssglyslssvvtvpssnfgt
qtytcnvdhkpsntkvdktverkccvecppcpappvagpsvflfppkpkdtlmisrtpevtcvvvdvshedpevqfnwyvd
gvevhnaktkpreeqfnstfrvvsvltvvhqdwlngkeykckvsnkglpapiektisktkgqprepqvytlppsreemtknq
vsltclvkgfypsdiavewesngqpennykttppmldsdgsfflyskltvdksrwqqgnvfscsvmhealhnhytqkslsls
pgk
**Amino Acid Sequence of V_{L} (SEQ ID NO.:37)** 
DIELTQPPSVSVAPGQTARISCSGDNIGDQYAHWYQQKPGQAPVVVIYQDKNRP
SGIPERFSGSNSGNTATLTISGTQAEDEADYYCATYTGFGSLAVFGGGTKLTVL
**Amino Acid Sequence of Full Length Light Chain (SEQ ID NO 38):** 
DIELTQPPSVSVAPGQTARISCSGDNIGDQYAHWYQQKPGQAPVVVIYQDKNRPSGIPE
RFSGSNSGNTATLTISGTQAEDEADYYCATYTGFGSLAVFGGGTKLTVLgqpkaapsvtlfpp
sseelqankatlvclisdfypgavtvawkadsspvkagvetttpskqsnnkyaassylsltpeqwkshrsyscqvthegstv
ektvaptecs
**Nucleic Acid Sequence of V_{H} (SEQ ID NO.:39)** 
caggtgcaattggttcagagcggcgcggaagtgaaaaaaccgggcgaaagcctgaaaattagctgcaaaggtt
ccggatattccttttctacttattggatttcttgggtgcgccagatgcctgggaagggtctcgagtggatgggcaagatctatccg
ggtgatagctataccaattattctccgagctttcagggccaggtgactattagcgcggataaaagcattagcaccgcgtatctt
caatggagcagcctgaaagcgagcgatacggccatgtattattgtgcgcgtggttatggtatttttgattattggggccaaggc
accctggtcaccgtctcctca
**Nucleic Acid Sequence of Full Length Heavy Chain (IgG2) (SEQ ID NO.: 41)** 
caggtgcaattggttcagagcggcgcggaagtgaaaaaaccgggcgaaagcctgaaaattagctgcaaaggttccggat
attccttttctacttattggatttcttgggtgcgccagatgcctgggaagggtctcgagtggatgggcaagatctatccgggtgat
agctataccaattattctccgagctttcagggccaggtgactattagcgcggataaaagcattagcaccgcgtatcttcaatgg
agcagcctgaaagcgagcgatacggccatgtattattgtgcgcgtggttatggtatttttgattattggggccaaggcaccctg
gtcaccgtctcctcagcctccaccaagggcccatcggtcttccccctggcgccctgctccaggagcacctccgagagcaca
gcggccctgggctgcctggtcaaggactacttccccgaaccggtgacggtgtcgtggaactcaggcgctctgaccagcgg
cgtgcacaccttcccggctgtcctacagtcctcaggactctactccctcagcagcgtagtgaccgtgccctccagcaacttcg
gcacccagacctacacctgcaacgtagatcacaagcccagcaacaccaaggtggacaagacagttgagcgcaaatgtt
gtgtcgagtgcccaccgtgcccagcaccacctgtggcaggaccgtcagtcttcctcttccccccaaaacccaaggacaccc
tcatgatctcccggacccctgaggtcacgtgcgtggtggtggacgtgagccacgaagaccccgaggtccagttcaactggt
acgtggacggcgtggaggtgcataatgccaagacaaagccacgggaggagcagttcaacagcacgttccgtgtggtcag
cgtcctcaccgtcgtgcaccaggactggctgaacggcaaggagtacaagtgcaaggtctccaacaaaggcctcccagcc
cccatcgagaaaaccatctccaaaaccaaagggcagccccgagaaccacaggtgtacaccctgcccccatcccggga
ggagatgaccaagaaccaggtcagcctgacctgcctggtcaaaggcttctaccccagcgacatcgccgtggagtgggag
agcaatgggcagccggagaacaactacaagaccacacctcccatgctggactccgacggctccttcttcctctacagcaa
gctcaccgtggacaagagcaggtggcagcaggggaacgtcttctcatgctccgtgatgcatgaggctctgcacaaccacta
cacacagaagagcctctccctgtctccgggtaaa
**Nucleic Acid Sequence of V_{L} (SEQ ID NO.: 40)** 
Gatatcgaactgacccagccgccttcagtgagcgttgcaccaggtcagaccgcgcgtatctcgtgtagcggcgat
aatattggtgatcagtatgctcattggtaccagcagaaacccgggcaggcgccagttgttgtgatttatcaggataagaatcgt
ccctcaggcatcccggaacgctttagcggatccaacagcggcaacaccgcgaccctgaccattagcggcactcaggcgg
aagacgaagcggattattattgcgctacttatactggttttggttctcttgctgtgtttggcggcggcacgaagttaaccgtccta
**Nucleic Acid Sequence of Full Length Light Chain (SEQ ID NO.: 42)**

gatatcgaactgacccagccgccttcagtgagcgttgcaccaggtcagaccgcgcgtatctcgtgtagcggcgat
aatattggtgatcagtatgctcattggtaccagcagaaacccgggcaggcgccagttgttgtgatttatcaggataagaatcgt
ccctcaggcatcccggaacgctttagcggatccaacagcggcaacaccgcgaccctgaccattagcggcactcaggcgg
aagacgaagcggattattattgcgctacttatactggttttggttctcttgctgtgtttggcggcggcacgaagttaaccgtccta

### D. ANTIBODY MOR 7480.1

**Amino Acid Sequence of V_{H} (SEQ ID NO.: 43):** 
EVQLVQSGAEVKKPGESLRISCKGSGYSFSTYWISWVRQMPGKGLEWMGKIYP
GDSYTNYSPSFQGQVTISADKSISTAYLQWSSLKASDTAMYYCARGYGIFDYWGQGTL
VTVSS
**Amino Acid Sequence of Full Length Heavy Chain (IgG2)(SEQ ID NO.: 44):** 
EVQLVQSGAEVKKPGESLRISCKGSGYSFSTYWISWVRQMPGKGLEWMGKIYPGDSY
TNYSPSFQGQVTISADKSISTAYLQWSSLKASDTAMYYCARGYGIFDYWGQGTLVTVS
Sastkgpsvfplapcsrstsestaalgclvkdyfpepvtvswnsgaltsgvhtfpavlqssglyslssvvtvpssnfgtqtytcn
vdhkpsntkvdktverkccvecppcpappvagpsvflfppkpkdtlmisrtpevtcvvvdvshedpevqfnwyvdgvevh
naktkpreeqfnstfrvvsvltvvhqdwlngkeykckvsnkglpapiektisktkgqprepqvytlppsreemtknqvsltclv
kgfypsdiavewesngqpennykttppmldsdgsfflyskltvdksrwqqgnvfscsvmhealhnhytqkslslspgk
**Amino Acid Sequence of V_{L} (SEQ ID NO.: 45):** 
SYELTQPPSVSVSPGQTASITCSGDNIGDQYAHWYQQKPGQSPVLVIYQDKNR
PSGIPERFSGSNSGNTATLTISGTQAMDEADYYCATYTGFGSLAVFGGGTKLTVL
**Amino Acid Sequence of Full Length Light Chain (SEQ ID NO.: 46):** 
SYELTQPPSVSVSPGQTASITCSGDNIGDQYAHWYQQKPGQSPVLVIYQDKNR
PSGIPERFSGSNSGNTATLTISGTQAMDEADYYCATYTGFGSLAVFGGGTKLTVLgqpka
apsvtlfppsseelqankatlvclisdfypgavtvawkadsspvkagvetttpskqsnnkyaassylsltpeqwkshrsysc
qvthegstvektvaptecs
**Nucleic Acid Sequence of V_{H} (SEQ ID NO.:47):** 
gaggtgcaattggttcagagcggcgcggaagtgaaaaaaccgggcgaaagcctgaggattagctgcaaaggt
tccggatattccttttctacttattggatttcttgggtgcgccagatgcctgggaagggtctcgagtggatgggcaagatctatccg
ggtgatagctataccaattattctccgagctttcagggccaggtgactattagcgcggataaaagcattagcaccgcgtatctt
caatggagcagcctgaaagcgagcgatacggccatgtattattgtgcgcgtggttatggtatttttgattattggggccaaggc
accctggtcaccgtctcctca
**Nucleic Acid Sequence of Full Length Heavy Chain (IgG2) (SEQ ID NO.: 49):** 
gaggtgcaattggttcagagcggcgcggaagtgaaaaaaccgggcgaaagcctgaggattagctgcaaaggt
tccggatattccttttctacttattggatttcttgggtgcgccagatgcctgggaagggtctcgagtggatgggcaagatctatccg
 
ggtgatagctataccaattattctccgagctttcagggccaggtgactattagcgcggataaaagcattagcaccgcgtatctt
caatggagcagcctgaaagcgagcgatacggccatgtattattgtgcgcgtggttatggtatttttgattattggggccaaggc
accctggtcaccgtctcctcagcctccaccaagggcccatcggtcttccccctggcgccctgctccaggagcacctccgaga
gcacagcggccctgggctgcctggtcaaggactacttccccgaaccggtgacggtgtcgtggaactcaggcgctctgacc
agcggcgtgcacaccttcccggctgtcctacagtcctcaggactctactccctcagcagcgtagtgaccgtgccctccagca
acttcggcacccagacctacacctgcaacgtagatcacaagcccagcaacaccaaggtggacaagacagttgagcgca
aatgttgtgtcgagtgcccaccgtgcccagcaccacctgtggcaggaccgtcagtcttcctcttccccccaaaacccaagga
caccctcatgatctcccggacccctgaggtcacgtgcgtggtggtggacgtgagccacgaagaccccgaggtccagttca
actggtacgtggacggcgtggaggtgcataatgccaagacaaagccacgggaggagcagttcaacagcacgttccgtgt
ggtcagcgtcctcaccgtcgtgcaccaggactggctgaacggcaaggagtacaagtgcaaggtctccaacaaaggcctc
ccagcccccatcgagaaaaccatctccaaaaccaaagggcagccccgagaaccacaggtgtacaccctgcccccatcc
cgggaggagatgaccaagaaccaggtcagcctgacctgcctggtcaaaggcttctaccccagcgacatcgccgtggagt
gggagagcaatgggcagccggagaacaactacaagaccacacctcccatgctggactccgacggctccttcttcctctac
agcaagctcaccgtggacaagagcaggtggcagcaggggaacgtcttctcatgctccgtgatgcatgaggctctgcacaa
ccactacacacagaagagcctctccctgtctccgggtaaa
**Nucleic Acid Sequence of V_{L} (SEQ ID NO.:48):** 
agctacgagctgacccagcccccagcgtgtccgtgagccctggccagaccgccagcatcacctgcagcggc
gacaacatcggcgaccagtacgcccactggtatcagcagaagcccggccagagccccgtgctggtgatctaccaggac
aagaaccggcccagcggcatccccgagcggttcagcggcagcaacagcggcaacaccgccaccctgaccatcagcg
gcacccaggccatggacgaggccgactactactgcgccacctacaccggcttcggcagcctggccgtgttcggcggagg
gaccaagctgaccgtccta
**Nucleic Acid Sequence of Full Length Light Chain (SEQ ID NO.: 50):**

agctacgagctgacccagcccccagcgtgtccgtgagccctggccagaccgccagcatcacctgcagcggcgacaac
atcggcgaccagtacgcccactggtatcagcagaagcccggccagagccccgtgctggtgatctaccaggacaagaac
cggcccagcggcatccccgagcggttcagcggcagcaacagcggcaacaccgccaccctgaccatcagcggcaccca
ggccatggacgaggccgactactactgcgccacctacaccggcttcggcagcctggccgtgttcggcggagggaccaag
ctgaccgtcctaggtcagcccaaggctgcccctcggtcactctgttcccaccctcctctgaggagcttcaagccaacaagg
ccacactggtgtgtctcataagtgacttctacccgggagccgtgacagtggcctggaaggcagatagcagccccgtcaagg
cgggagtggagaccaccacaccctccaaacaaagcaacaacaagtacgcggccagcagctacctgagcctgacgcct
gagcagtggaagtcccacagaagctacagctgccaggtcacgcatgaagggagcaccgtggagaagacagtggcccc
tacagaatgttca

### E. ANTIBODY MOR 7480.2

**Amino Acid Sequence of V_{H} (SEQ ID NO.: 43):**
   (same as MOR-7480.1)
**Amino Acid Sequence of Full Length Heavy Chain (IgG2) (SEQ ID NO.: 44):**
   (same as MOR-7480.1 IgG2)
**Amino Acid Sequence of V_{L} (SEQ ID NO.:51):** 
SYELTQPPSVSVSPGQTASITCSGDNIGDQYAHWYQQKPGQSPVVVIYQDKNR
PSGIPERFSGSNSGNTATLTISGTQAMDEADYYCATYTGFGSLAVFGGGTKLTVL
**Amino Acid Sequence of Full Length Light Chain (SEQ ID NO 52):** 
SYELTQPPSVSVSPGQTASITCSGDNIGDQYAHWYQQKPGQSPVVVIYQDKNRPSGIP
ERFSGSNSGNTATLTISGTQAMDEADYYCATYTGFGSLAVFGGGTKLTVLgqpkaapsvtlf
ppsseelqankatlvclisdfypgavtvawkadsspvkagvetttpskqsnnkyaassylsltpeqwkshrsyscqvtheg
stvektvaptecs
**Nucleic Acid Sequence of V_{H} (SEQ ID NO.:47):**
   (same as MOR-7480.1)
**Nucleic Acid Sequence of Full Length Heavy Chain (IgG2) (SEQ ID NO.: 49):**
   (same as MOR-7480.1 IgG2)
**Nucleic Acid Sequence of V_{L} (SEQ ID NO.:53):** 
agctacgagctgacccagcccccagcgtgtccgtgagccctggccagaccgccagcatcacctgcagcggc
gacaacatcggcgaccagtacgcccactggtatcagcagaagcccggccagagccccgtggtggtgatctaccaggac
aagaaccggcccagcggcatccccgagcggttcagcggcagcaacagcggcaacaccgccaccctgaccatcagcg
gcacccaggccatggacgaggccgactactactgcgccacctacaccggcttcggcagcctggccgtgttcggcggagg
gaccaagctgaccgtccta
**Nucleic Acid Sequence of Full Length Light Chain (SEQ ID NO.: 54):**

agctacgagctgacccagcccccagcgtgtccgtgagccctggccagaccgccagcatcacctgcagcggcgacaac
atcggcgaccagtacgcccactggtatcagcagaagcccggccagagccccgtggtggtgatctaccaggacaagaac
cggcccagcggcatccccgagcggttcagcggcagcaacagcggcaacaccgccaccctgaccatcagcggcaccca
ggccatggacgaggccgactactactgcgccacctacaccggcttcggcagcctggccgtgttcggcggagggaccaag
ctgaccgtcctaggtcagcccaaggctgccccctcggtcactctgttcccaccctcctctgaggagcttcaagccaacaagg

ccacactggtgtgtctcataagtgacttctacccgggagccgtgacagtggcctggaaggcagatagcagccccgtcaagg
cgggagtggagaccaccacaccctccaaacaaagcaacaacaagtacgcggccagcagctacctgagcctgacgcct
gagcagtggaagtcccacagaagctacagctgccaggtcacgcatgaagggagcaccgtggagaagacagtggcccc
tacagaatgttca

### F. ANTIBODY MOR-7483

**Amino Acid Sequence of V_{H} (SEQ ID NO.:32):**
   (Same as MOR 7480)
**Amino Acid Sequence of Full Length Heavy Chain (IgG2) (SEQ ID NO.: 33):**
   (Same as MOR 7480 IgG2)
**Amino Acid Sequence of V_{L} (SEQ ID NO.:56):** 
DIELTQPPSVSVAPGQTARISCSGDNIGDQYAHWYQQKPGQAPVVVIYQDKNRP
SGIPERFSGSNSGNTATLTISGTQAEDEADYYCSTYTFVGFTTVFGGGTKLTVL
**Amino Acid Sequence of Full Length Light Chain (SEQ ID NO:57):** 
DIELTQPPSVSVAPGQTARISCSGDNIGDQYAHWYQQKPGQAPVVVIYQDKNRP
SGIPERFSGSNSGNTATLTISGTQAEDEADYYCSTYTFVGFTTVFGGGTKLTVLgqpkaap
svtlfppsseelqankatlvclisdfypgavtvawkadsspvkagvetttpskqsnnkyaassylsltpeqwkshrsyscqvt
hegstvektvaptecs
**Nucleic Acid Sequence of V_{H} (SEQ ID NO.:39):**
   (Same as MOR 7480)
**Nucleic Acid Sequence of Full Length Heavy Chain (IgG2) (SEQ ID NO.: 41):**
   (Same as MOR 7480 IgG2)
**Nucleic Acid Sequence of V_{L} (SEQ ID NO.: 58):** 
Gatatcgaactgacccagccgccttcagtgagcgttgcaccaggtcagaccgcgcgtatctcgtgtagcggcgat
aatattggtgatcagtatgctcattggtaccagcagaaacccgggcaggcgccagttgttgtgatttatcaggataagaatcgt
ccctcaggcatcccggaacgctttagcggatccaacagcggcaacaccgcgaccctgaccattagcggcactcaggcgg
aagacgaagcggattattattgctctacttatacttttgttggttttactactgtgtttggcggcggcacgaagttaaccgtccta
**Nucleic Acid Sequence of Full Length Light Chain (SEQ ID NO.:59):**

Gatatcgaactgacccagccgccttcagtgagcgttgcaccaggtcagaccgcgcgtatctcgtgtagcggcgataatattg
gtgatcagtatgctcattggtaccagcagaaacccgggcaggcgccagttgttgtgatttatcaggataagaatcgtccctca
ggcatcccggaacgctttagcggatccaacagcggcaacaccgcgaccctgaccattagcggcactcaggcggaagac
gaagcggattattattgctctacttatacttttgttggttttactactgtgtttggcggcggcacgaagttaaccgtcctaggtcagcc
caaggctgccccctcggtcactctgttcccaccctcctctgaggagcttcaagccaacaaggccacactggtgtgtctcataa
gtgacttctacccgggagccgtgacagtggcctggaaggcagatagcagccccgtcaaggcgggagtggagaccacca
caccctccaaacaaagcaacaacaagtacgcggccagcagctacctgagcctgacgcctgagcagtggaagtcccaca
gaagctacagctgccaggtcacgcatgaagggagcaccgtggagaagacagtggcccctacagaatgttca

### G. ANTIBODY MOR-7483.1

**Amino Acid Sequence of V_{H} (SEQ ID NO.:43):**
   (same as MOR 7480.1)
**Amino Acid Sequence of Full Length Heavy Chain (IgG2) (SEQ ID NO.:44):**
   (same as MOR-7480.1 IgG2)
**Amino Acid Sequence of V_{L} (SEQ ID NO.:60):** 
SYELTQPPSVSVSPGQTASITCSGDNIGDQYAHWYQQKPGQSPVLVIYQDKNR
PSGIPERFSGSNSGNTATLTISGTQAMDEADYYCSTYTFVGFTTVFGGGTKLTVL
**Amino Acid Sequence of Full Length Light Chain (SEQ ID NO.: 61):** 
DIELTQPPSVSVAPGQTARISCSGDNIGDQYAHWYQQKPGQAPVVVIYQDKNRPSGIPE
RFSGSNSGNTATLTISGTQAEDEADYYCSTYTFVGFTTVFGGGTKLTVLgqpkaapsvtlfpp
sseelqankatlvclisdfypgavtvawkadsspvkagvetttpskqsnnkyaassylsltpeqwkshrsyscqvthegstv
ektvaptecs
**Nucleic Acid Sequence of V_{H} (SEQ ID NO.: 47):**
   (same as MOR-7480.1)
**Nucleic Acid Sequence of Full Length Heavy Chain (IgG2) (SEQ ID NO.: 49):**
   (same as MOR-7480.1 IgG2)
**Nucleic Acid Sequence of V_{L} (SEQ ID NO.: 62):** 
Agctacgagctgacccagcccccagcgtgtccgtgagccctggccagaccgccagcatcacctgcagcggc
gacaacatcggcgaccagtacgcccactggtatcagcagaagcccggccagagccccgtgctggtgatctaccaggac
 
aagaaccggcccagcggcatccccgagcggttcagcggcagcaacagcggcaacaccgccaccctgaccatcagcg
gcacccaggccatggacgaggccgactactactgctctacttatacttttgttggttttactactgtgttcggcggagggaccaa
gctgaccgtccta
**Nucleic Acid Sequence of Full Length Light Chain (SEQ ID NO.: 63):**

agctacgagctgacccagccccccagcgtgtccgtgagccctggccagaccgccagcatcacctgcagcggcgacaac
atcggcgaccagtacgcccactggtatcagcagaagcccggccagagccccgtgctggtgatctaccaggacaagaac
cggcccagcggcatccccgagcggttcagcggcagcaacagcggcaacaccgccaccctgaccatcagcggcaccca
ggccatggacgaggccgactactactgctctacttatacttttgttggttttactactgtgttcggcggagggaccaagctgacc
gtcctaggtcagcccaaggctgcccccctcggtcactctgttcccaccctcctctgaggagcttcaagccaacaaggccacac
tggtgtgtctcataagtgacttctacccgggagccgtgacagtggcctggaaggcagatagcagccccgtcaaggcggga
gtggagaccaccacaccctccaaacaaagcaacaacaagtacgcggccagcagctacctgagcctgacgcctgagca
gtggaagtcccacagaagctacagctgccaggtcacgcatgaagggagcaccgtggagaagacagtggcccctacag
aatgttca

### H. ANTIBODY MOR-7483.2

**Amino Acid Sequence of V_{H} (SEQ ID NO.: 43):**
   (same as MOR-7480.1)
**Amino Acid Sequence of Full Length Heavy Chain (IgG2) (SEQ ID NO.:44):**
   (same as MOR-7480.1 IgG2)
**Amino Acid Sequence of V_{L} (SEQ ID NO.: 64):** 
SYELTQPPSVSVSPGQTASITCSGDNIGDQYAHWYQQKPGQSPVVVIYQDKNR
PSGIPERFSGSNSGNTATLTISGTQAMDEADYYCSTYTFVGFTTVFGGGTKLTVL
**Amino Acid Sequence of Full Length Light Chain (SEQ ID NO 65):** 
SYELTQPPSVSVSPGQTASITCSGDNIGDQYAHWYQQKPGQSPVVVIYQDKNRPSGIP
ERFSGSNSGNTATLTISGTQAMDEADYYCSTYTFVGFTTVFGGGTKLTVLgqpkaapsvtlfp
psseelqankatlvclisdfypgavtvawkadsspvkagvetttpskqsnnkyaassylsltpeqwkshrsyscqvthegst
vektvaptecs
**Nucleic Acid Sequence of V_{H} (SEQ ID NO.:47):**
   (same as MOR-7480.1)
**Nucleic Acid Sequence of Full Length Heavy Chain (IgG2) (SEQ ID NO.: 49):**
   (same as MOR-7480.1 IgG2)
**Nucleic Acid Sequence of V_{L} (SEQ ID NO.:66):** 
agctacgagctgacccagcccccagcgtgtccgtgagccctggccagaccgccagcatcacctgcagcggc
gacaacatcggcgaccagtacgcccactggtatcagcagaagcccggccagagccccgtggtggtgatctaccaggac
aagaaccggcccagcggcatccccgagcggttcagcggcagcaacagcggcaacaccgccaccctgaccatcagcg
gcacccaggccatggacgaggccgactactactgctctacttatacttttgttggttttactactgtgttcggcggagggaccaa
gctgaccgtccta
**Nucleic Acid Sequence of Full Length Light Chain (SEQ ID NO.: 67):**

agctacgagctgacccagcccccagcgtgtccgtgagccctggccagaccgccagcatcacctgcagcggcgacaac
atcggcgaccagtacgcccactggtatcagcagaagcccggccagagccccgtggtggtgatctaccaggacaagaac
cggcccagcggcatccccgagcggttcagcggcagcaacagcggcaacaccgccaccctgaccatcagcggcaccca
ggccatggacgaggccgactactactgctctacttatacttttgttggttttactactgtgttcggcggagggaccaagctgacc
gtcctaggtcagcccaaggctgcccctcggtcactctgttcccaccctcctctgaggagcttcaagccaacaaggccacac
tggtgtgtctcataagtgacttctacccgggagccgtgacagtggcctggaaggcagatagcagccccgtcaaggcggga
gtggagaccaccacaccctccaaacaaagcaacaacaagtacgcggccagcagctacctgagcctgacgcctgagca
gtggaagtcccacagaagctacagctgccaggtcacgcatgaagggagcaccgtggagaagacagtggcccctacag
aatgttca

**I. Amino Acid Sequence of Human 4-1BB (SEQ ID NO:68):**

mgnscynivatlllvlnfertrslqdpcsncpagtfcdnnrnqicspcppnsfssaggqrtcdicrqckgvfrtrkecs
stsnaecdctpgfhclgagcsmceqdckqgqeltkkgckdccfgtfndqkrgicrpwtncsldgksvlvngtkerdvvcgps
padlspgassvtppaparepghspqiisfflaltstallfllffltlrfsvvkrgrkkllyifkqpfmrpvqttqeedgcscrfpeeeegg
cel

**J. Amino Acid Sequence of Human IgG1 Constant Region (SEQ ID NO: 69):**

astkgpsvfplapsskstsggtaalgclvkdyfpepvtvswnsgaltsgvhtfpavlqssglyslssvvtvpssslgtq
tyicnvnhkpsntkvdkkvepkscdkthtcppcpapellggpsvflfppkpkdtlmisrtpevtcvvvdvshedpevkfnwy
vdgvevhnaktkpreeqynstyrvvsvltvlhqdwlngkeykckvsnkalpapiektiskakgqprepqvytlppsreemtk
nqvsltclvkgfypsdiavewesngqpennykttppvldsdgsfflyskltvdksrwqqgnvfscsvmhealhnhytqkslsl
spgk

**K. Nucleic Acid Sequence of Human IgG1 Constant Region (SEQ ID NO: 70):**

GCCTCCACCAAGGGCCCATCGGTCTTCCCCCTGGCACCCTCCTCCAAGAGCACCT
CTGGGGGCACAGCGGCCCTGGGCTGCCTGGTCAAGGACTACTTCCCCGAACCGGT
GACGGTGTCGTGGAACTCAGGCGCCCTGACCAGCGGCGTGCACACCTTCCCGGCT
GTCCTACAGTCCTCAGGACTCTACTCCCTCAGCAGCGTAGTGACCGTGCCCTCCAG
CAGCTTGGGCACCCAGACCTACATCTGCAACGTGAATCACAAGCCCAGCAACACCA
AGGTGGACAAGAAAGTTGAGCCCAAATCTTGTGACAAAACTCACACATGCCCACCG
TGCCCAGCACCTGAACTCCTGGGGGGACCGTCAGTCTTCCTCTTCCCCCCAAAACC
CAAGGACACCCTCATGATCTCCCGGACCCCTGAGGTCACATGCGTGGTGGTGGAC
GTGAGCCACGAAGACCCTGAGGTCAAGTTCAACTGGTACGTGGACGGCGTGGAGG
TGCATAATGCCAAGACAAAGCCGCGGGAGGAGCAGTACAACAGCACGTACCGTGT
GGTCAGCGTCCTCACCGTCCTGCACCAGGACTGGCTGAATGGCAAGGAGTACAAG
TGCAAGGTCTCCAACAAAGCCCTCCCAGCCCCCATCGAGAAAACCATCTCCAAAGC
CAAAGGGCAGCCCCGAGAACCACAGGTGTACACCCTGCCCCCATCCCGGGAGGAG
ATGACCAAGAACCAGGTCAGCCTGACCTGCCTGGTCAAAGGCTTCTATCCCAGCGA
CATCGCCGTGGAGTGGGAGAGCAATGGGCAGCCGGAGAACAACTACAAGACCACG
CCTCCCGTGCTGGACTCCGACGGCTCCTTCTTCCTCTATAGCAAGCTCACCGTGGA
CAAGAGCAGGTGGCAGCAGGGGAACGTCTTCTCATGCTCCGTGATGCATGAGGCT
CTGCACAACCACTACACGCAGAAGAGCCTCTCCCTGTCTCCGGGTAAA

**L. Amino Acid Sequence of Human IgG2 Constant Region** (SEQ ID NO: 71)

astkgpsvfplapcsrstsestaalgclvkdyfpepvtvswnsgaltsgvhtfpavlqssglyslssvvtvpssnfgtq
tytcnvdhkpsntkvdktverkccvecppcpappvagpsvflfppkpkdtlmisrtpevtcvvvdvshedpevqfnwyvdg
vevhnaktkpreeqfnstfrvvsvltvvhqdwlngkeykckvsnkglpapiektisktkgqprepqvytlppsreemtknqvs
ltclvkgfypsdiavewesngqpennykttppmldsdgsfflyskltvdksrwqqgnvfscsvmhealhnhytqkslslspg
k

**M. Nucleic Acid Sequence of Human IgG2 Constant Region (SEQ ID NO: 72):**

GCCTCCACCAAGGGCCCATCGGTCTTCCCCCTGGCGCCCTGCTCCAGGAGCACCT
CCGAGAGCACAGCGGCCCTGGGCTGCCTGGTCAAGGACTACTTCCCCGAACCGGT
GACGGTGTCGTGGAACTCAGGCGCTCTGACCAGCGGCGTGCACACCTTCCCGGCT
GTCCTACAGTCCTCAGGACTCTACTCCCTCAGCAGCGTAGTGACCGTGCCCTCCAG

CAACTTCGGCACCCAGACCTACACCTGCAACGTAGATCACAAGCCCAGCAACACCA
AGGTGGACAAGACAGTTGAGCGCAAATGTTGTGTCGAGTGCCCACCGTGCCCAGC
ACCACCTGTGGCAGGACCGTCAGTCTTCCTCTTCCCCCCAAAACCCAAGGACACCC
TCATGATCTCCCGGACCCCTGAGGTCACGTGCGTGGTGGTGGACGTGAGCCACGA
AGACCCCGAGGTCCAGTTCAACTGGTACGTGGACGGCGTGGAGGTGCATAATGCC
AAGACAAAGCCACGGGAGGAGCAGTTCAACAGCACGTTCCGTGTGGTCAGCGTCC
TCACCGTCGTGCACCAGGACTGGCTGAACGGCAAGGAGTACAAGTGCAAGGTCTC
CAACAAAGGCCTCCCAGCCCCCATCGAGAAAACCATCTCCAAAACCAAAGGGCAGC
CCCGAGAACCACAGGTGTACACCCTGCCCCCATCCCGGGAGGAGATGACCAAGAA
CCAGGTCAGCCTGACCTGCCTGGTCAAAGGCTTCTACCCCAGCGACATCGCCGTG
GAGTGGGAGAGCAATGGGCAGCCGGAGAACAACTACAAGACCACACCTCCCATGC
TGGACTCCGACGGCTCCTTCTTCCTCTACAGCAAGCTCACCGTGGACAAGAGCAGG
TGGCAGCAGGGGAACGTCTTCTCATGCTCCGTGATGCATGAGGCTCTGCACAACCA
CTACACACAGAAGAGCCTCTCCCTGTCTCCGGGTAAA

**N. Amino Acid Sequence of Human Lambda Light Chain Constant Region (SEQ ID NO:73):**

gqpkaapsvtlfppsseelqankatlvclisdfypgavtvawkadsspvkagvetttpskqsnnkyaassylsltp
eqwkshrsyscqvthegstvektvaptecs

**O. Nucleic Acid Sequence of Human Lambda Light Chain Constant Region (SEQ ID NO:74):**

GGTCAGCCCAAGGCTGCCCCCTCGGTCACTCTGTTCCCACCCTCCTCTGAGGAGC
TTCAAGCCAACAAGGCCACACTGGTGTGTCTCATAAGTGACTTCTACCCGGGAGCC
GTGACAGTGGCCTGGAAGGCAGATAGCAGCCCCGTCAAGGCGGGAGTGGAGACC
ACCACACCCTCCAAACAAAGCAACAACAAGTACGCGGCCAGCAGCTACCTGAGCCT
GACGCCTGAGCAGTGGAAGTCCCACAGAAGCTACAGCTGCCAGGTCACGCATGAA
GGGAGCACCGTGGAGAAGACAGTGGCCCCTACAGAATGTTCA

### P. P-Cadherin ANTIBODY (g-194-g09 or anti-P-cadherin1)

**Amino Acid Sequence of H-CDR1 (SEQ ID NO:75):**
   SYAMS
**Amino Acid Sequence of H-CDR2 (SEQ ID NO:76):**
   AISGSGGSTYYADSVKG
**Amino Acid Sequence of H-CDR3 (SEQ ID NO:77):**
   TNSAKFDP
**Amino Acid Sequence of L-CDR1 (SEQ ID NO:78):**
   TGTSNDVGAYNYVS
**Amino Acid Sequence of L-CDR2 (SEQ ID NO:79):**
   EVNKRPS
**Amino Acid Sequence of L-CDR3 (SEQ ID NO:80):**
   SSYTMGSTFM L
**Amino Acid Sequence of V_{H} (SEQ ID NO:81):** 
EVQLLESGGGLVQPGGSLRLSCAASGFTFSSYAMSWVRQAPGKGLEWVSAISG
SGGSTYYADSVKGRFTISRDNSKNTLYLQMNSLRAEDTAVYYCAKTNSAKFDPWGQGT
MVTVSS
**Amino Acid Sequence of V_{L} (SEQ ID NO:82):** 
QSALTQPASVSGSPGQSITISCTGTSNDVGAYNYVSWYQQHPGKAPKLMISEVN
KRPSGVSNRFSGSKSGNTASLTISGLQAEDEADYYCSSYTMGSTFMLFGGGTKLTVL
**Amino Acid Sequence of the Heavy Chain Constant Region (SEQ ID NO:83):** 
astkgpsvfplapsskstsggtaalgclvkdyfpepvtvswnsgaltsgvhtfpavlqssglyslssvvtvpssslgtq
tyicnvnhkpsntkvdkkvepkscdkthtcppcpapellggpsvflfppkpkdtlmisrtpevtcvvvdvshedpevkfnwy
vdgvevhnaktkpreeqynstyrvvsvltvlhqdwlngkeykckvsnkalpapiektiskakgqprepqvytlppsreemtk
nqvsltclvkgfypsdiavewesngqpennykttppvldsdgsfflyskltvdksrwqqgnvfscsvmhealhnhytqkslsl
spgk
**Amino Acid Sequence of the Light Chain Constant Region (SEQ ID NO:84):**

gqpkaapsvtlfppsseelqankatlvclisdfypgavtvawkadsspvkagvetttpskqsnnkyaassylsltp
eqwkshrsyscqvthegstvektvaptecs

The invention also relates to the following embodiments:
Embodiment 1. A method for the treatment of cancer in a patient in need of such treatment, said method comprising administering to said patient a therapeutically effective amount of an anti-4-1BB antibody, or antigen-binding portion thereof, in combination with a therapeutically effective amount of an anti-CD20 antibody, or antigen-binding portion thereof.
Embodiment 2. The method of embodiment 1, wherein said anti-4-1BB antibody, or antigen-binding portion thereof, comprises:
   (a) an H-CDR1 as set forth in SEQ ID NO:29;
   (b) an H-CDR2 as set forth in SEQ ID NO:30;
   (c) an H-CDR3 as set forth in SEQ ID NO:31;
   (d) an L-CDR1 as set forth in SEQ ID NO:34;
   (e) an L-CDR2 as set forth in SEQ ID NO:35; and
   (f) an L-CDR3 as set forth in SEQ ID NO:36.
Embodiment 3. The method of any one of embodiments 1-2, wherein said anti-CD20 antibody, or antigen-binding portion thereof, comprises the 6 CDRs of rituximab.
Embodiment 4. The method of any one of embodiments 1-3, wherein said anti-4-1BB antibody, or antigen-binding portion thereof, comprises a V_{H} region comprising the amino acid sequence set forth in SEQ ID NO:43 and a V_{L} region comprising the amino acid sequence set forth in SEQ ID NO:45.
Embodiment 5. The method of any one of embodiments 1-4, wherein said anti-CD20 antibody, or antigen-binding portion thereof, comprises the V_{H} region of rituximab and the V_{L} region of rituximab.
Embodiment 6. The method of any one of embodiments 1-5, wherein said anti-4-1BB antibody, or antigen-binding portion thereof, comprises a heavy chain amino acid sequence as set forth in SEQ ID NO:44 and further comprises a light chain amino acid sequence set forth in SEQ ID NO:46, with the proviso that the C-terminal lysine residue of SEQ ID NO:44 is optionally absent.
Embodiment 7. The method of any one of embodiments 1-6, wherein said anti-CD20 antibody, or antigen-binding portion thereof, comprises the heavy chain amino acid sequence of rituximab and the light chain amino acid sequence of rituximab.
Embodiment 8. A method for the treatment of cancer in a patient in need of such treatment, said method comprising administering to said patient a therapeutically effective amount of MOR-7480.1, or antigen-binding portion thereof, in combination with a therapeutically effective amount of rituximab, or antigen-binding portion thereof.
Embodiment 9. A method for the treatment of cancer in a patient in need of such treatment, said method comprising administering to said patient a therapeutically effective amount of an anti-4-1BB antibody, or antigen-binding portion thereof, in combination with a therapeutically effective amount of an anti-P-cadherin antibody, or antigen-binding portion thereof.
Embodiment 10. The method of embodiment 9, wherein said anti-4-1BB antibody, or antigen-binding portion thereof, comprises:
   (a) an H-CDR1 as set forth in SEQ ID NO:29;
   (b) an H-CDR2 as set forth in SEQ ID NO:30;
   (c) an H-CDR3 as set forth in SEQ ID NO:31;
   (d) an L-CDR1 as set forth in SEQ ID NO:34;
   (e) an L-CDR2 as set forth in SEQ ID NO:35; and
   (f) an L-CDR3 as set forth in SEQ ID NO:36.
Embodiment 11. The method of any one of embodiments 9-10, wherein said anti-P-cadherin antibody, or antigen-binding portion thereof, comprises:
   (a) an H-CDR1 as set forth in SEQ ID NO:75;
   (b) an H-CDR2 as set forth in SEQ ID NO:76;
   (c) an H-CDR3 as set forth in SEQ ID NO:77;
   (d) an L-CDR1 as set forth in SEQ ID NO:78;
   (e) an L-CDR2 as set forth in SEQ ID NO:79; and
   (f) an L-CDR3 as set forth in SEQ ID NO:80.
Embodiment 12. The method of any one of embodiments 9-11, wherein said anti-4-1BB antibody, or antigen-binding portion thereof, comprises a V_{H} region comprising the amino acid sequence set forth in SEQ ID NO:43 and a V_{L} region comprising the amino acid sequence set forth in SEQ ID NO:45.
Embodiment 13. The method of any one of embodiments 9-12, wherein said anti-P-cadherin antibody, or antigen-binding portion thereof, comprises a V_{H} region comprising the amino acid sequence set forth in SEQ ID NO:81 and a V_{L} region comprising the amino acid sequence set forth in SEQ ID NO:82.
Embodiment 14. The method of any one of embodiments 9-13, wherein said anti-4-1BB antibody, or antigen-binding portion thereof, comprises a heavy chain amino acid sequence as set forth in SEQ ID NO:44 and further comprises a light chain amino acid sequence set forth in SEQ ID NO:46, with the proviso that the C-terminal lysine residue of SEQ ID NO:44 is optionally absent.
Embodiment 15. The method of any one of embodiments 9-14, wherein said anti-4-1BB antibody comprises MOR-7480.1.

## Claims

1. An anti-4-1BB agonist antibody, or antigen-binding portion thereof, for use in the treatment of cancer in combination with an anti-P-cadherin antibody, or antigen-binding portion thereof.

2. The anti-4-1BB antibody, or antigen-binding portion thereof, for use of claim 1, wherein said anti-4-1BB antibody, or antigen-binding portion thereof is an agonist antibody.

3. The anti-4-1BB antibody, or antigen-binding portion thereof, for use of claim 1 or 2, wherein said anti-4-1BB antibody, or antigen-binding portion thereof comprises:
(a) an H-CDR1 as set forth in SEQ ID NO:29;
(b) an H-CDR2 as set forth in SEQ ID NO:30;
(c) an H-CDR3 as set forth in SEQ ID NO:31;
(d) an L-CDR1 as set forth in SEQ ID NO:34;
(e) an L-CDR2 as set forth in SEQ ID NO:35; and
(f) an L-CDR3 as set forth in SEQ ID NO:36.

4. The anti-4-1BB antibody, or antigen-binding portion thereof, for use of any one of claims 1 to 3, wherein said anti-P-cadherin antibody, or antigen-binding portion thereof, is an antagonist antibody.

5. The anti-4-1BB antibody, or antigen-binding portion thereof, for use of any one of claims 1 to 4, wherein said anti-4-1BB antibody, or anti-gen binding portion thereof comprises a V_{H} region comprising the amino acid sequence set forth in SEQ ID NO:43 and a V_{L} region comprising the amino acid sequence set forth in SEQ ID NO:45.

6. The anti-4-1BB antibody, or antigen-binding portion thereof, for use of any one of claims 1 to 5, wherein said anti-4-1BB antibody, or anti-gen binding portion thereof comprises a heavy chain amino acid sequence as set forth in SEQ ID NO:44 and further comprises a light chain amino acid sequence set forth in SEQ ID NO:46, with the proviso that the C-terminal lysine residue of SEQ ID NO:44 is optionally absent.


SEQUENCE LISTING

<110>  Pfizer Inc.
       Sharp, Leslie Lynne

<120>  Combinations of Anti-4-1BB Antibodies and ADCC-Inducing
       Antibodies for the Treatment of Cancer

<130>  PC71799A

<160>  84

<170>  PatentIn version 3.4

<210>  1
<211>  6
<212>  PRT
<213>  Homo sapiens

<400>  1

Asn Ser Tyr Ala Ile Ser
1               5

<210>  2
<211>  17
<212>  PRT
<213>  Homo sapiens

<400>  2

Gly Ile Ile Pro Gly Phe Gly Thr Ala Asn Tyr Ala Gln Lys Phe Gln
1               5                   10                  15

Gly

<210>  3
<211>  11
<212>  PRT
<213>  Homo sapiens

<400>  3

Arg Lys Asn Glu Glu Asp Gly Gly Phe Asp His
1               5                   10

<210>  4
<211>  119
<212>  PRT
<213>  Homo sapiens

<400>  4

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Asn Ser Tyr

20                  25                  30

Ala Ile Ser Trp Val Arg Gln Ala Pro Gly Gln Gly Leu Glu Trp Met
        35                  40                  45

Gly Gly Ile Ile Pro Gly Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Lys Asn Glu Glu Asp Gly Gly Phe Asp His Trp Gly Gln Gly
            100                 105                 110

Thr Leu Val Thr Val Ser Ser
        115

<210>  5
<211>  445
<212>  PRT
<213>  Homo sapiens

<400>  5

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Asn Ser Tyr
            20                  25                  30

Ala Ile Ser Trp Val Arg Gln Ala Pro Gly Gln Gly Leu Glu Trp Met
        35                  40                  45

Gly Gly Ile Ile Pro Gly Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Lys Asn Glu Glu Asp Gly Gly Phe Asp His Trp Gly Gln Gly
            100                 105                 110

Thr Leu Val Thr Val Ser Ser Ala Ser Thr Lys Gly Pro Ser Val Phe

115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Cys Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210>  6
<211>  11
<212>  PRT
<213>  Homo sapiens

<400>  6

Ser Gly Asp Asn Leu Gly Asp Tyr Tyr Ala Ser
1               5                   10

<210>  7
<211>  7
<212>  PRT
<213>  Homo sapiens

<400>  7

Asp Asp Ser Asn Arg Pro Ser
1               5

<210>  8
<211>  10
<212>  PRT
<213>  Homo sapiens

<400>  8

Gln Thr Trp Asp Gly Thr Leu His Phe Val
1               5                   10

<210>  9
<211>  107
<212>  PRT
<213>  Homo sapiens

<400>  9

Asp Ile Glu Leu Thr Gln Pro Pro Ser Val Ser Val Ala Pro Gly Gln
1               5                   10                  15

Thr Ala Arg Ile Ser Cys Ser Gly Asp Asn Leu Gly Asp Tyr Tyr Ala
            20                  25                  30

Ser Trp Tyr Gln Gln Lys Pro Gly Gln Ala Pro Val Leu Val Ile Tyr
        35                  40                  45

Asp Asp Ser Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Thr Trp Asp Gly Thr Leu His Phe
                85                  90                  95

Val Phe Gly Gly Gly Thr Lys Leu Thr Val Leu
            100                 105

<210>  10
<211>  213
<212>  PRT
<213>  Homo sapiens

<400>  10

Asp Ile Glu Leu Thr Gln Pro Pro Ser Val Ser Val Ala Pro Gly Gln
1               5                   10                  15

Thr Ala Arg Ile Ser Cys Ser Gly Asp Asn Leu Gly Asp Tyr Tyr Ala
            20                  25                  30

Ser Trp Tyr Gln Gln Lys Pro Gly Gln Ala Pro Val Leu Val Ile Tyr
        35                  40                  45

Asp Asp Ser Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Thr Trp Asp Gly Thr Leu His Phe
                85                  90                  95

Val Phe Gly Gly Gly Thr Lys Leu Thr Val Leu Gly Gln Pro Lys Ala
            100                 105                 110

Ala Pro Ser Val Thr Leu Phe Pro Pro Ser Ser Glu Glu Leu Gln Ala
        115                 120                 125

Asn Lys Ala Thr Leu Val Cys Leu Ile Ser Asp Phe Tyr Pro Gly Ala
    130                 135                 140

Val Thr Val Ala Trp Lys Ala Asp Ser Ser Pro Val Lys Ala Gly Val
145                 150                 155                 160

Glu Thr Thr Thr Pro Ser Lys Gln Ser Asn Asn Lys Tyr Ala Ala Ser
                165                 170                 175

Ser Tyr Leu Ser Leu Thr Pro Glu Gln Trp Lys Ser His Arg Ser Tyr
            180                 185                 190

Ser Cys Gln Val Thr His Glu Gly Ser Thr Val Glu Lys Thr Val Ala
        195                 200                 205

Pro Thr Glu Cys Ser
    210

<210>  11
<211>  357
<212>  DNA
<213>  Homo sapiens

<400>  11
caggtgcaat tggttcagtc tggcgcggaa gtgaaaaaac cgggcagcag cgtgaaagtg      60

agctgcaaag cctccggagg cacttttaat tcttatgcta tttcttgggt gcgccaagcc     120

cctgggcagg gtctcgagtg gatgggcggt atcattccgg gttttggcac tgcgaattac     180

gcgcagaagt ttcagggccg ggtgaccatt accgcggatg aaagcaccag caccgcgtat     240

atggaactga gcagcctgcg tagcgaagat acggccgtgt attattgcgc gcgtaagaat     300

gaggaggatg gtggttttga tcattggggc caaggcaccc tggtgacggt tagctca        357

<210>  12
<211>  321
<212>  DNA
<213>  Homo sapiens

<400>  12
gatatcgaac tgacccagcc gccttcagtg agcgttgcac caggtcagac cgcgcgtatc      60

tcgtgtagcg gcgataatct tggtgattat tatgcttctt ggtaccagca gaaacccggg     120

caggcgccag ttcttgtgat ttatgatgat tctaatcgtc cctcaggcat cccggaacgc     180

tttagcggat ccaacagcgg caacaccgcg accctgacca ttagcggcac tcaggcggaa     240

gacgaagcgg attattattg ccagacttgg gatggtactc ttcattttgt gtttggcggc     300

ggcacgaagt taaccgttct t                                               321

<210> 13
<211> 1335
<212> DNA
<213> Homo sapiens

<400> 13
caggtgcaat tggttcagtc tggcgcggaa gtgaaaaaac cgggcagcag cgtgaaagtg 60

agctgcaaag cctccggagg cacttttaat tcttatgcta tttcttgggt gcgccaagcc 120

cctgggcagg gtctcgagtg gatgggcggt atcattccgg gttttggcac tgcgaattac 180

gcgcagaagt ttcagggccg ggtgaccatt accgcggatg aaagcaccag caccgcgtat 240

atggaactga gcagcctgcg tagcgaagat acggccgtgt attattgcgc gcgtaagaat 300

gaggaggatg gtggttttga tcattggggc caaggcaccc tggtgacggt tagctcagcc 360

tccaccaagg gcccatcggt cttccccctg gcgccctgct ccaggagcac ctccgagagc 420

acagcggccc tgggctgcct ggtcaaggac tacttccccg aaccggtgac ggtgtcgtgg 480

aactcaggcg ctctgaccag cggcgtgcac accttcccgg ctgtcctaca gtcctcagga 540

ctctactccc tcagcagcgt agtgaccgtg ccctccagca acttcggcac ccagacctac 600

acctgcaacg tagatcacaa gcccagcaac accaaggtgg acaagacagt tgagcgcaaa 660

tgttgtgtcg agtgcccacc gtgcccagca ccacctgtgg caggaccgtc agtcttcctc 720

ttccccccaa aacccaagga caccctcatg atctcccgga cccctgaggt cacgtgcgtg 780

gtggtggacg tgagccacga agaccccgag gtccagttca actggtacgt ggacggcgtg 840

gaggtgcata atgccaagac aaagccacgg gaggagcagt tcaacagcac gttccgtgtg 900

gtcagcgtcc tcaccgtcgt gcaccaggac tggctgaacg gcaaggagta caagtgcaag 960

gtctccaaca aaggcctccc agcccccatc gagaaaacca tctccaaaac caaagggcag 1020

ccccgagaac cacaggtgta caccctgccc ccatcccggg aggagatgac caagaaccag 1080

gtcagcctga cctgcctggt caaaggcttc taccccagcg acatcgccgt ggagtgggag 1140

agcaatgggc agccggagaa caactacaag accacacctc ccatgctgga ctccgacggc 1200

tccttcttcc tctacagcaa gctcaccgtg gacaagagca ggtggcagca ggggaacgtc 1260

ttctcatgct ccgtgatgca tgaggctctg cacaaccact acacacagaa gagcctctcc 1320

ctgtctccgg gtaaa 1335

<210> 14
<211> 639
<212> DNA
<213> Homo sapiens

<400> 14
gatatcgaac tgacccagcc gccttcagtg agcgttgcac caggtcagac cgcgcgtatc 60

tcgtgtagcg gcgataatct tggtgattat tatgcttctt ggtaccagca gaaacccggg 120

caggcgccag ttcttgtgat ttatgatgat tctaatcgtc cctcaggcat cccggaacgc     180

tttagcggat ccaacagcgg caacaccgcg accctgacca ttagcggcac tcaggcggaa     240

gacgaagcgg attattattg ccagacttgg gatggtactc ttcattttgt gtttggcggc     300

ggcacgaagt taaccgttct tggtcagccc aaggctgccc cctcggtcac tctgttccca     360

ccctcctctg aggagcttca agccaacaag gccacactgg tgtgtctcat aagtgacttc     420

tacccgggag ccgtgacagt ggcctggaag gcagatagca gccccgtcaa ggcgggagtg     480

gagaccacca caccctccaa acaaagcaac aacaagtacg cggccagcag ctacctgagc     540

ctgacgcctg agcagtggaa gtcccacaga agctacagct gccaggtcac gcatgaaggg     600

agcaccgtgg agaagacagt ggcccctaca gaatgttca                            639

<210>  15
<211>  6
<212>  PRT
<213>  Homo sapiens

<400>  15

Ser Asp Tyr Tyr Met His
1               5

<210>  16
<211>  17
<212>  PRT
<213>  Homo sapiens

<400>  16

Val Ile Ser Gly Ser Gly Ser Asn Thr Tyr Tyr Ala Asp Ser Val Lys
1               5                   10                  15

Gly

<210>  17
<211>  10
<212>  PRT
<213>  Homo sapiens

<400>  17

Arg Leu Tyr Ala Gln Phe Glu Gly Asp Phe
1               5                   10

<210>  18
<211>  118
<212>  PRT
<213>  Homo sapiens

<400>  18

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met His Trp Val Arg Gln Ala Pro Gly Lys Gly Leu Glu Trp Val
        35                  40                  45

Ser Val Ile Ser Gly Ser Gly Ser Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Leu Tyr Ala Gln Phe Glu Gly Asp Phe Trp Gly Gln Gly Thr
            100                 105                 110

Leu Val Thr Val Ser Ser
        115

<210>  19
<211>  444
<212>  PRT
<213>  Homo sapiens

<400>  19

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met His Trp Val Arg Gln Ala Pro Gly Lys Gly Leu Glu Trp Val
        35                  40                  45

Ser Val Ile Ser Gly Ser Gly Ser Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Leu Tyr Ala Gln Phe Glu Gly Asp Phe Trp Gly Gln Gly Thr
        100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Asn Phe Gly Thr Gln Thr Tyr Thr Cys Asn Val Asp His Lys Pro Ser
        195                 200                 205

Asn Thr Lys Val Asp Lys Thr Val Glu Arg Lys Cys Cys Val Glu Cys
    210                 215                 220

Pro Pro Cys Pro Ala Pro Pro Val Ala Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Phe Arg Val Val Ser Val Leu Thr
    290                 295                 300

Val Val His Gln Asp Trp Leu Asn Gly Lys Glu Tyr Lys Cys Lys Val
305                 310                 315                 320

Ser Asn Lys Gly Leu Pro Ala Pro Ile Glu Lys Thr Ile Ser Lys Thr
                325                 330                 335

Lys Gly Gln Pro Arg Glu Pro Gln Val Tyr Thr Leu Pro Pro Ser Arg
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Met Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys
        435                 440


<210>  20
<211>  11
<212>  PRT
<213>  Homo sapiens

<400>  20

Ser Gly Asp Asn Ile Gly Ser Lys Tyr Val Ser
1               5                   10


<210>  21
<211>  7
<212>  PRT
<213>  Homo sapiens

<400>  21

Ser Asp Ser Glu Arg Pro Ser
1               5


<210>  22
<211>  10
<212>  PRT
<213>  Homo sapiens

<400>  22

Gln Ser Trp Asp Gly Ser Ile Ser Arg Val
1               5                   10


<210>  23
<211>  107
<212>  PRT
<213>  Homo sapiens

<400>  23

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Ser Lys Tyr Val
            20                  25                  30

Ser Trp Tyr Gln Gln Lys Pro Gly Gln Ala Pro Val Leu Val Ile Tyr
        35                  40                  45

Ser Asp Ser Glu Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Ser Trp Asp Gly Ser Ile Ser Arg
                85                  90                  95

Val Phe Gly Gly Gly Thr Lys Leu Thr Val Leu
            100                 105


<210>  24
<211>  213
<212>  PRT
<213>  Homo sapiens

<400>  24

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Ser Lys Tyr Val
            20                  25                  30

Ser Trp Tyr Gln Gln Lys Pro Gly Gln Ala Pro Val Leu Val Ile Tyr
        35                  40                  45

Ser Asp Ser Glu Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Ser Trp Asp Gly Ser Ile Ser Arg
                85                  90                  95

Val Phe Gly Gly Gly Thr Lys Leu Thr Val Leu Gly Gln Pro Lys Ala
            100                 105                 110

Ala Pro Ser Val Thr Leu Phe Pro Pro Ser Ser Glu Glu Leu Gln Ala
        115                 120                 125

Asn Lys Ala Thr Leu Val Cys Leu Ile Ser Asp Phe Tyr Pro Gly Ala
    130                 135                 140

Val Thr Val Ala Trp Lys Ala Asp Ser Ser Pro Val Lys Ala Gly Val
145                 150                 155                 160

Glu Thr Thr Thr Pro Ser Lys Gln Ser Asn Asn Lys Tyr Ala Ala Ser
                165                 170                 175

Ser Tyr Leu Ser Leu Thr Pro Glu Gln Trp Lys Ser His Arg Ser Tyr
            180                 185                 190

Ser Cys Gln Val Thr His Glu Gly Ser Thr Val Glu Lys Thr Val Ala
        195                 200                 205

Pro Thr Glu Cys Ser
    210

<210>  25
<211>  354
<212>  DNA
<213>  Homo sapiens

<400>  25
caggtgcaat tggtggaaag cggcggcggc ctggtgcaac cgggcggcag cctgcgtctg      60

agctgcgcgg cctccggatt taccttttct gattattata tgcattgggt gcgccaagcc      120

cctgggaagg gtctcgagtg ggtgagcgtt atctctggtt ctggtagcaa tacctattat      180

gcggatagcg tgaaaggccg ttttaccatt tcacgtgata attcgaaaaa caccctgtat      240

ctgcaaatga acagcctgcg tgcggaagat acggccgtgt attattgcgc gcgtctttat      300

gctcagtttg agggtgattt ttggggccaa ggcaccctgg tgacggttag ctca            354

<210>  26
<211>  327
<212>  DNA
<213>  Homo sapiens

<400>  26
gatatcgaac tgacccagcc gccttcagtg agcgttgcac caggtcagac cgcgcgtatc      60

tcgtgtagcg gcgataatat tggttctaag tatgtttctt ggtaccagca gaaacccggg      120

caggcgccag ttcttgtgat ttattctgat tctgagcgtc cctcaggcat cccggaacgc      180

tttagcggat ccaacagcgg caacaccgcg accctgacca ttagcggcac tcaggcggaa      240

gacgaagcgg attattattg ccagtcttgg gatggttcta tttctcgtgt gtttggcggc 300

ggcacgaagt taaccgtcct aggtcag 327

<210> 27
<211> 1332
<212> DNA
<213> Homo sapiens

<400> 27
caggtgcaat tggtggaaag cggcggcggc ctggtgcaac cgggcggcag cctgcgtctg 60

agctgcgcgg cctccggatt taccttttct gattattata tgcattgggt gcgccaagcc 120

cctgggaagg gtctcgagtg ggtgagcgtt atctctggtt ctggtagcaa tacctattat 180

gcggatagcg tgaaaggccg ttttaccatt tcacgtgata attcgaaaaa caccctgtat 240

ctgcaaatga acagcctgcg tgcggaagat acggccgtgt attattgcgc gcgtctttat 300

gctcagtttg agggtgattt ttggggccaa ggcaccctgg tgacggttag ctcagcctcc 360

accaagggcc catcggtctt ccccctggcg ccctgctcca ggagcacctc cgagagcaca 420

gcggccctgg gctgcctggt caaggactac ttccccgaac cggtgacggt gtcgtggaac 480

tcaggcgctc tgaccagcgg cgtgcacacc ttcccggctg tcctacagtc ctcaggactc 540

tactccctca gcagcgtagt gaccgtgccc tccagcaact tcggcaccca gacctacacc 600

tgcaacgtag atcacaagcc cagcaacacc aaggtggaca agacagttga gcgcaaatgt 660

tgtgtcgagt gcccaccgtg cccagcacca cctgtggcag gaccgtcagt cttcctcttc 720

cccccaaaac ccaaggacac cctcatgatc tcccggaccc ctgaggtcac gtgcgtggtg 780

gtggacgtga gccacgaaga ccccgaggtc cagttcaact ggtacgtgga cggcgtggag 840

gtgcataatg ccaagacaaa gccacgggag gagcagttca acagcacgtt ccgtgtggtc 900

agcgtcctca ccgtcgtgca ccaggactgg ctgaacggca aggagtacaa gtgcaaggtc 960

tccaacaaag gcctcccagc ccccatcgag aaaaccatct ccaaaaccaa agggcagccc 1020

cgagaaccac aggtgtacac cctgccccca tcccgggagg agatgaccaa gaaccaggtc 1080

agcctgacct gcctggtcaa aggcttctac cccagcgaca tcgccgtgga gtgggagagc 1140

aatgggcagc cggagaacaa ctacaagacc acacctccca tgctggactc cgacggctcc 1200

ttcttcctct acagcaagct caccgtggac aagagcaggt ggcagcaggg gaacgtcttc 1260

tcatgctccg tgatgcatga ggctctgcac aaccactaca cacagaagag cctctccctg 1320

tctccgggta aa 1332

<210> 28
<211> 327
<212> DNA
<213> Homo sapiens

<400>  28
gatatcgaac tgacccagcc gccttcagtg agcgttgcac caggtcagac cgcgcgtatc      60

tcgtgtagcg gcgataatat tggttctaag tatgtttctt ggtaccagca gaaacccggg     120

caggcgccag ttcttgtgat ttattctgat tctgagcgtc cctcaggcat cccggaacgc     180

tttagcggat ccaacagcgg caacaccgcg accctgacca ttagcggcac tcaggcggaa     240

gacgaagcgg attattattg ccagtcttgg gatggttcta tttctcgtgt gtttggcggc     300

ggcacgaagt taaccgtcct aggtcag                                         327


<210>  29
<211>  6
<212>  PRT
<213>  Homo sapiens

<400>  29

Ser Thr Tyr Trp Ile Ser
1               5


<210>  30
<211>  17
<212>  PRT
<213>  Homo sapiens

<400>  30

Lys Ile Tyr Pro Gly Asp Ser Tyr Thr Asn Tyr Ser Pro Ser Phe Gln
1               5                   10                  15


Gly


<210>  31
<211>  8
<212>  PRT
<213>  Homo sapiens

<400>  31

Arg Gly Tyr Gly Ile Phe Asp Tyr
1               5


<210>  32
<211>  116
<212>  PRT
<213>  Homo sapiens

<400>  32

Gln Val Gln Leu Val Gln Ser Gly Ala Glu Val Lys Lys Pro Gly Glu
1               5                   10                  15


Ser Leu Lys Ile Ser Cys Lys Gly Ser Gly Tyr Ser Phe Ser Thr Tyr

20                  25                  30

Trp Ile Ser Trp Val Arg Gln Met Pro Gly Lys Gly Leu Glu Trp Met
        35                  40                  45

Gly Lys Ile Tyr Pro Gly Asp Ser Tyr Thr Asn Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Tyr Gly Ile Phe Asp Tyr Trp Gly Gln Gly Thr Leu Val
            100                 105                 110

Thr Val Ser Ser
        115

<210>  33
<211>  442
<212>  PRT
<213>  Homo sapiens

<400>  33

Gln Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ser Thr Tyr
            20                  25                  30

Trp Ile Ser Trp Val Arg Gln Met Pro Gly Lys Gly Leu Glu Trp Met
        35                  40                  45

Gly Lys Ile Tyr Pro Gly Asp Ser Tyr Thr Asn Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Tyr Gly Ile Phe Asp Tyr Trp Gly Gln Gly Thr Leu Val
            100                 105                 110

Thr Val Ser Ser Ala Ser Thr Lys Gly Pro Ser Val Phe Pro Leu Ala

        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Asn Phe
            180                 185                 190

Gly Thr Gln Thr Tyr Thr Cys Asn Val Asp His Lys Pro Ser Asn Thr
        195                 200                 205

Lys Val Asp Lys Thr Val Glu Arg Lys Cys Cys Val Glu Cys Pro Pro
    210                 215                 220

Cys Pro Ala Pro Pro Val Ala Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Gln Phe Asn Trp
            260                 265                 270

Tyr Val Asp Gly Val Glu Val His Asn Ala Lys Thr Lys Pro Arg Glu
        275                 280                 285

Glu Gln Phe Asn Ser Thr Phe Arg Val Val Ser Val Leu Thr Val Val
    290                 295                 300

His Gln Asp Trp Leu Asn Gly Lys Glu Tyr Lys Cys Lys Val Ser Asn
305                 310                 315                 320

Lys Gly Leu Pro Ala Pro Ile Glu Lys Thr Ile Ser Lys Thr Lys Gly
                325                 330                 335

Gln Pro Arg Glu Pro Gln Val Tyr Thr Leu Pro Pro Ser Arg Glu Glu
            340                 345                 350

Met Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Met Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  34
<211>  11
<212>  PRT
<213>  Homo sapiens

<400>  34

Ser Gly Asp Asn Ile Gly Asp Gln Tyr Ala His
1               5                   10

<210>  35
<211>  7
<212>  PRT
<213>  Homo sapiens

<400>  35

Gln Asp Lys Asn Arg Pro Ser
1               5

<210>  36
<211>  11
<212>  PRT
<213>  Homo sapiens

<400>  36

Ala Thr Tyr Thr Gly Phe Gly Ser Leu Ala Val
1               5                   10

<210>  37
<211>  108
<212>  PRT
<213>  Homo sapiens

<400>  37

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Asp Gln Tyr Ala
            20                  25                  30

His Trp Tyr Gln Gln Lys Pro Gly Gln Ala Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Ala Thr Tyr Thr Gly Phe Gly Ser Leu
                85                  90                  95

Ala Val Phe Gly Gly Gly Thr Lys Leu Thr Val Leu
            100                 105

<210>  38
<211>  214
<212>  PRT
<213>  Homo sapiens

<400>  38

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Asp Gln Tyr Ala
            20                  25                  30

His Trp Tyr Gln Gln Lys Pro Gly Gln Ala Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Ala Thr Tyr Thr Gly Phe Gly Ser Leu
                85                  90                  95

Ala Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  39
<211>  348
<212>  DNA
<213>  Homo sapiens

<400>  39
caggtgcaat tggttcagag cggcgcggaa gtgaaaaaac cgggcgaaag cctgaaaatt      60

agctgcaaag gttccggata ttccttttct acttattgga tttcttgggt gcgccagatg     120

cctgggaagg gtctcgagtg gatgggcaag atctatccgg gtgatagcta taccaattat     180

tctccgagct ttcagggcca ggtgactatt agcgcggata aaagcattag caccgcgtat     240

cttcaatgga gcagcctgaa agcgagcgat acggccatgt attattgtgc gcgtggttat     300

ggtatttttg attattgggg ccaaggcacc ctggtcaccg tctcctca                  348

<210>  40
<211>  324
<212>  DNA
<213>  Homo sapiens

<400>  40
gatatcgaac tgacccagcc gccttcagtg agcgttgcac caggtcagac cgcgcgtatc      60

tcgtgtagcg gcgataatat tggtgatcag tatgctcatt ggtaccagca gaaacccggg     120

caggcgccag ttgttgtgat ttatcaggat aagaatcgtc cctcaggcat cccggaacgc     180

tttagcggat ccaacagcgg caacaccgcg accctgacca ttagcggcac tcaggcggaa     240

gacgaagcgg attattattg cgctacttat actggttttg gttctcttgc tgtgtttggc     300

ggcggcacga agttaaccgt ccta                                            324

<210> 41
<211> 1326
<212> DNA
<213> Homo sapiens

<400> 41
caggtgcaat tggttcagag cggcgcggaa gtgaaaaaac cgggcgaaag cctgaaaatt 60

agctgcaaag gttccggata ttccttttct acttattgga tttcttgggt gcgccagatg 120

cctgggaagg gtctcgagtg gatgggcaag atctatccgg gtgatagcta taccaattat 180

tctccgagct ttcagggcca ggtgactatt agcgcggata aaagcattag caccgcgtat 240

cttcaatgga gcagcctgaa agcgagcgat acggccatgt attattgtgc gcgtggttat 300

ggtatttttg attattgggg ccaaggcacc ctggtcaccg tctcctcagc ctccaccaag 360

ggcccatcgg tcttccccct ggcgccctgc tccaggagca cctccgagag cacagcggcc 420

ctgggctgcc tggtcaagga ctacttcccc gaaccggtga cggtgtcgtg gaactcaggc 480

gctctgacca gcggcgtgca caccttcccg gctgtcctac agtcctcagg actctactcc 540

ctcagcagcg tagtgaccgt gccctccagc aacttcggca cccagaccta cacctgcaac 600

gtagatcaca agcccagcaa caccaaggtg gacaagacag ttgagcgcaa atgttgtgtc 660

gagtgcccac cgtgcccagc accacctgtg gcaggaccgt cagtcttcct cttcccccca 720

aaacccaagg acaccctcat gatctcccgg acccctgagg tcacgtgcgt ggtggtggac 780

gtgagccacg aagaccccga ggtccagttc aactggtacg tggacggcgt ggaggtgcat 840

aatgccaaga caaagccacg ggaggagcag ttcaacagca cgttccgtgt ggtcagcgtc 900

ctcaccgtcg tgcaccagga ctggctgaac ggcaaggagt acaagtgcaa ggtctccaac 960

aaaggcctcc cagcccccat cgagaaaacc atctccaaaa ccaaagggca gccccgagaa 1020

ccacaggtgt acaccctgcc cccatcccgg gaggagatga ccaagaacca ggtcagcctg 1080

acctgcctgg tcaaaggctt ctaccccagc gacatcgccg tggagtggga gagcaatggg 1140

cagccggaga acaactacaa gaccacacct cccatgctgg actccgacgg ctccttcttc 1200

ctctacagca agctcaccgt ggacaagagc aggtggcagc aggggaacgt cttctcatgc 1260

tccgtgatgc atgaggctct gcacaaccac tacacacaga agagcctctc cctgtctccg 1320

ggtaaa 1326

<210> 42
<211> 324
<212> DNA
<213> Homo sapiens

<400> 42
gatatcgaac tgacccagcc gccttcagtg agcgttgcac caggtcagac cgcgcgtatc 60

tcgtgtagcg gcgataatat tggtgatcag tatgctcatt ggtaccagca gaaacccggg 120

caggcgccag ttgttgtgat ttatcaggat aagaatcgtc cctcaggcat cccggaacgc 180

tttagcggat ccaacagcgg caacaccgcg accctgacca ttagcggcac tcaggcggaa 240

gacgaagcgg attattattg cgctacttat actggttttg gttctcttgc tgtgtttggc 300

ggcggcacga agttaaccgt ccta 324

<210> 43
<211> 116
<212> PRT
<213> Homo sapiens

<400> 43

Glu Val Gln Leu Val Gln Ser Gly Ala Glu Val Lys Lys Pro Gly Glu
1               5                   10                  15

Ser Leu Arg Ile Ser Cys Lys Gly Ser Gly Tyr Ser Phe Ser Thr Tyr
            20                  25                  30

Trp Ile Ser Trp Val Arg Gln Met Pro Gly Lys Gly Leu Glu Trp Met
        35                  40                  45

Gly Lys Ile Tyr Pro Gly Asp Ser Tyr Thr Asn Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Tyr Gly Ile Phe Asp Tyr Trp Gly Gln Gly Thr Leu Val
            100                 105                 110

Thr Val Ser Ser
        115

<210> 44
<211> 442
<212> PRT
<213> Homo sapiens

<400> 44

Glu Val Gln Leu Val Gln Ser Gly Ala Glu Val Lys Lys Pro Gly Glu
1               5                   10                  15

Ser Leu Arg Ile Ser Cys Lys Gly Ser Gly Tyr Ser Phe Ser Thr Tyr
            20                  25                  30

Trp Ile Ser Trp Val Arg Gln Met Pro Gly Lys Gly Leu Glu Trp Met
        35                  40                  45

Gly Lys Ile Tyr Pro Gly Asp Ser Tyr Thr Asn Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Tyr Gly Ile Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Asn Phe
            180                 185                 190

Gly Thr Gln Thr Tyr Thr Cys Asn Val Asp His Lys Pro Ser Asn Thr
        195                 200                 205

Lys Val Asp Lys Thr Val Glu Arg Lys Cys Cys Val Glu Cys Pro Pro
    210                 215                 220

Cys Pro Ala Pro Pro Val Ala Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Gln Phe Asn Trp
            260                 265                 270

Tyr Val Asp Gly Val Glu Val His Asn Ala Lys Thr Lys Pro Arg Glu
        275                 280                 285

Glu Gln Phe Asn Ser Thr Phe Arg Val Val Ser Val Leu Thr Val Val
    290                 295                 300

His Gln Asp Trp Leu Asn Gly Lys Glu Tyr Lys Cys Lys Val Ser Asn
305                 310                 315                 320

Lys Gly Leu Pro Ala Pro Ile Glu Lys Thr Ile Ser Lys Thr Lys Gly
                325                 330                 335

Gln Pro Arg Glu Pro Gln Val Tyr Thr Leu Pro Pro Ser Arg Glu Glu
            340                 345                 350

Met Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Met Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  45
<211>  108
<212>  PRT
<213>  Homo sapiens

<400>  45

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Leu Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Ala Thr Tyr Thr Gly Phe Gly Ser Leu
                85                  90                  95

Ala Val Phe Gly Gly Gly Thr Lys Leu Thr Val Leu
            100                 105

<210>  46
<211>  214
<212>  PRT
<213>  Homo sapiens

<400>  46

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Leu Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Ala Thr Tyr Thr Gly Phe Gly Ser Leu
                85                  90                  95

Ala Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  47
<211>  348
<212>  DNA
<213>  Homo sapiens

<400>  47
gaggtgcaat tggttcagag cggcgcggaa gtgaaaaaac cgggcgaaag cctgaggatt      60

agctgcaaag gttccggata ttccttttct acttattgga tttcttgggt gcgccagatg     120

cctgggaagg gtctcgagtg gatgggcaag atctatccgg gtgatagcta taccaattat     180

tctccgagct ttcagggcca ggtgactatt agcgcggata aaagcattag caccgcgtat     240

cttcaatgga gcagcctgaa agcgagcgat acggccatgt attattgtgc gcgtggttat     300

ggtatttttg attattgggg ccaaggcacc ctggtcaccg tctcctca                   348

<210>  48
<211>  324
<212>  DNA
<213>  Homo sapiens

<400>  48
agctacgagc tgacccagcc ccccagcgtg tccgtgagcc ctggccagac cgccagcatc      60

acctgcagcg gcgacaacat cggcgaccag tacgcccact ggtatcagca gaagcccggc     120

cagagccccg tgctggtgat ctaccaggac aagaaccggc ccagcggcat ccccgagcgg     180

ttcagcggca gcaacagcgg caacaccgcc accctgacca tcagcggcac ccaggccatg     240

gacgaggccg actactactg cgccacctac accggcttcg gcagcctggc cgtgttcggc     300

ggagggacca agctgaccgt ccta                                            324

<210>  49
<211>  1326
<212>  DNA
<213>  Homo sapiens

<400>  49
gaggtgcaat tggttcagag cggcgcggaa gtgaaaaaac cgggcgaaag cctgaggatt      60

agctgcaaag gttccggata ttccttttct acttattgga tttcttgggt gcgccagatg     120

cctgggaagg gtctcgagtg gatgggcaag atctatccgg gtgatagcta taccaattat     180

tctccgagct ttcagggcca ggtgactatt agcgcggata aaagcattag caccgcgtat 240

cttcaatgga gcagcctgaa agcgagcgat acggccatgt attattgtgc gcgtggttat 300

ggtatttttg attattgggg ccaaggcacc ctggtcaccg tctcctcagc ctccaccaag 360

ggcccatcgg tcttccccct ggcgccctgc tccaggagca cctccgagag cacagcggcc 420

ctgggctgcc tggtcaagga ctacttcccc gaaccggtga cggtgtcgtg gaactcaggc 480

gctctgacca gcggcgtgca caccttcccg gctgtcctac agtcctcagg actctactcc 540

ctcagcagcg tagtgaccgt gccctccagc aacttcggca cccagaccta cacctgcaac 600

gtagatcaca agcccagcaa caccaaggtg gacaagacag ttgagcgcaa atgttgtgtc 660

gagtgcccac cgtgcccagc accacctgtg gcaggaccgt cagtcttcct cttcccccca 720

aaacccaagg acaccctcat gatctcccgg acccctgagg tcacgtgcgt ggtggtggac 780

gtgagccacg aagaccccga ggtccagttc aactggtacg tggacggcgt ggaggtgcat 840

aatgccaaga caaagccacg ggaggagcag ttcaacagca cgttccgtgt ggtcagcgtc 900

ctcaccgtcg tgcaccagga ctggctgaac ggcaaggagt acaagtgcaa ggtctccaac 960

aaaggcctcc cagcccccat cgagaaaacc atctccaaaa ccaaagggca gccccgagaa 1020

ccacaggtgt acaccctgcc cccatcccgg gaggagatga ccaagaacca ggtcagcctg 1080

acctgcctgg tcaaaggctt ctaccccagc gacatcgccg tggagtggga gagcaatggg 1140

cagccggaga acaactacaa gaccacacct cccatgctgg actccgacgg ctccttcttc 1200

ctctacagca agctcaccgt ggacaagagc aggtggcagc aggggaacgt cttctcatgc 1260

tccgtgatgc atgaggctct gcacaaccac tacacacaga agagcctctc cctgtctccg 1320

ggtaaa 1326

<210> 50
<211> 1326
<212> DNA
<213> Homo sapiens

<400> 50
gaggtgcaat tggttcagag cggcgcggaa gtgaaaaaac cgggcgaaag cctgaggatt 60

agctgcaaag gttccggata ttccttttct acttattgga tttcttgggt gcgccagatg 120

cctgggaagg gtctcgagtg gatgggcaag atctatccgg gtgatagcta taccaattat 180

tctccgagct ttcagggcca ggtgactatt agcgcggata aaagcattag caccgcgtat 240

cttcaatgga gcagcctgaa agcgagcgat acggccatgt attattgtgc gcgtggttat 300

ggtatttttg attattgggg ccaaggcacc ctggtcaccg tctcctcagc ctccaccaag 360

ggcccatcgg tcttccccct ggcgccctgc tccaggagca cctccgagag cacagcggcc 420

ctgggctgcc tggtcaagga ctacttcccc gaaccggtga cggtgtcgtg gaactcaggc 480

gctctgacca gcggcgtgca caccttcccg gctgtcctac agtcctcagg actctactcc     540

ctcagcagcg tagtgaccgt gccctccagc aacttcggca cccagaccta cacctgcaac     600

gtagatcaca agcccagcaa caccaaggtg gacaagacag ttgagcgcaa atgttgtgtc     660

gagtgcccac cgtgcccagc accacctgtg gcaggaccgt cagtcttcct cttcccccca     720

aaacccaagg acaccctcat gatctcccgg acccctgagg tcacgtgcgt ggtggtggac     780

gtgagccacg aagaccccga ggtccagttc aactggtacg tggacggcgt ggaggtgcat     840

aatgccaaga caaagccacg ggaggagcag ttcaacagca cgttccgtgt ggtcagcgtc     900

ctcaccgtcg tgcaccagga ctggctgaac ggcaaggagt acaagtgcaa ggtctccaac     960

aaaggcctcc cagcccccat cgagaaaacc atctccaaaa ccaaagggca gccccgagaa    1020

ccacaggtgt acaccctgcc cccatcccgg gaggagatga ccaagaacca ggtcagcctg    1080

acctgcctgg tcaaaggctt ctaccccagc gacatcgccg tggagtggga gagcaatggg    1140

cagccggaga acaactacaa gaccacacct cccatgctgg actccgacgg ctccttcttc    1200

ctctacagca agctcaccgt ggacaagagc aggtggcagc aggggaacgt cttctcatgc    1260

tccgtgatgc atgaggctct gcacaaccac tacacacaga agagcctctc cctgtctccg    1320

ggtaaa                                                                1326

<210>  51
<211>  108
<212>  PRT
<213>  Homo sapiens

<400>  51

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Ala Thr Tyr Thr Gly Phe Gly Ser Leu
                85                  90                  95

Ala Val Phe Gly Gly Gly Thr Lys Leu Thr Val Leu

            100                 105

<210> 52
<211> 214
<212> PRT
<213> Homo sapiens

<400> 52

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Ala Thr Tyr Thr Gly Phe Gly Ser Leu
                85                  90                  95

Ala Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser

    210

<210>  53
<211>  324
<212>  DNA
<213>  Homo sapiens

<400>  53
agctacgagc tgacccagcc ccccagcgtg tccgtgagcc ctggccagac cgccagcatc      60

acctgcagcg gcgacaacat cggcgaccag tacgcccact ggtatcagca gaagcccggc     120

cagagccccg tggtggtgat ctaccaggac aagaaccggc ccagcggcat ccccgagcgg     180

ttcagcggca gcaacagcgg caacaccgcc accctgacca tcagcggcac ccaggccatg     240

gacgaggccg actactactg cgccacctac accggcttcg gcagcctggc cgtgttcggc     300

ggagggacca agctgaccgt ccta                                            324


<210>  54
<211>  642
<212>  DNA
<213>  Homo sapiens

<400>  54
agctacgagc tgacccagcc ccccagcgtg tccgtgagcc ctggccagac cgccagcatc      60

acctgcagcg gcgacaacat cggcgaccag tacgcccact ggtatcagca gaagcccggc     120

cagagccccg tggtggtgat ctaccaggac aagaaccggc ccagcggcat ccccgagcgg     180

ttcagcggca gcaacagcgg caacaccgcc accctgacca tcagcggcac ccaggccatg     240

gacgaggccg actactactg cgccacctac accggcttcg gcagcctggc cgtgttcggc     300

ggagggacca agctgaccgt cctaggtcag cccaaggctg ccccctcggt cactctgttc     360

ccaccctcct ctgaggagct tcaagccaac aaggccacac tggtgtgtct cataagtgac     420

ttctacccgg gagccgtgac agtggcctgg aaggcagata gcagccccgt caaggcggga     480

gtggagacca ccacaccctc caaacaaagc aacaacaagt acgcggccag cagctacctg     540

agcctgacgc ctgagcagtg gaagtcccac agaagctaca gctgccaggt cacgcatgaa     600

gggagcaccg tggagaagac agtggcccct acagaatgtt ca                        642


<210>  55
<211>  11
<212>  PRT
<213>  Homo sapiens

<400>  55

Ser Thr Tyr Thr Phe Val Gly Phe Thr Thr Val
1               5                   10


<210>  56

<211>  108
<212>  PRT
<213>  Homo sapiens

<400>  56

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Asp Gln Tyr Ala
            20                  25                  30

His Trp Tyr Gln Gln Lys Pro Gly Gln Ala Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Ser Thr Tyr Thr Phe Val Gly Phe Thr
                85                  90                  95

Thr Val Phe Gly Gly Gly Thr Lys Leu Thr Val Leu
            100                 105


<210>  57
<211>  214
<212>  PRT
<213>  Homo sapiens

<400>  57

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Asp Gln Tyr Ala
            20                  25                  30

His Trp Tyr Gln Gln Lys Pro Gly Gln Ala Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Ser Thr Tyr Thr Phe Val Gly Phe Thr
                85                  90                  95

Thr Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210> 58
<211> 324
<212> DNA
<213> Homo sapiens

<400> 58
gatatcgaac tgacccagcc gccttcagtg agcgttgcac caggtcagac cgcgcgtatc 60

tcgtgtagcg gcgataatat tggtgatcag tatgctcatt ggtaccagca gaaacccggg 120

caggcgccag ttgttgtgat ttatcaggat aagaatcgtc cctcaggcat cccggaacgc 180

tttagcggat ccaacagcgg caacaccgcg accctgacca ttagcggcac tcaggcggaa 240

gacgaagcgg attattattg ctctacttat acttttgttg gttttactac tgtgtttggc 300

ggcggcacga agttaaccgt ccta 324

<210> 59
<211> 642
<212> DNA
<213> Homo sapiens

<400> 59
gatatcgaac tgacccagcc gccttcagtg agcgttgcac caggtcagac cgcgcgtatc 60

tcgtgtagcg gcgataatat tggtgatcag tatgctcatt ggtaccagca gaaacccggg 120

caggcgccag ttgttgtgat ttatcaggat aagaatcgtc cctcaggcat cccggaacgc 180

tttagcggat ccaacagcgg caacaccgcg accctgacca ttagcggcac tcaggcggaa 240

gacgaagcgg attattattg ctctacttat acttttgttg gttttactac tgtgtttggc 300

ggcggcacga agttaaccgt cctaggtcag cccaaggctg ccccctcggt cactctgttc 360

ccaccctcct ctgaggagct tcaagccaac aaggccacac tggtgtgtct cataagtgac 420

ttctacccgg gagccgtgac agtggcctgg aaggcagata gcagccccgt caaggcggga 480

gtggagacca ccacaccctc caaacaaagc aacaacaagt acgcggccag cagctacctg 540

agcctgacgc ctgagcagtg gaagtcccac agaagctaca gctgccaggt cacgcatgaa 600

gggagcaccg tggagaagac agtggcccct acagaatgtt ca 642

<210> 60
<211> 108
<212> PRT
<213> Homo sapiens

<400> 60

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Leu Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Ser Thr Tyr Thr Phe Val Gly Phe Thr
                85                  90                  95

Thr Val Phe Gly Gly Gly Thr Lys Leu Thr Val Leu
            100                 105

<210> 61
<211> 214
<212> PRT
<213> Homo sapiens

<400> 61

Asp Ile Glu Leu Thr Gln Pro Pro Ser Val Ser Val Ala Pro Gly Gln
1               5                   10                  15

Thr Ala Arg Ile Ser Cys Ser Gly Asp Asn Ile Gly Asp Gln Tyr Ala
            20                  25                  30

His Trp Tyr Gln Gln Lys Pro Gly Gln Ala Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Ser Thr Tyr Thr Phe Val Gly Phe Thr
                85                  90                  95

Thr Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  62
<211>  324
<212>  DNA
<213>  Homo sapiens

<400>  62
agctacgagc tgacccagcc ccccagcgtg tccgtgagcc ctggccagac cgccagcatc      60

acctgcagcg gcgacaacat cggcgaccag tacgcccact ggtatcagca gaagcccggc     120

cagagccccg tgctggtgat ctaccaggac aagaaccggc ccagcggcat ccccgagcgg 180

ttcagcggca gcaacagcgg caacaccgcc accctgacca tcagcggcac ccaggccatg 240

gacgaggccg actactactg ctctacttat acttttgttg gttttactac tgtgttcggc 300

ggagggacca agctgaccgt ccta 324

<210> 63
<211> 642
<212> DNA
<213> Homo sapiens

<400> 63
agctacgagc tgacccagcc ccccagcgtg tccgtgagcc ctggccagac cgccagcatc 60

acctgcagcg gcgacaacat cggcgaccag tacgcccact ggtatcagca gaagcccggc 120

cagagccccg tgctggtgat ctaccaggac aagaaccggc ccagcggcat ccccgagcgg 180

ttcagcggca gcaacagcgg caacaccgcc accctgacca tcagcggcac ccaggccatg 240

gacgaggccg actactactg ctctacttat acttttgttg gttttactac tgtgttcggc 300

ggagggacca agctgaccgt cctaggtcag cccaaggctg ccccctcggt cactctgttc 360

ccaccctcct ctgaggagct tcaagccaac aaggccacac tggtgtgtct cataagtgac 420

ttctacccgg gagccgtgac agtggcctgg aaggcagata gcagccccgt caaggcggga 480

gtggagacca ccacaccctc caaacaaagc aacaacaagt acgcggccag cagctacctg 540

agcctgacgc ctgagcagtg gaagtcccac agaagctaca gctgccaggt cacgcatgaa 600

gggagcaccg tggagaagac agtggcccct acagaatgtt ca 642

<210> 64
<211> 108
<212> PRT
<213> Homo sapiens

<400> 64

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met

65                  70                  75                  80

Asp Glu Ala Asp Tyr Tyr Cys Ser Thr Tyr Thr Phe Val Gly Phe Thr
                85                  90                  95

Thr Val Phe Gly Gly Gly Thr Lys Leu Thr Val Leu
            100                 105

<210>  65
<211>  214
<212>  PRT
<213>  Homo sapiens

<400>  65

Ser Tyr Glu Leu Thr Gln Pro Pro Ser Val Ser Val Ser Pro Gly Gln
1               5                   10                  15

Thr Ala Ser Ile Thr Cys Ser Gly Asp Asn Ile Gly Asp Gln Tyr Ala
            20                  25                  30

His Trp Tyr Gln Gln Lys Pro Gly Gln Ser Pro Val Val Val Ile Tyr
        35                  40                  45

Gln Asp Lys Asn Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Ser Thr Tyr Thr Phe Val Gly Phe Thr
                85                  90                  95

Thr Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser

180                 185                 190

Tyr Ser Cys Gln Val Thr His Glu Gly Ser Thr Val Glu Lys Thr Val
        195                 200                 205

Ala Pro Thr Glu Cys Ser
    210

<210>  66
<211>  324
<212>  DNA
<213>  Homo sapiens

<400>  66
agctacgagc tgacccagcc ccccagcgtg tccgtgagcc ctggccagac cgccagcatc      60

acctgcagcg gcgacaacat cggcgaccag tacgcccact ggtatcagca gaagcccggc     120

cagagccccg tggtggtgat ctaccaggac aagaaccggc ccagcggcat ccccgagcgg     180

ttcagcggca gcaacagcgg caacaccgcc accctgacca tcagcggcac ccaggccatg     240

gacgaggccg actactactg ctctacttat acttttgttg gttttactac tgtgttcggc     300

ggagggacca agctgaccgt ccta                                            324

<210>  67
<211>  642
<212>  DNA
<213>  Homo sapiens

<400>  67
agctacgagc tgacccagcc ccccagcgtg tccgtgagcc ctggccagac cgccagcatc      60

acctgcagcg gcgacaacat cggcgaccag tacgcccact ggtatcagca gaagcccggc     120

cagagccccg tggtggtgat ctaccaggac aagaaccggc ccagcggcat ccccgagcgg     180

ttcagcggca gcaacagcgg caacaccgcc accctgacca tcagcggcac ccaggccatg     240

gacgaggccg actactactg ctctacttat acttttgttg gttttactac tgtgttcggc     300

ggagggacca agctgaccgt cctaggtcag cccaaggctg ccccctcggt cactctgttc     360

ccaccctcct ctgaggagct tcaagccaac aaggccacac tggtgtgtct cataagtgac     420

ttctacccgg gagccgtgac agtggcctgg aaggcagata gcagccccgt caaggcggga     480

gtggagacca ccacaccctc caaacaaagc aacaacaagt acgcggccag cagctacctg     540

agcctgacgc ctgagcagtg gaagtcccac agaagctaca gctgccaggt cacgcatgaa     600

gggagcaccg tggagaagac agtggcccct acagaatgtt ca                        642

<210>  68
<211>  255
<212>  PRT
<213>  Homo sapiens

<400>  68
   Met Gly Asn Ser Cys Tyr Asn Ile Val Ala Thr Leu Leu Leu Val Leu
   1               5                   10                  15
   Asn Phe Glu Arg Thr Arg Ser Leu Gln Asp Pro Cys Ser Asn Cys Pro
               20                  25                  30
   Ala Gly Thr Phe Cys Asp Asn Asn Arg Asn Gln Ile Cys Ser Pro Cys
           35                  40                  45
   Pro Pro Asn Ser Phe Ser Ser Ala Gly Gly Gln Arg Thr Cys Asp Ile
       50                  55                  60
   Cys Arg Gln Cys Lys Gly Val Phe Arg Thr Arg Lys Glu Cys Ser Ser
   65                  70                  75                  80
   Thr Ser Asn Ala Glu Cys Asp Cys Thr Pro Gly Phe His Cys Leu Gly
                   85                  90                  95
   Ala Gly Cys Ser Met Cys Glu Gln Asp Cys Lys Gln Gly Gln Glu Leu
               100                 105                 110
   Thr Lys Lys Gly Cys Lys Asp Cys Cys Phe Gly Thr Phe Asn Asp Gln
           115                 120                 125
   Lys Arg Gly Ile Cys Arg Pro Trp Thr Asn Cys Ser Leu Asp Gly Lys
       130                 135                 140
   Ser Val Leu Val Asn Gly Thr Lys Glu Arg Asp Val Val Cys Gly Pro
   145                 150                 155                 160
   Ser Pro Ala Asp Leu Ser Pro Gly Ala Ser Ser Val Thr Pro Pro Ala
                   165                 170                 175
   Pro Ala Arg Glu Pro Gly His Ser Pro Gln Ile Ile Ser Phe Phe Leu
               180                 185                 190
   Ala Leu Thr Ser Thr Ala Leu Leu Phe Leu Leu Phe Phe Leu Thr Leu
           195                 200                 205
   Arg Phe Ser Val Val Lys Arg Gly Arg Lys Lys Leu Leu Tyr Ile Phe
       210                 215                 220
   Lys Gln Pro Phe Met Arg Pro Val Gln Thr Thr Gln Glu Glu Asp Gly
   225                 230                 235                 240
   Cys Ser Cys Arg Phe Pro Glu Glu Glu Glu Gly Gly Cys Glu Leu
                   245                 250                 255

<210>  69
<211>  330
<212>  PRT
<213>  Homo sapiens

<400>  69

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  70
<211>  990
<212>  DNA
<213>  Homo sapiens

<400>  70
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtagtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggaggag     720

atgaccaaga accaggtcag cctgacctgc ctggtcaaag gcttctatcc cagcgacatc     780

gccgtggagt gggagagcaa tgggcagccg gagaacaact acaagaccac gcctcccgtg 840

ctggactccg acggctcctt cttcctctat agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 71
<211> 326
<212> PRT
<213> Homo sapiens

<400> 71

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  72
<211>  978
<212>  DNA
<213>  Homo sapiens

<400>  72
gcctccacca agggcccatc ggtcttcccc ctggcgccct gctccaggag cacctccgag      60

agcacagcgg ccctgggctg cctggtcaag gactacttcc ccgaaccggt gacggtgtcg     120

tggaactcag gcgctctgac cagcggcgtg cacaccttcc cggctgtcct acagtcctca     180

ggactctact ccctcagcag cgtagtgacc gtgccctcca gcaacttcgg cacccagacc     240

tacacctgca acgtagatca caagcccagc aacaccaagg tggacaagac agttgagcgc     300

aaatgttgtg tcgagtgccc accgtgccca gcaccacctg tggcaggacc gtcagtcttc     360

ctcttccccc caaaacccaa ggacaccctc atgatctccc ggacccctga ggtcacgtgc     420

gtggtggtgg acgtgagcca cgaagacccc gaggtccagt tcaactggta cgtggacggc     480

gtggaggtgc ataatgccaa gacaaagcca cgggaggagc agttcaacag cacgttccgt     540

gtggtcagcg tcctcaccgt cgtgcaccag gactggctga acggcaagga gtacaagtgc     600

aaggtctcca acaaaggcct cccagccccc atcgagaaaa ccatctccaa aaccaaaggg 660

cagccccgag aaccacaggt gtacaccctg cccccatccc gggaggagat gaccaagaac 720

caggtcagcc tgacctgcct ggtcaaaggc ttctacccca gcgacatcgc cgtggagtgg 780

gagagcaatg ggcagccgga gaacaactac aagaccacac ctcccatgct ggactccgac 840

ggctccttct tcctctacag caagctcacc gtggacaaga gcaggtggca gcaggggaac 900

gtcttctcat gctccgtgat gcatgaggct ctgcacaacc actacacaca gaagagcctc 960

tccctgtctc cgggtaaa 978

<210> 73
<211> 106
<212> PRT
<213> Homo sapiens

<400> 73

Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val
                85                  90                  95

Glu Lys Thr Val Ala Pro Thr Glu Cys Ser
            100                 105

<210> 74
<211> 318
<212> DNA
<213> Homo sapiens

<400> 74
ggtcagccca aggctgcccc ctcggtcact ctgttcccac cctcctctga ggagcttcaa 60

gccaacaagg ccacactggt gtgtctcata agtgacttct acccgggagc cgtgacagtg 120

gcctggaagg cagatagcag ccccgtcaag gcgggagtgg agaccaccac accctccaaa 180

caaagcaaca acaagtacgc ggccagcagc tacctgagcc tgacgcctga gcagtggaag    240

tcccacagaa gctacagctg ccaggtcacg catgaaggga gcaccgtgga gaagacagtg    300

gcccctacag aatgttca                                                  318

<210>  75
<211>  5
<212>  PRT
<213>  Homo sapiens

<400>  75

Ser Tyr Ala Met Ser
1               5

<210>  76
<211>  17
<212>  PRT
<213>  Homo sapiens

<400>  76

Ala Ile Ser Gly Ser Gly Gly Ser Thr Tyr Tyr Ala Asp Ser Val Lys
1               5                   10                  15

Gly

<210>  77
<211>  8
<212>  PRT
<213>  Homo sapiens

<400>  77

Thr Asn Ser Ala Lys Phe Asp Pro
1               5

<210>  78
<211>  14
<212>  PRT
<213>  Homo sapiens

<400>  78

Thr Gly Thr Ser Asn Asp Val Gly Ala Tyr Asn Tyr Val Ser
1               5                   10

<210>  79
<211>  7
<212>  PRT
<213>  Homo sapiens

<400>  79

Glu Val Asn Lys Arg Pro Ser

1               5


<210>  80
<211>  11
<212>  PRT
<213>  Homo sapiens

<400>  80

Ser Ser Tyr Thr Met Gly Ser Thr Phe Met Leu
1               5                   10


<210>  81
<211>  117
<212>  PRT
<213>  Homo sapiens

<400>  81

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Ala Met Ser Trp Val Arg Gln Ala Pro Gly Lys Gly Leu Glu Trp Val
        35                  40                  45


Ser Ala Ile Ser Gly Ser Gly Gly Ser Thr Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Thr Asn Ser Ala Lys Phe Asp Pro Trp Gly Gln Gly Thr Met
            100                 105                 110


Val Thr Val Ser Ser
        115


<210>  82
<211>  111
<212>  PRT
<213>  Homo sapiens

<400>  82

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Asn Asp Val Gly Ala Tyr
            20                  25                  30

Asn Tyr Val Ser Trp Tyr Gln Gln His Pro Gly Lys Ala Pro Lys Leu
        35                  40                  45

Met Ile Ser Glu Val Asn Lys Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Met Gly
                85                  90                  95

Ser Thr Phe Met Leu Phe Gly Gly Gly Thr Lys Leu Thr Val Leu
            100                 105                 110

<210>  83
<211>  330
<212>  PRT
<213>  Homo sapiens

<400>  83

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  84
<211>  106
<212>  PRT
<213>  Homo sapiens

<400>  84

Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val
                85                  90                  95

Glu Lys Thr Val Ala Pro Thr Glu Cys Ser
            100                 105